(19) Europäisches Patentamt
European Patent Office
Office européen des brevets

(11) EP 2 997 980 B1

(12) **EUROPEAN PATENT SPECIFICATION**

(45) Date of publication and mention of the grant of the patent:
**20.12.2017 Bulletin 2017/51**

(51) Int Cl.:
***A61K 48/00*** *(2006.01)* ***C12N 15/11*** *(2006.01)*
***A61P 3/06*** *(2006.01)*

(21) Application number: **15177512.9**

(22) Date of filing: **10.08.2005**

(54) **METHODS FOR MODULATING LIPOPROTEIN AND CHOLESTEROL LEVELS IN HUMANS**

VERFAHREN ZUR MODULATION DER LIPOPROTEIN- UND CHOLESTERINSPIEGEL BEIM MENSCHEN

PROCÉDÉS DE MODULATION DES NIVEAUX DE LIPOPROTÉINE ET CHOLESTÉROL CHEZ L'HOMME

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.08.2004 US 600785 P**
**23.09.2004 US 612831 P**

(43) Date of publication of application:
**23.03.2016 Bulletin 2016/12**

(62) Document number(s) of the earlier application(s) in accordance with Art. 76 EPC:
**11171206.3 / 2 409 713**
**05784428.4 / 1 786 472**

(73) Proprietor: **Kastle Therapeutics, LLC**
**Chicago, IL 60602 (US)**

(72) Inventors:
- **GEARY, Richard S.**
  **Carlsbad, CA California 92009 (US)**
- **YU, Zhengrong**
  **Carlsbad, CA California 92008 (US)**
- **CROOKE, Rosanne M.**
  **Carlsbad, CA California 92009 (US)**

(74) Representative: **Chapman, Desmond Mark**
**Carpmaels & Ransford LLP**
**One Southampton Row**
**London WC1B 5HA (GB)**

(56) References cited:
**WO-A-03/011887** **WO-A-03/097662**
**WO-A-2004/044181**

- **A. D. MARAIS ET AL: "Statins in homozygous familial hypercholesterolemia", CURRENT ATHEROSCLEROSIS REPORTS, vol. 4, no. 1, 1 January 2002 (2002-01-01) , pages 19-25, XP055242596, US ISSN: 1523-3804, DOI: 10.1007/s11883-002-0058-7**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> GENZYME CORPORATION

<120> METHODS FOR MODULATING LIPOPROTEIN AND CHOLESTEROL LEVELS IN HUMANS

<130> P066292EP

<150> US60612831
<151> 2004-09-23

<150> US60600785
<151> 2004-08-10

<150> PCT/US2005/028342
<151> 2005-08-10

<150> EP05784428.4
<151> 2005-08-10

<150> EP11171206.3
<151> 2005-08-10

<160> 3

<170> FastSEQ for Windows Version 4.0

<210> 1
<211> 14121
<212> DNA
<213> H. sapiens

<400> 1

```
attcccaccg ggacctgcgg ggctgagtgc ccttctcggt tgctgccgct gaggagcccg 60
cccagccagc cagggccgcg aggccgaggc caggccgcag cccaggagcc gccccaccgc 120
agctggcgat ggacccgccg aggcccgcgc tgctggcgct gctggcgctg cctgcgctgc 180
tgctgctgct gctggcgggc gccagggccg aagaggaaat gctggaaaat gtcagcctgg 240
tctgtccaaa agatgcgacc cgattcaagc acctccggaa gtacacatac aactatgagg 300
ctgagagttc cagtggagtc cctgggactg ctgattcaag aagtgccacc aggatcaact 360
gcaaggttga gctggaggtt ccccagctct gcagcttcat cctgaagacc agccagtgca 420
ccctgaaaga ggtgtatggc ttcaaccctg agggcaaagc cttgctgaag aaaaccaaga 480
actctgagga gtttgctgca gccatgtcca ggtatgagct caagctggcc attccagaag 540
ggaagcaggt tttcctttac ccggagaaag atgaacctac ttacatcctg aacatcaaga 600
ggggcatcat ttctgccctc ctggttcccc cagagacaga agaagccaag caagtgttgt 660
ttctggatac cgtgtatgga aactgctcca ctcactttac cgtcaagacg aggaagggca 720
atgtggcaac agaaatatcc actgaaagag acctggggca gtgtgatcgc ttcaagccca 780
tccgcacagg catcagccca cttgctctca tcaaaggcat gacccgcccc ttgtcaactc 840
tgatcagcag cagccagtcc tgtcagtaca cactggacgc taagaggaag catgtggcag 900
aagccatctg caaggagcaa cacctcttcc tgcctttctc ctacaacaat aagtatggga 960
tggtagcaca agtgacacag actttgaaac ttgaagacac accaaagatc aacagccgct 1020
tctttggtga aggtactaag aagatgggcc tcgcatttga gagcaccaaa tccacatcac 1080
ctccaaagca ggccgaagct gttttgaaga ctctccagga actgaaaaaa ctaaccatct 1140
ctgagcaaaa tatccagaga gctaatctct tcaataagct ggttactgag ctgagaggcc 1200
tcagtgatga agcagtcaca tctctcttgc cacagctgat tgaggtgtcc agccccatca 1260
ctttacaagc cttggttcag tgtggacagc ctcagtgctc cactcacatc ctccagtggc 1320
tgaaacgtgt gcatgccaac ccccttctga tagatgtggt cacctacctg gtggccctga 1380
tccccgagcc ctcagcacag cagctgcgag agatcttcaa catggcgagg gatcagcgca 1440
gccgagccac cttgtatgcg ctgagccacg cggtcaacaa ctatcataag acaaacccta 1500
cagggaccca ggagctgctg gacattgcta attacctgat ggaacagatt caagatgact 1560
gcactgggga tgaagattac acctatttga ttctgcgggt cattggaaat atgggccaaa 1620
ccatggagca gttaactcca gaactcaagt cttcaatcct caaatgtgtc caaagtacaa 1680
agccatcact gatgatccag aaagctgcca tccaggctct gcggaaaatg gagcctaaag 1740
acaaggacca ggaggttctt cttcagactt tccttgatga tgcttctccg ggagataagc 1800
```

gactggctgc ctatcttatg ttgatgagga gtccttcaca ggcagatatt aacaaaattg 1860
tccaaattct accatgggaa cagaatgagc aagtgaagaa ctttgtggct tcccatattg 1920
ccaatatctt gaactcagaa gaattggata tccaagatct gaaaaagtta gtgaaagaag 1980
ctctgaaaga atctcaactt ccaactgtca tggacttcag aaaattctct cggaactatc 2040
aactctacaa atctgtttct cttccatcac ttgacccagc ctcagccaaa atagaaggga 2100
atcttatatt tgatccaaat aactaccttc ctaaagaaag catgctgaaa actaccctca 2160
ctgcctttgg atttgcttca gctgacctca tcgagattgg cttggaagga aaaggctttg 2220
agccaacatt ggaagctctt tttgggaagc aaggattttt cccagacagt gtcaacaaag 2280
ctttgtactg ggttaatggt caagttcctg atggtgtctc taaggtctta gtggaccact 2340
ttggctatac caaagatgat aaacatgagc aggatatggt aaatggaata atgctcagtg 2400
ttgagaagct gattaaagat ttgaaatcca aagaagtccc ggaagccaga gcctacctcc 2460
gcatcttggg agaggagctt ggttttgcca gtctccatga cctccagctc ctgggaaagc 2520
tgcttctgat gggtgcccgc actctgcagg ggatccccca gatgattgga gaggtcatca 2580
ggaagggctc aaagaatgac tttttcttc actacatctt catggagaat gcctttgaac 2640
tccccactgg agctggatta cagttgcaaa tatcttcatc tggagtcatt gctcccggag 2700
ccaaggctgg agtaaaactg gaagtagcca acatgcaggc tgaactggtg gcaaaaccct 2760
ccgtgtctgt ggagtttgtg acaaatatgg gcatcatcat tccggacttc gctaggagtg 2820
gggtccagat gaacaccaac ttcttccacg agtcgggtct ggaggctcat gttgccctaa 2880
aagctgggaa gctgaagttt atcattcctt ccccaaagag accagtcaag ctgctcagtg 2940
gaggcaacac attacatttg gtctctacca ccaaaacgga ggtgatccca cctctcattg 3000
agaacaggca gtcctggtca gtttgcaagc aagtctttcc tggcctgaat tactgcacct 3060
caggcgctta ctccaacgcc agctccacag actccgcctc ctactatccg ctgaccgggg 3120
acaccagatt agagctggaa ctgaggccta caggagagat tgagcagtat tctgtcagcg 3180
caacctatga gctccagaga gaggacagag ccttggtgga taccctgaag tttgtaactc 3240
aagcagaagg tgcgaagcag actgaggcta ccatgacatt caaatataat cggcagagta 3300
tgaccttgtc cagtgaagtc caaattccgg attttgatgt tgacctcgga acaatcctca 3360
gagttaatga tgaatctact gagggcaaaa cgtcttacag actcaccctg gacattcaga 3420
acaagaaaat tactgaggtc gccctcatgg gccacctaag ttgtgacaca aaggaagaaa 3480
gaaaaatcaa gggtgttatt tccatacccc gtttgcaagc agaagccaga agtgagatcc 3540
tcgcccactg gtcgcctgcc aaactgcttc tccaaatgga ctcatctgct acagcttatg 3600
gctccacagt ttccaagagg gtggcatggc attatgatga agagaagatt gaatttgaat 3660
ggaacacagg caccaatgta gataccaaaa aaatgacttc caatttccct gtggatctct 3720
ccgattatcc taagagcttg catatgtatg ctaatagact cctggatcac agagtccctg 3780
aaacagacat gactttccgg cacgtgggtt ccaaattaat agttgcaatg agctcatggc 3840
ttcagaaggc atctgggagt cttccttata cccagacttt gcaagaccac ctcaatagcc 3900
tgaaggagtt caacctccag aacatgggat tgccagactt ccacatccca gaaaacctct 3960
tcttaaaaag cgatggccgg gtcaaatata ccttgaacaa gaacagtttg aaaattgaga 4020
ttcctttgcc ttttggtggc aaatcctcca gagatctaaa gatgttagag actgttagga 4080
caccagccct ccacttcaag tctgtgggat tccatctgcc atctcgagag ttccaagtcc 4140
ctacttttac cattcccaag ttgtatcaac tgcaagtgcc tctcctgggt gttctagacc 4200
tctccacgaa tgtctacagc aacttgtaca actggtccgc ctcctacagt ggtggcaaca 4260
ccagcacaga ccatttcagc cttcgggctc gttaccacat gaaggctgac tctgtggttg 4320
acctgctttc ctacaatgtg caaggatctg gagaaacaac atatgaccac aagaatacgt 4380
tcacactatc atgtgatggg tctctacgcc acaaatttct agattcgaat atcaaattca 4440
gtcatgtaga aaaacttgga aacaacccag tctcaaaagg tttactaata ttcgatgcat 4500
ctagttcctg gggaccacag atgtctgctt cagttcattt ggactccaaa aagaaacagc 4560
atttgtttgt caaagaagtc aagattgatg ggcagttcag agtctcttcg ttctatgcta 4620
aaggcacata tggcctgtct tgtcagaggg atcctaacac tggccggctc aatggagagt 4680
ccaacctgag gtttaactcc tcctacctcc aaggcaccaa ccagataaca ggaagatatg 4740
aagatggaac cctctccctc acctccacct ctgatctgca aagtggcatc attaaaaata 4800
ctgcttccct aaagtatgag aactacgagc tgactttaaa atctgacacc aatgggaagt 4860
ataagaactt tgccacttct aacaagatgg atatgacctt ctctaagcaa aatgcactgc 4920
tgcgttctga atatcaggct gattacgagt cattgaggtt cttcagcctg ctttctggat 4980
cactaaattc ccatggtctt gagttaaatg ctgacatctt aggcactgac aaaattaata 5040
gtggtgctca caaggcgaca ctaaggattg gccaagatgg aatatctacc agtgcaacga 5100
ccaacttgaa gtgtagtctc ctggtgctgg agaatgagct gaatgcagag cttggcctct 5160
ctggggcatc tatgaaatta acaacaaatg gccgcttcag ggaacacaat gcaaaattca 5220
gtctggatgg gaaagccgcc ctcacagagc tatcactggg aagtgcttat caggccatga 5280
ttctgggtgt cgacagcaaa aacattttca acttcaaggt cagtcaagaa ggacttaagc 5340
tctcaaatga catgatgggc tcatatgctg aaatgaaatt tgaccacaca aacagtctga 5400
acattgcagg cttatcactg gacttctctt caaaacttga caacatttac agctctgaca 5460
agttttataa gcaaactgtt aatttacagc tacagcccta ttctctggta actactttaa 5520
acagtgacct gaaatacaat gctctggatc tcaccaacaa tgggaaacta cggctagaac 5580

ccctgaagct gcatgtggct ggtaacctaa aaggagccta ccaaaataat gaaataaaac 5640
acatctatgc catctcttct gctgccttat cagcaagcta taaagcagac actgttgcta 5700
aggttcaggg tgtggagttt agccatcggc tcaacacaga catcgctggg ctggcttcag 5760
ccattgacat gagcacaaac tataattcag actcactgca tttcagcaat gtcttccgtt 5820
ctgtaatggc cccgtttacc atgaccatcg atgcacatac aaatggcaat gggaaactcg 5880
ctctctgggg agaacatact gggcagctgt atagcaaatt cctgttgaaa gcagaacctc 5940
tggcatttac tttctctcat gattacaaag gctccacaag tcatcatctc gtgtctagga 6000
aaagcatcag tgcagctctt gaacacaaag tcagtgccct gcttactcca gctgagcaga 6060
caggcacctg gaaactcaag acccaattta acaacaatga atacagccag gacttggatg 6120
cttacaacac taaagataaa attggcgtgg agcttactgg acgaactctg gctgacctaa 6180
ctctactaga ctccccaatt aaagtgccac ttttactcag tgagcccatc aatatcattg 6240
atgctttaga gatgagagat gccgttgaga agccccaaga atttacaatt gttgcttttg 6300
taaagtatga taaaaaccaa gatgttcact ccattaacct cccatttttt gagaccttgc 6360
aagaatattt tgagaggaat cgacaaacca ttatagttgt agtggaaaac gtacagagaa 6420
acctgaagca catcaatatt gatcaatttg taagaaaata cagagcagcc ctgggaaaac 6480
tcccacagca agctaatgat tatctgaatt cattcaattg ggagagacaa gtttcacatg 6540
ccaaggagaa actgactgct ctcacaaaaa agtatagaat tacagaaaat gatatacaaa 6600
ttgcattaga tgatgccaaa atcaacttta atgaaaaact atctcaactg cagacatata 6660
tgatacaatt tgatcagtat attaaagata gttatgattt acatgatttg aaaatagcta 6720
ttgctaatat tattgatgaa atcattgaaa aattaaaaag tcttgatgag cactatcata 6780
tccgtgtaaa tttagtaaaa acaatccatg atctacattt gtttattgaa aatattgatt 6840
ttaacaaaag tggaagtagt actgcatcct ggattcaaaa tgtggatact aagtaccaaa 6900
tcagaatcca gatacaagaa aaactgcagc agcttaagag acacatacag aatatagaca 6960
tccagcacct agctggaaag ttaaaacaac acattgaggc tattgatgtt agagtgcttt 7020
tagatcaatt gggaactaca atttcatttg aaagaataaa tgatgttctt gagcatgtca 7080
aacactttgt tataaatctt attggggatt ttgaagtagc tgagaaaatc aatgccttca 7140
gagccaaagt ccatgagtta atcgagaggt atgaagtaga ccaacaaatc caggttttaa 7200
tggataaatt agtagagttg acccaccaat acaagttgaa ggagactatt cagaagctaa 7260
gcaatgtcct acaacaagtt aagataaaag attactttga gaaattggtt ggatttattg 7320
atgatgctgt gaagaagctt aatgaattat cttttaaaac attcattgaa gatgttaaca 7380
aattccttga catgttgata aagaaattaa agtcatttga ttaccaccag tttgtagatg 7440
aaaccaatga caaaatccgt gaggtgactc agagactcaa tggtgaaatt caggctctgg 7500
aactaccaca aaaagctgaa gcattaaaac tgtttttaga ggaaaccaag gccacagttg 7560
cagtgtatct ggaaagccta caggacacca aaataacctt aatcatcaat tggttacagg 7620
aggctttaag ttcagcatct ttggctcaca tgaaggccaa attccgagag actctagaag 7680
atacacgaga ccgaatgtat caaatggaca ttcagcagga acttcaacga tacctgtctc 7740
tggtaggcca ggtttatagc acacttgtca cctacatttc tgattggtgg actcttgctg 7800
ctaagaacct tactgacttt gcagagcaat attctatcca agattgggct aaacgtatga 7860
aagcattggt agagcaaggg ttcactgttc ctgaaatcaa gaccatcctt gggaccatgc 7920
ctgcctttga agtcagtctt caggctcttc agaaagctac cttccagaca cctgatttta 7980
tagtcccccct aacagatttg aggattccat cagttcagat aaacttcaaa gacttaaaaa 8040
atataaaaat cccatccagg ttttccacac cagaatttac catccttaac accttccaca 8100
ttccttcctt tacaattgac tttgtcgaaa tgaaagtaaa gatcatcaga accattgacc 8160
agatgcagaa cagtgagctg cagtggcccg ttccagatat atatctcagg gatctgaagg 8220
tggaggacat tcctctagcg agaatcaccc tgccagactt ccgtttacca gaaatcgcaa 8280
ttccagaatt cataatccca actctcaacc ttaatgattt tcaagttcct gaccttcaca 8340
taccagaatt ccagcttccc cacatctcac acacaattga agtacctact tttggcaagc 8400
tatacagtat tctgaaaatc caatctcctc ttttcacatt agatgcaaat gctgacatag 8460
ggaatggaac cacctcagca aacgaagcag gtatcgcagc ttccatcact gccaaaggag 8520
agtccaaatt agaagttctc aattttgatt ttcaagcaaa tgcacaactc tcaaacccta 8580
agattaatcc gctggctctg aaggagtcag tgaagttctc cagcaagtac ctgagaacgg 8640
agcatgggag tgaaatgctg ttttttggaa atgctattga gggaaaatca aacacagtgg 8700
caagtttaca cacagaaaaa aatacactgg agcttagtaa tggagtgatt gtcaagataa 8760
acaatcagct taccctggat agcaacacta aatacttcca caaattgaac atccccaaac 8820
tggacttctc tagtcaggct gacctgcgca acgagatcaa gacactgttg aaagctggcc 8880
acatagcatg gacttcttct ggaaaagggt catggaaatg ggcctgcccc agattctcag 8940
atgagggaac acatgaatca caaattagtt tcaccataga aggacccctc acttcctttg 9000
gactgtccaa taagatcaat agcaaacacc taagagtaaa ccaaaacttg gtttatgaat 9060
ctggctccct caacttttct aaacttgaaa ttcaatcaca agtcgattcc cagcatgtgg 9120
gccacagtgt tctaactgct aaaggcatgg cactgtttgg agaagggaag gcagagttta 9180
ctgggaggca tgatgctcat ttaaatggaa aggttattgg aactttgaaa aattctcttt 9240
tcttttcagc ccagccattt gagatcacgg catccacaaa caatgaaggg aatttgaaag 9300
ttcgttttcc attaaggtta acagggaaga tagacttcct gaataactat gcactgtttc 9360

tgagtcccag tgcccagcaa gcaagttggc aagtaagtgc taggttcaat cagtataagt 9420
acaaccaaaa tttctctgct ggaaacaacg agaacattat ggaggcccat gtaggaataa 9480
atggagaagc aaatctggat ttcttaaaca ttcctttaac aattcctgaa atgcgtctac 9540
cttacacaat aatcacaact cctccactga aagatttctc tctatgggaa aaaacaggct 9600
tgaaggaatt cttgaaaacg acaaagcaat catttgattt aagtgtaaaa gctcagtata 9660
agaaaaacaa acacaggcat tccatcacaa atcctttggc tgtgctttgt gagtttatca 9720
gtcagagcat caaatccttt gacaggcatt ttgaaaaaaa cagaaacaat gcattagatt 9780
ttgtcaccaa atcctataat gaaacaaaaa ttaagtttga taagtacaaa gctgaaaaat 9840
ctcacgacga gctcccccagg acctttcaaa ttcctggata cactgttcca gttgtcaatg 9900
ttgaagtgtc tccattcacc atagagatgt cggcattcgg ctatgtgttc ccaaaagcag 9960
tcagcatgcc tagtttctcc atcctaggtt ctgacgtccg tgtgccttca tacacattaa 10020
tcctgccatc attagagctg ccagtccttc atgtccctag aaatctcaag ctttctcttc 10080
cacatttcaa ggaattgtgt accataagcc atatttttat tcctgccatg ggcaatatta 10140
cctatgattt ctcctttaaa tcaagtgtca tcacactgaa taccaatgct gaacttttta 10200
accagtcaga tattgttgct catctccttt cttcatcttc atctgtcatt gatgcactgc 10260
agtacaaatt agagggcacc acaagattga caagaaaaag ggattgaag ttagccacag 10320
ctctgtctct gagcaacaaa tttgtggagg gtagtcataa cagtactgtg agcttaacca 10380
cgaaaaatat ggaagtgtca gtggcaaaaa ccacaaaagc cgaaattcca atttgagaa 10440
tgaatttcaa gcaagaactt aatggaaata ccaagtcaaa acctactgtc tcttcctcca 10500
tggaatttaa gtatgatttc aattcttcaa tgctgtactc taccgctaaa ggagcagttg 10560
accacaagct tagcttggaa agcctcacct cttacttttc cattgagtca tctaccaaag 10620
gagatgtcaa gggttcggtt ctttctcggg aatattcagg aactattgct agtgaggcca 10680
acacttactt gaattccaag agcacacggt cttcagtgaa gctgcagggc acttccaaaa 10740
ttgatgatat ctggaacctt gaagtaaaag aaaattttgc tggagaagcc acactccaac 10800
gcatatattc cctctgggag cacagtacga aaaaccactt acagctagag ggcctctttt 10860
tcaccaacgg agaacataca agcaaagcca ccctggaact ctctccatgg caaatgtcag 10920
ctcttgttca ggtccatgca agtcagccca gttccttcca tgatttccct gaccttggcc 10980
aggaagtggc cctgaatgct aacactaaga accagaagat cagatggaaa aatgaagtcc 11040
ggattcattc tgggtctttc cagagccagg tcgagctttc caatgaccaa gaaaaggcac 11100
accttgacat tgcaggatcc ttagaaggac acctaaggtt cctcaaaaat atcatcctac 11160
cagtctatga caagagctta tgggatttcc taaagctgga tgtaaccacc agcattggta 11220
ggagacagca tcttcgtgtt tcaactgcct ttgtgtacac caaaaacccc aatggctatt 11280
cattctccat ccctgtaaaa gttttggctg ataaattcat tactcctggg ctgaaactaa 11340
atgatctaaa ttcagttctt gtcatgccta cgttccatgt cccatttaca gatcttcagg 11400
ttccatcgtg caaacttgac ttcagagaaa tacaaatcta taagaagctg agaacttcat 11460
catttgccct caacctacca acactccccg aggtaaaatt ccctgaagtt gatgtgttaa 11520
caaaatattc tcaaccagaa gactccttga ttcccttttt tgagataacc gtgcctgaat 11580
ctcagttaac tgtgtcccag ttcacgcttc caaaaagtgt ttcagatggc attgctgctt 11640
tggatctaaa tgcagtagcc aacaagatcg cagactttga gttgcccacc atcatcgtgc 11700
ctgagcagac cattgagatt ccctccatta agttctctgt acctgctgga attgtcattc 11760
cttcctttca agcactgact gcacgctttg aggtagactc tcccgtgtat aatgccactt 11820
ggagtgccag tttgaaaaac aaagcagatt atgttgaaac agtcctggat tccacatgca 11880
gctcaaccgt acagttccta gaatatgaac taaatgtttt gggaacacac aaaatcgaag 11940
atggtacgtt agcctctaag actaaaggaa cacttgcaca ccgtgacttc agtgcagaat 12000
atgaagaaga tggcaaattt gaaggacttc aggaatggga aggaaaagcg cacctcaata 12060
tcaaaagccc agcgttcacc gatctccatc tgcgctacca gaaagacaag aaaggcatct 12120
ccacctcagc agcctcccca gccgtaggca ccgtgggcat ggatatggat gaagatgacg 12180
acttttctaa atggaacttc tactacagcc ctcagtcctc tccagataaa aaactcacca 12240
tattcaaaac tgagttgagg gtccgggaat ctgatgagga aactcagatc aaagttaatt 12300
gggaagaaga ggcagcttct ggcttgctaa cctctctgaa agacaacgtg cccaaggcca 12360
caggggtcct ttatgattat gtcaacaagt accactggga acacacaggg ctcaccctga 12420
gagaagtgtc ttcaaagctg agaagaaatc tgcagaacaa tgctgagtgg gtttatcaag 12480
gggccattag gcaaattgat gatatcgacg tgaggttcca gaaagcagcc agtggcacca 12540
ctgggaccta ccaagagtgg aaggacaagg cccagaatct gtaccaggaa ctgttgactc 12600
aggaaggcca agccagtttc cagggactca aggataacgt gtttgatggc ttggtacgag 12660
ttactcaaaa attccatatg aaagtcaagc atctgattga ctcactcatt gattttctga 12720
acttccccag attccagttt ccggggaaac ctgggatata cactagggag gaactttgca 12780
ctatgttcat aagggaggta gggacggtac tgtcccaggt atattcgaaa gtccataatg 12840
gttcagaaat actgttttcc tatttccaag acctagtgat tacacttcct ttcgagttaa 12900
ggaaacataa actaatagat gtaatctcga tgtataggga actgttgaaa gatttatcaa 12960
aagaagccca agaggtattt aaagccattc agtctctcaa gaccacagag gtgctacgta 13020
atcttcagga cctttacaa ttcattttcc aactaataga agataacatt aaacagctga 13080
aagagatgaa atttacttat cttattaatt atatccaaga tgagatcaac acaatcttca 13140

```
atgattatat cccatatgtt tttaaattgt tgaaagaaaa cctatgcctt aatcttcata 13200
agttcaatga atttattcaa aacgagcttc aggaagcttc tcaagagtta cagcagatcc 13260
atcaatacat tatggccctt cgtgaagaat attttgatcc aagtatagtt ggctggacag 13320
tgaaatatta tgaacttgaa gaaaagatag tcagtctgat caagaacctg ttagttgctc 13380
ttaaggactt ccattctgaa tatattgtca gtgcctctaa ctttacttcc caactctcaa 13440
gtcaagttga gcaatttctg cacagaaata ttcaggaata tcttagcatc cttaccgatc 13500
cagatggaaa agggaaagag aagattgcag agctttctgc cactgctcag gaaataatta 13560
aaagccaggc cattgcgacg aagaaaataa tttctgatta ccaccagcag tttagatata 13620
aactgcaaga tttttcagac caactctctg attactatga aaaatttatt gctgaatcca 13680
aaagattgat tgacctgtcc attcaaaact accacacatt tctgatatac atcacggagt 13740
tactgaaaaa gctgcaatca accacagtca tgaacccta catgaagctt gctccaggag 13800
aacttactat catcctctaa tttttaaaa gaaatcttca tttattcttc ttttccaatt 13860
gaactttcac atagcacaga aaaaattcaa actgcctata ttgataaaac catacagtga 13920
gccagccttg cagtaggcag tagactataa gcagaagcac atatgaactg gacctgcacc 13980
aaagctggca ccagggctcg gaaggtctct gaactcagaa ggatggcatt ttttgcaagt 14040
taaagaaaat caggatctga gttattttgc taaacttggg ggaggaggaa caaataaatg 14100
gagtctttat tgtgtatcat a                                           14121
```

<210> 2
<211> 20
<212> DNA
<213> Artificial Sequence

<220>
<223> Oligonucleotide

<400> 2
gcctcagtct gcttcgcacc 20

<210> 3
<211> 20
<212> DNA
<213> Artificial Sequence

<220>
<223> Oligonucleotide

<400> 3
gcctcagtct gctttacacc 20

**Description**

BACKGROUND OF THE INVENTION

Field of the Invention

The present invention relates to an oligonucleotide comprising the nucleobase of SEQ ID No:2 for use in treating homozygous familial hypercholesterolemia according to the claims. Disclosed herein are compositions and methods for lowering LDL-cholesterol and treatment of conditions associated with elevated cholesterol levels. More specifically, disclosed are compositions and methods for inhibiting apolipoprotein B expression in the liver.

Description of the Related Art

Coronary heart disease (CHD) has been the leading cause of death in the United States for over a century, and complications from atherosclerosis are the most common causes of death in Western societies (Knopp, New Engl. J. Medicine, 1999, 341, 498-511; Davis and Hui, Arterioscler. Thromb. Vasc. Biol., 2001, 21, 887-898; Bonow, Circulation, 2002, 106, 3140-3141). Elevated low density lipoprotein-cholesterol (LDL-cholesterol) is widely recognized as a risk factor for CHD. However, despite pharmacologic intervention, many individuals are unable to lower LDL-cholesterol levels.

The guidelines for lipid lowering therapy were established by the Adult Treatment Panel III of the National Cholesterol Education Program (NCEP) in 2001. Modifications to these guidelines were recommended by the Coordinating Committee of the NCEP in 2004, and included more aggressive treatment goals (Grundy et al., Circulation, 2004, 110, 227-239). These guidelines define 3 categories of risk for major coronary events and provide desirable LDL-cholesterol target levels. Those at highest risk are patients with CHD or CHD risk equivalent and should maintain LDL-cholesterol below 100 mg/dL. The most recent NCEP guidelines recommend that patients at very high risk for CHD use drug therapy to achieve LDL-cholesterol levels of less than 70 mg/dL. CHD equivalent is defined as subjects with diabetes, peripheral vascular disease, abdominal aortic aneurysm, symptomatic carotid artery disease, and those with multiple risk factors that confer a 10 year risk for CHD greater than 20%. For the second category, those patients at moderately high risk for CHD with multiple (2 or more) risk factors in whom the 10 year risk for CHD is 20%, the goal is LDL-cholesterol of less than 130 mg/dL. The most recent recommendations include a therapeutic option to lower LDL-cholesterol levels to less than 100 mg/dL in the moderately high-risk category. The third category includes individuals with 0-1 risk factors and the target LDL-cholesterol is less than 160mg/dL. The risk factors include age, cigarette smoking, hypertension, low HDL-cholesterol, and family history of CHD. Drug therapy should be initiated when serum LDL-cholesterol remains above 130, 160 and 190 mg/dL in the 3 risk groups, respectively, despite therapeutic lifestyle changes (Grundy et al., Circulation, 2004, 110, 227-239).

Low density lipoproteins are one of five broad classes of lipoproteins, which include the following: chylomicrons, responsible for the transport dietary lipids from intestine to tissues; very low density lipoproteins (VLDL); intermediate density lipoproteins (IDL); low density lipoproteins (LDL); all of which transport triacylglycerols and cholesterol from the liver to tissues; and high density lipoproteins (HDL), which transport endogenous cholesterol from tissues to the liver. Lipoprotein particles undergo continuous metabolic processing and have variable properties and compositions. The protein components of lipoproteins are known as apolipoproteins. At least nine apolipoproteins, one of which is apolipoprotein B, are distributed in significant amounts among the various human lipoproteins.

Apolipoprotein B (also known as ApoB, apolipoprotein B-100; ApoB-100, apolipoprotein B-48; ApoB-48 and Ag(x) antigen), is a large glycoprotein involved in the assembly and secretion of lipids and in the transport and receptor-mediated uptake and delivery of distinct classes of lipoproteins. Apolipoprotein B performs a variety of functions, including the absorption and processing of dietary lipids, as well as the regulation of circulating lipoprotein levels (Davidson and Shelness, Annu. Rev. Nutr., 2000, 20, 169-193).

Two forms of apolipoprotein B exist in mammals. ApoB-100 represents the full-length protein containing 4536 amino acid residues, synthesized primarily in the human liver (Davidson and Shelness, Annu. Rev. Nutr., 2000, 20, 169-193). A truncated form known as apoB-48 is colinear with the amino terminal 2152 residues and is synthesized in the small intestine of all mammals (Davidson and Shelness, Annu. Rev. Nutr., 2000, 20, 169-193). In humans, apoB-48 circulates in association with chylomicrons and chylomicron remnants and these particles are cleared by a distinct receptor known as the LDL-receptor-related protein (Davidson and Shelness, Annu. Rev. Nutr., 2000, 20, 169-193). ApoB-48 can be viewed as an adaptation by which dietary lipid is delivered from the small intestine to the liver, while apoB-100 participates in the transport and delivery of cholesterol (Davidson and Shelness, Annu. Rev. Nutr., 2000, 20, 169-193). ApoB-100 is the major protein component of LDL and contains the domain required for interaction of this lipoprotein species with the LDL receptor. In addition, apoB-100 contains an unpaired cysteine residue which mediates an interaction with apolipoprotein(a) and generates lipoprotein(a) or Lp(a), another distinct lipoprotein with atherogenic potential (Davidson and Shelness, Annu. Rev. Nutr., 2000, 20, 169-193). Elevated plasma levels of the apoB-100-containing lipoprotein Lp(a) are associated with increased risk for atherosclerosis and its manifestations, which may include hypercholesterolemia (Seed et al., N. Engl. J. Med., 1990, 322, 1494-1499), myocardial infarction (Sandkamp et al., Clin. Chem., 1990, 36, 20-23), and thrombosis (Nowak-Gottl et al., Pediatrics, 1997, 99, E11).

Apolipoprotein B is involved cholesterol homeostasis and its overproduction has been associated with various diseases, including familial hypercholesterolemia, familial defective apoB-100 and familial combined hypercholesterolemia (Kane and Havel, The Metabolic and Molecular Bases of Inherited Diseases, 2001, 8th edition, 2717-2751). Perturbations in the metabolism of apoB-100 that correspond with an increased risk of CHD are also observed in diabetes and obesity (Grundy, Am. J. Cardiol., 1998, 81, 18B-25B; Chan et al., Diabetes, 2002, 51, 2377-2386; Chan et al., Metabolism, 2002, 51, 1041-1046). Furthermore, genetic studies in mouse models have demonstrated a correlation between elevated apolipoprotein B, elevated cholesterol levels and atherosclerosis (Kim and Young, J. Lipid Res., 1998, 39, 703-723; Nishina et al., J. Lipid Res., 1990, 31, 859-869).

In studies of patients with familial hypobetalipoproteinemia (FHBL), these patients exhibit lowered serum apolipoprotein B levels, lowered serum LDL-cholesterol levels and a reduced incidence of coronary artery disease (Schonfeld et al., J. Lipid Res., 2003, 44, 878-883). Murine studies have demonstrated that mice having heterozygous deficiencies in apolipoprotein B exhibit reduced serum LDL-cholesterol and apolipoprotein B levels, and, furthermore, are protected from diet-induced hypercholesterolemia. (Farese et al., Proc. Natl. Acad. Sci. U. S. A., 1995, 92, 1774-1778).

## SUMMARY OF THE INVENTION

Some embodiments of the present disclosure are described in the numbered paragraphs below:

1. A method of reducing serum cholesterol levels in a human subject, comprising administering to said subject a plurality of doses of an oligonucleotide targeted to apolipoprotein B, wherein said administering results in a plasma trough AUC from about 2 μg·hr/mL to about 20 μg·hr/mL for the oligonucleotide in said human subject.
2. The method of Paragraph 1, wherein said administering results in a plasma trough AUC from about 2 μg·hr/mL to about 10 μg·hr/mL.
3. The method of Paragraph 1, wherein said administering results in a plasma trough AUC from about 4 μg·hr/mL to about 6 μg·hr/mL.
4. The method of Paragraph 1, wherein said administering results in a plasma trough AUC of about 5 μg·hr/mL.
5. The method of Paragraphs 1, 2, 3 or 4, wherein said plasma trough AUC is achieved from about 3 to about 33 days subsequent to the administration of a dose of said plurality of doses of the oligonucleotide.
6. The method of Paragraph 1, wherein said serum cholesterol levels are reduced in said human subject by at least about ten percent.
7. The method of Paragraph 1, wherein said serum cholesterol levels are reduced in said human subject by at least about thirty percent.
8. The method of Paragraph 1, wherein said serum cholesterol levels are serum low density lipoprotein (LDL) cholesterol levels.
9. The method of Paragraph 1, wherein said serum cholesterol levels are serum very low density lipoprotein (VLDL) cholesterol levels.
10. The method of Paragraph 1, wherein said oligonucleotide comprises the nucleobase sequence "GCCTCAGTCTGCTTCGCACC" (SEQ ID NO: 2).
11. The method of Paragraph 1, wherein at least one dose of said plurality of doses is administered about once a week.
12. The method of Paragraph 1, wherein at least one dose of said plurality of doses is administered about once a month.
13. The method of Paragraph 1, wherein said human subject suffers from hypercholesterolemia.
14. The method of Paragraph 1, wherein each dose of said plurality of doses comprises from about 50 mg to about 400 mg of the oligonucleotide.
15. The method of Paragraph 1, wherein each dose of said plurality of doses comprises about 200 mg of the oligonucleotide.
16. A method of reducing serum cholesterol levels in a human subject, comprising administering to said subject a plurality of doses of an oligonucleotide targeted to apolipoprotein B, wherein said administering results in a plasma trough concentration from about 5 ng/mL to about 40 ng/mL of the oligonucleotide in said human subject.
17. The method of Paragraph 16, wherein said administering results in a plasma trough concentration from about 5 ng/mL to about 20 ng/mL.
18. The method of Paragraphs 16 or 17, wherein said plasma trough concentration is achieved about 7 days subsequent to the administration of a dose of said plurality of doses of the oligonucleotide.
19. The method of Paragraph 16, wherein said serum cholesterol levels are reduced in said human subject by at least about ten percent.

20. The method of Paragraph 16, wherein said serum cholesterol levels are reduced in said human subject by at least about thirty percent.

21. The method of Paragraph 16, wherein said serum cholesterol levels are serum low density lipoprotein (LDL) cholesterol levels.

22. The method of Paragraph 16, wherein said serum cholesterol levels are serum very low density lipoprotein (VLDL) cholesterol levels.

23. The method of Paragraph 16, wherein said oligonucleotide comprises the nucleobase sequence "GCCTCAGTCTGCTTCGCACC" (SEQ ID NO: 2).

24. The method of Paragraph 16, wherein at least one dose of said plurality of doses is administered about once a week.

25. The method of Paragraph 16, wherein at least one dose of said plurality of doses is administered about once a month.

26. The method of Paragraph 16, wherein said human subject suffers from hypercholesterolemia.

27. The method of Paragraph 16, wherein each dose of said plurality of doses comprises from about 50 mg to about 400 mg of the oligonucleotide.

28. The method of Paragraph 16, wherein each dose of said plurality of doses comprises about 200 mg of the oligonucleotide.

29. A method of reducing serum LDL-cholesterol in a human subject, comprising administering to the human subject a dose of an oligonucleotide comprising the nucleobase sequence "GCCTCAGTCTGCTTCGCACC" (SEQ ID NO: 2), sufficient to achieve a plasma trough AUC of at least about 2 μg·hr/mL.

30. The method of Paragraph 29, wherein said dose is sufficient to achieve a plasma trough AUC in the range of about 2 μg·hr/mL to about 20 μg·hr/mL.

31. The method of Paragraphs 29 or 30, wherein said plasma trough AUC is achieved from about 3 to about 33 days subsequent to the administration of said dose of the oligonucleotide.

32. The method of Paragraph 31, wherein said dose is administered about once a week, about once a month or about once every three months.

33. A method of reducing serum LDL-cholesterol in a human subject, comprising administering to the human subject a dose of an oligonucleotide comprising the nucleobase sequence "GCCTCAGTCTGCTTCGCACC" (SEQ ID NO: 2), sufficient to achieve a plasma trough concentration of at least about 5 ng/mL.

34. The method of Paragraph 33, wherein said dose is sufficient to achieve a plasma trough concentration in the range of about 5 ng/mL to about 40 ng/mL.

35. The method of Paragraphs 33 or 34, wherein said plasma trough concentration is achieved about 7 days subsequent to the administration of said dose of the oligonucleotide.

36. The method of Paragraph 33, wherein said dose is administered about once a week, about once a month or about once every three months.

37. A method of reducing serum LDL-cholesterol in a human subject, comprising administering to the human subject a dose of an oligonucleotide comprising the nucleobase sequence "GCCTCAGTCTGCTTCGCACC" (SEQ ID NO: 2), sufficient to achieve an estimated liver concentration of at least about 10 μg/g.

38. The method of Paragraph 37, wherein said dose is sufficient to achieve an estimated liver concentration in the range of about 10 μg/g to about 150 μg/g.

39. The method of Paragraph 37, wherein said dose is administered about once a week, about once a month or about once every three months.

40. A method of reducing serum LDL-cholesterol in a human subject comprising administering to said human subject an oligonucleotide comprising the nucleobase sequence "GCCTCAGTCTGCTTCGCACC" (SEQ ID NO: 2), wherein said oligonucleotide is administered during a loading period and a maintenance period.

41. The method of Paragraph 40, wherein said oligonucleotide is 20 to 30 nucleobases in length.

42. The method of Paragraph 40, wherein the loading period comprises administering the oligonucleotide to the human subject once a day for up to 10 days.

43. The method of Paragraph 42, wherein the oligonucleotide during the loading period is administered intravenously or subcutaneously.

44. The method of Paragraph 40, wherein the maintenance period comprises administering the oligonucleotide at least once about every 3 months.

45. The method of Paragraph 44, wherein the oligonucleotide during the maintenance period is administered once about every month.

46. The method of Paragraph 44, wherein the oligonucleotide is administered once about every 2 weeks.

47. The method of Paragraph 44, wherein the oligonucleotide is administered once about every 7 days.

48. The method of Paragraph 44, wherein the oligonucleotide during the maintenance period is administered subcutaneously.

49. The method of Paragraph 42 or 44, wherein a dose from about 0.1 mg/kg/day to about 5 mg/kg/day is administered.

50. The method of Paragraph 49, wherein the dose is from about 0.1 mg/kg/day to about 1.2 mg/kg/day.

51. The method of Paragraph 42 or 44, where the dose is from about 50 mg per week to about 600 mg per week.

52. The method of Paragraph 51, wherein the dose is about 50 mg per week.

53. The method of Paragraph 51, wherein the dose is about 100 mg per week.

54. The method of Paragraph 51, wherein the dose is about 200 mg per week.

55. The method of Paragraph 51, wherein the dose is about 400 mg per week

56. The method of Paragraph 40, wherein said oligonucleotide comprises ISIS 301012.

57. The method of Paragraph 40, wherein said method results in a reduction in serum VLDL-cholesterol, serum triglycerides, serum lipoprotein(a) or any combination of VLDL-cholesterol, serum triglycerides and serum lipoprotein(a).

58. The method of Paragraph 40, wherein said human subject exhibits at least one indication selected from the group consisting of: an elevated serum total cholesterol level, an elevated serum LDL-cholesterol level, an elevated total cholesterol:HDL ratio and an elevated LDL:HDL ratio.

59. The method of Paragraph 40, wherein said human subject has suffered from or suffers from homozygous familial hypercholesterolemia or heterozygous familial hypercholesterolemia.

60. The method of Paragraph 40, wherein said human subject has suffered from, suffers from or is at an increased risk for nonfamilial hypercholesterolemia.

61. The method of Paragraph 40, wherein the human subject has serum LDL-cholesterol levels above about 70 mg/dL prior to administering said oligonucleotide.

62. The method of Paragraph 40, wherein the human subject has serum LDL-cholesterol levels above about 100 mg/dL prior to administering said oligonucleotide.

63. The method of Paragraph 40, wherein the human subject has serum LDL-cholesterol levels above about 130 mg/dL prior to administering said oligonucleotide.

64. The method of Paragraph 61, 62, or 63, wherein administering said oligonucleotide reduces serum LDL-cholesterol levels in the human subject to less than about 70 mg/dL.

65. The method of Paragraph 62 or 63, wherein administering said oligonucleotide reduces serum LDL-cholesterol levels in the human subject to less than about 100 mg/dL.

66. The method of Paragraph 63, wherein administering said oligonucleotide reduces serum LDL cholesterol levels in the human subject to less than about 130 mg/dL.

67. A method of reducing serum cholesterol levels in a human subject, comprising selecting a human subject previously unsuccessfully treated by a statin; and administering to said human subject an oligonucleotide comprising the nucleobase sequence "GCCTCAGTCTGCTTCGCACC" (SEQ ID NO: 2).

68. The method of Paragraph 67, wherein said serum cholesterol levels comprise serum LDL-cholesterol levels.

69. The method of Paragraph 67, wherein the human subject is intolerant to a statin.

70. The method of Paragraph 67, wherein the subject has experienced adverse effects resulting from treatment with said statin.

71. The method of Paragraph 70, wherein the subject has experienced myopathy, fatigue, Central Nervous System (CNS) effects resulting from treatment with said statin or any combination of myopathy, fatigue, and CNS effects resulting from treatment with said statin.

72. The method of Paragraph 67, wherein the human subject has insufficient LDL-receptor activity.

73. The method of Paragraph 67, wherein said oligonucleotide comprises ISIS 301012.

74. A method of using an oligonucleotide comprising the nucleobase sequence "GCCTCAGTCTGCTTCGCACC" (SEQ ID NO: 2) in a treatment for reducing serum LDL-cholesterol in a human subject, said method comprising informing said human subject that the administration of said oligonucleotide results in a plasma trough AUC of at least about 2 μg·hr/mL.

75. The method of Paragraph 74, wherein said human subject is informed that the administration of said oligonucleotide results in a plasma trough AUC in the range of about 2 μg·hr/mL to about 20 μg·hr/mL.

76. The method of Paragraphs 74 or 75, wherein said plasma trough AUC is achieved from about 3 to about 33 days subsequent to the administration of said dose of the oligonucleotide.

77. The method of Paragraph 74, wherein informing said human subject comprises providing printed matter that advises that the administration of said oligonucleotide results in a plasma trough AUC of at least about 2 μg·hr/mL.

78. The method of Paragraph 77, wherein said printed matter is a label.

79. A method of using an oligonucleotide comprising the nucleobase sequence "GCCTCAGTCTGCTTCGCACC" (SEQ ID NO: 2) in a treatment for reducing serum LDL-cholesterol in a human subject, said method comprising informing said human subject that the administration of said oligonucleotide results in a plasma trough concentration of at least about 5 ng/mL.

80. The method of Paragraph 79, wherein said human subject is informed that the administration of said oligonucleotide results in a plasma trough concentration in the range of about 5 ng/mL to about 40 ng/mL.
81. The method of Paragraphs 79 or 80, wherein said plasma trough concentration is achieved about 7 days subsequent to the administration of said dose of the oligonucleotide.
82. The method of Paragraph 79, wherein informing said human subject comprises providing printed matter that advises that the administration of said oligonucleotide results in a plasma trough concentration of at least about 5 ng/mL.
83. The method of Paragraph 82, wherein said printed matter is a label.
84. A method of using an oligonucleotide comprising the nucleobase sequence "GCCTCAGTCTGCTTCGCACC" (SEQ ID NO: 2) in a treatment for reducing serum LDL-cholesterol in a human subject, said method comprising informing said human subject that the administration of said oligonucleotide results in an estimated liver concentration of said oligonucleotide of at least about 10 μg/g.
85. The method of Paragraph 84, wherein said human subject is informed that the administration of said oligonucleotide results in an estimated liver concentration of said oligonucleotide in the range of about 10 μg/g to about 150 μg/g.
86. The method of Paragraph 84, wherein informing said human subject comprises providing printed matter that advises that the administration of said oligonucleotide results in an estimated liver concentration of said oligonucleotide of at least about 10 μg/g.
87. The method of Paragraph 86, wherein said printed matter is a label.
88. A pharmaceutical composition comprising one or more doses of an oligonucleotide that is 14 to 30 nucleobases in length and which hybridizes to a nucleic acid sequence encoding apolipoprotein B, wherein each of said one or more doses ranges from about 50 mg to about 400 mg, and wherein intravenous administration to a human subject of said oligonucleotide at about 0.7 mg/kg of body weight to about 5.9 mg/kg of body weight is sufficient to achieve a plasma $AUC_{0-48}$ from about 11 μg·hr/mL to about 148 μg·hr/mL.
89. The pharmaceutical composition of Paragraph 88, wherein intravenous administration to a human subject of said oligonucleotide at about 0.7 mg/kg of body weight is sufficient to achieve a plasma $AUC_{0-48}$ of 11 μg·hr/mL ± 3 μg·hr/mL.
90. The pharmaceutical composition of Paragraph 88, wherein intravenous administration to a human subject of said oligonucleotide at about 1 mg/kg of body weight is sufficient to achieve a plasma $AUC_{0-48}$ of 24 μg·hr/mL ± 3 μg·hr/mL.
91. The pharmaceutical composition of Paragraph 88, wherein intravenous administration to a human subject of said oligonucleotide at about 2.7 mg/kg of body weight is sufficient to achieve a plasma $AUC_{0-48}$ of 68 μg·hr/mL ± 14 μg·hr/mL.
92. The pharmaceutical composition of Paragraph 88, wherein intravenous administration to a human subject of said oligonucleotide at about 5.9 mg/kg of body weight is sufficient to achieve a plasma $AUC_{0-48}$ of 148 μg·hr/mL ± 14 μg·hr/mL.
93. The pharmaceutical composition of any of Paragraphs 88 to 92, wherein said oligonucleotide hybridizes to a region of said nucleic acid sequence encoding apolipoprotein B that comprises nucleotides 2920 to 3420 of SEQ ID NO: 1.
94. The pharmaceutical composition of any of Paragraphs 88 to 92, wherein said oligonucleotide hybridizes to a region of said nucleic acid sequence encoding apolipoprotein B that comprises nucleotides 3230 to 3288 of SEQ ID NO: 1.
95. The pharmaceutical composition of any of Paragraphs 88 to 92, wherein said oligonucleotide comprises the nucleobase sequence "GCCTCAGTCTGCTTCGCACC" (SEQ ID NO: 2).
96. The pharmaceutical composition of Paragraph 95, wherein said oligonucleotide comprises ISIS 301012.
97. A pharmaceutical composition comprising one or more doses of an oligonucleotide that is 14 to 30 nucleobases in length and which hybridizes to a nucleic acid sequence encoding apolipoprotein B, wherein each of said one or more doses ranges from about 50 mg to about 400 mg, and wherein subcutaneous administration to a human subject of said oligonucleotide at about 0.7 mg/kg of body weight to about 5.9 mg/kg of body weight, subsequent to the administration of one or more loading doses, is sufficient to achieve a plasma AUC from about 19 μg·hr/mL to about 160 μg·hr/mL.
98. The pharmaceutical composition of Paragraph 97, wherein subcutaneous administration to a human subject of said oligonucleotide at about 0.7 mg/kg of body weight is sufficient to achieve a plasma AUC of 19 μg·hr/mL ± 9 μg·hr/mL.
99. The pharmaceutical composition of Paragraph 97, wherein subcutaneous administration to a human subject of said oligonucleotide at about 1 mg/kg of body weight is sufficient to achieve a plasma AUC of 28 μg·hr/mL ± 5 μg·hr/mL.
100. The pharmaceutical composition of Paragraph 97, wherein subcutaneous administration to a human subject of said oligonucleotide at about 2.7 mg/kg of body weight is sufficient to achieve a plasma AUC of 63 μg·hr/mL ± 13 μg·hr/mL.

101. The pharmaceutical composition of Paragraph 97, wherein subcutaneous administration to a human subject of said oligonucleotide at about 5.9 mg/kg of body weight is sufficient to achieve a plasma AUC of 160 μg·hr/mL.

102. The pharmaceutical composition of any of Paragraphs 97 to 101, wherein said oligonucleotide hybridizes to a region of said nucleic acid sequence encoding apolipoprotein B that comprises nucleotides 2920 to 3420 of SEQ ID NO: 1.

103. The pharmaceutical composition of any of Paragraphs 97 to 101, wherein said oligonucleotide hybridizes to a region of said nucleic acid sequence encoding apolipoprotein B that comprises nucleotides 3230 to 3288 of SEQ ID NO: 1.

104. The pharmaceutical composition of any of Paragraphs 97 to 101, wherein said oligonucleotide comprises the nucleobase sequence "GCCTCAGTCTGCTTCGCACC" (SEQ ID NO: 2).

105. The pharmaceutical composition of Paragraph 104, wherein said oligonucleotide comprises ISIS 301012.

106. A pharmaceutical composition comprising one or more doses of an oligonucleotide that is 14 to 30 nucleobases in length and which hybridizes to a nucleic acid sequence encoding apolipoprotein B, wherein each of said one or more doses ranges from about 50 mg to about 400 mg, and wherein subcutaneous administration to a human subject of said oligonucleotide at about 0.7 mg/kg of body weight to about 5.9 mg/kg of body weight, subsequent to the administration of one or more loading doses, is sufficient to achieve a plasma trough concentration from about 2 ng/mL to about 40 ng/mL.

107. The pharmaceutical composition of Paragraph 106, wherein said oligonucleotide hybridizes to a region of said nucleic acid sequence encoding apolipoprotein B that comprises nucleotides 2920 to 3420 of SEQ ID NO: 1.

108. The pharmaceutical composition of Paragraph 106, wherein said oligonucleotide hybridizes to a region of said nucleic acid sequence encoding apolipoprotein B that comprises nucleotides 3230 to 3288 of SEQ ID NO: 1.

109. The pharmaceutical composition of Paragraph 106, wherein said oligonucleotide comprises the nucleobase sequence "GCCTCAGTCTGCTTCGCACC" (SEQ ID NO: 2).

110. A pharmaceutical composition comprising one or more doses of an oligonucleotide that is 14 to 30 nucleobases in length and which hybridizes to a nucleic acid sequence encoding apolipoprotein B, wherein each of said one or more doses ranges from about 50 mg to about 400 mg, and wherein subcutaneous administration to a human subject of said oligonucleotide at about 0.7 mg/kg of body weight to about 5.9 mg/kg of body weight, subsequent to the administration of one or more loading doses, is sufficient to achieve a bioavailability of at least about 54%.

111. The pharmaceutical composition of Paragraph 110, wherein said oligonucleotide hybridizes to a region of said nucleic acid sequence encoding apolipoprotein B that comprises nucleotides 2920 to 3420 of SEQ ID NO: 1.

112. The pharmaceutical composition of Paragraph 110, wherein said oligonucleotide hybridizes to a region of said nucleic acid sequence encoding apolipoprotein B that comprises nucleotides 3230 to 3288 of SEQ ID NO: 1.

113. The pharmaceutical composition of Paragraph 110, wherein said oligonucleotide comprises the nucleobase sequence "GCCTCAGTCTGCTTCGCACC" (SEQ ID NO: 2).

114. A pharmaceutical composition comprising one or more doses of an oligonucleotide that is 14 to 30 nucleobases in length and which hybridizes to a nucleic acid sequence encoding apolipoprotein B, wherein each of said one or more doses ranges from about 50 mg to about 400 mg, and wherein subcutaneous administration to a human subject of said oligonucleotide at about 0.7 mg/kg of body weight to about 5.9 mg/kg of body weight, subsequent to the administration of one or more loading doses, is sufficient to achieve a terminal elimination $t_{1/2}$ from about 23 to about 47 days.

115. The pharmaceutical composition of Paragraph 114, wherein subcutaneous administration to a human subject of said oligonucleotide at about 0.7 mg/kg of body weight is sufficient to achieve a terminal elimination $t_{1/2}$ of about 23 days ± 1 day.

116. The pharmaceutical composition of Paragraph 114, wherein subcutaneous administration to a human subject of said oligonucleotide at about 1 mg/kg of body weight is sufficient to achieve a terminal elimination $t_{1/2}$ of about 27 days ± 12 days.

117. The pharmaceutical composition of Paragraph 114, wherein subcutaneous administration to a human subject of said oligonucleotide at about 2.7 mg/kg of body weight is sufficient to achieve a terminal elimination $t_{1/2}$ of about 31 days ± 11 days.

118. The pharmaceutical composition of Paragraph 114, wherein subcutaneous administration to a human subject of said oligonucleotide at about 5.9 mg/kg of body weight is sufficient to achieve a terminal elimination $t_{1/2}$ of about 47 days.

119. The pharmaceutical composition of any of Paragraphs 114 to 118, wherein said oligonucleotide hybridizes to a region of said nucleic acid sequence encoding apolipoprotein B that comprises nucleotides 2920 to 3420 of SEQ ID NO: 1.

120. The pharmaceutical composition of any of Paragraphs 114 to 118, wherein said oligonucleotide hybridizes to a region of said nucleic acid sequence encoding apolipoprotein B that comprises nucleotides 3230 to 3288 of SEQ ID NO: 1.

121. The pharmaceutical composition of any of Paragraphs 114 to 118, wherein said oligonucleotide comprises the nucleobase sequence "GCCTCAGTCTGCTTCGCACC" (SEQ ID NO: 2).

122. The pharmaceutical composition of Paragraph 121, wherein said oligonucleotide comprises ISIS 301012.

123. A plurality of doses of an oligonucleotide targeted to apolipoprotein B for reducing serum cholesterol levels wherein administration of said plurality of doses said oligonucleotide results in a plasma trough AUC for said oligonucleotide from about 2 μg·hr/mL to about 20 μg·hr/mL.

124. The plurality of doses of Paragraph 123, wherein said administration results in a plasma trough AUC from about 2 μg·hr/mL to about 10 μg·hr/mL.

125. The plurality of doses of Paragraph 123, wherein said administration results in a plasma trough AUC from about 4 μg·hr/mL to about 6 μg·hr/mL.

126. The plurality of doses of Paragraph 123, wherein said administration results in a plasma trough AUC of about 5 μg·hr/mL.

127. The plurality of doses of Paragraphs 123, 124, 125 or 126, wherein said plasma trough AUC is achieved from about 3 to about 33 days subsequent to the administration of a dose of said plurality of doses of the oligonucleotide.

128. The plurality of doses of Paragraph 123, wherein said serum cholesterol levels are reduced by least ten percent.

129. The plurality of doses of Paragraph 123, wherein said serum cholesterol levels is are reduced by least thirty percent.

130. The plurality of doses of Paragraph 123, wherein said serum cholesterol levels are serum LDL-cholesterol levels.

131. The plurality of doses of Paragraph 123, wherein said serum cholesterol levels are serum VLDL- cholesterol levels.

132. The plurality of doses of Paragraph 123, wherein said oligonucleotide comprises the nucleobase sequence "GCCTCAGTCTGCTTCGCACC" (SEQ ID NO: 2).

133. The plurality of doses of Paragraph 127, wherein administration is about once a week.

134. The plurality of doses of Paragraph 127, wherein administration is about once a month.

135. The plurality of doses of Paragraph 127, wherein each of said plurality of doses comprises from about 50 mg to about 400 mg of said oligonucleotide.

136. The plurality of doses of Paragraph 127, wherein each of said plurality of doses comprises about 200 mg of said oligonucleotide.

137. A plurality of doses of an oligonucleotide targeted to apolipoprotein B for reducing serum cholesterol levels wherein administration of said plurality of doses said oligonucleotide results in a plasma trough concentration from about 5 ng/mL to about 40 ng/mL.

138. The plurality of doses of Paragraph 137, wherein administration results in a plasma trough concentration from about 5 ng/mL to about 20 ng/mL.

139. The plurality of doses of Paragraphs 137 or 138, wherein said plasma trough concentration is achieved about 7 days subsequent to the administration of a dose of said plurality of doses of the oligonucleotide.

140. The plurality of doses of Paragraph 137, wherein said serum cholesterol levels are reduced by least ten percent.

141. The plurality of doses of Paragraph 137, wherein said serum cholesterol levels are reduced by least thirty percent.

142. The plurality of doses of Paragraph 137, wherein said serum cholesterol levels are serum LDL-cholesterol levels.

143. The plurality of doses of Paragraph 137, wherein said serum cholesterol levels are serum VLDL-cholesterol levels.

144. The plurality of doses of Paragraph 137, wherein said oligonucleotide comprises the nucleobase sequence "GCCTCAGTCTGCTTCGCACC" (SEQ ID NO: 2).

145. The plurality of doses of Paragraph 137, wherein administration is about once a week.

146. The plurality of doses of Paragraph 137, wherein administration is about once a month.

147.The plurality of doses of Paragraph 137, wherein each of said plurality of doses comprises from about 50 mg to about 400 mg of said oligonucleotide.

148.The plurality of doses of Paragraph 137, wherein each of said plurality of doses comprises about 200 mg of said oligonucleotide.

149. Use of a plurality of doses of an oligonucleotide targeted to apolipoprotein B for the preparation of a medicament for reducing serum cholesterol levels, wherein administration of said medicament results in a plasma trough AUC for said oligonucleotide from about 2 μg·hr/mL to about 10 μg·hr/mL.

150. The use of Paragraph 149, wherein administration of said medicament results in a plasma trough AUC from about 2 μg·hr/mL to about 10 μg·hr/mL.

151. The use of Paragraph 149, wherein administration of said medicament results in a plasma trough AUC from about 4 μg·hr/mL to about 6 μg·hr/mL.

152. The use of Paragraph 149, wherein administration of said medicament results in a plasma trough AUC of about

5 μg.hr/mL.

153. The use of Paragraphs 149, 150, 151 or 152, wherein said plasma trough AUC is achieved from about 3 to about 33 days subsequent to the administration of a dose of said medicament.

154. The use of Paragraph 149, wherein said serum cholesterol levels are reduced by least about ten percent.

155. The use of Paragraph 149, wherein said serum cholesterol levels are reduced by least about thirty percent.

156. The use of Paragraph 149, wherein said serum cholesterol levels are serum LDL-cholesterol levels.

157. The use of Paragraph 149, wherein said serum cholesterol levels are serum VLDL-cholesterol levels.

158. The use of Paragraph 149, wherein said oligonucleotide comprises the nucleobase sequence "GCCTCAGTCTGCTTCGCACC" (SEQ ID NO: 2).

159. The use of Paragraph 149, wherein said administration is about once a week.

160. The use of Paragraph 149, wherein said administration is about once a month.

161. The use of Paragraph 149, wherein each of said plurality of doses comprises from about 50 mg to about 400 mg of said oligonucleotide.

162. The use of Paragraph 149, wherein each of said plurality of doses comprises about 200 mg of said oligonucleotide.

163. Use of a plurality of doses of an oligonucleotide targeted to apolipoprotein B for the preparation of a medicament for reducing serum cholesterol levels, wherein administration of said medicament results in a plasma trough concentration of said oligonucleotide from about 5 ng/mL to about 40 ng/mL.

164. The use of Paragraph 163, wherein administration of said medicament results in a plasma trough concentration from about 5 ng/mL to about 20 ng/mL.

165. The use of Paragraphs 163 or 164, wherein said plasma trough concentration is achieved about 7 days subsequent to the administration of a dose of said medicament.

166. The use of Paragraph 163, wherein said serum cholesterol levels are reduced by least about ten percent.

167. The use of Paragraph 163, wherein said serum cholesterol levels are reduced by least about thirty percent.

168. The use of Paragraph 163, wherein said serum cholesterol levels are serum LDL-cholesterol levels.

169. The use of Paragraph 163, wherein said serum cholesterol levels are serum VLDL-cholesterol levels.

170. The use of Paragraph 163, wherein said oligonucleotide comprises the nucleobase sequence "GCCTCAGTCTGCTTCGCACC" (SEQ ID NO: 2).

171. The use of Paragraph 163, wherein administration is about once a week.

172. The use of Paragraph 163, wherein administration is about once a month.

173. The use of Paragraph 163, wherein each of said plurality of doses comprises from about 50 mg to about 400 mg of said oligonucleotide.

174. The use of Paragraph 163, wherein each of said plurality of doses comprises about 200 mg of said oligonucleotide.

175. Use of an oligonucleotide comprising the nucleobase sequence "GCCTCAGTCTGCTTCGCACC" (SEQ ID NO: 2) for the preparation of a medicament for reducing serum LDL-cholesterol, wherein administration of said medicament is sufficient to achieve a plasma trough AUC for said oligonucleotide of at least about 2 μg·hr/mL.

176. The use of Paragraph 175, wherein said administration of said medicament is sufficient to achieve a plasma trough AUC in the range of about 2 μg·hr/mL to about 20 μg·hr/mL.

177. The use of Paragraphs 175 or 176, wherein said plasma trough AUC is achieved from about 3 to about 33 days subsequent to the administration of said medicament.

178. The use of Paragraph 175, wherein the administration of said medicament occurs about once a week, about once a month or about once every three months.

179. Use of an oligonucleotide comprising the nucleobase sequence "GCCTCAGTCTGCTTCGCACC" (SEQ ID NO: 2) for the preparation of a medicament for reducing serum LDL-cholesterol, wherein administration of said medicament is sufficient to achieve a plasma trough concentration for said oligonucleotide of at least about 5 ng/mL.

180. The use of Paragraph 179, wherein said administration of said medicament is sufficient to achieve a plasma trough concentration in the range of about 5 ng/mL to about 40 ng/mL.

181. The use of Paragraphs 179 or 180, wherein said plasma trough concentration is achieved about 7 days subsequent to the administration of said medicament.

182. The use of Paragraph 179, wherein the administration of said medicament occurs about once a week, about once a month or about once every three months.

183. Use of an oligonucleotide comprising the nucleobase sequence "GCCTCAGTCTGCTTCGCACC" (SEQ ID NO: 2) for the preparation of a medicament for reducing serum LDL-cholesterol, wherein administration of said medicament is sufficient to achieve an estimated liver concentration of at least about 10 μg/g.

184. The use of Paragraph 183, wherein said administration of said medicament is sufficient to achieve an estimated liver concentration in the range of about 10 μg/g to about 150 μg/g.

185. The use of Paragraph 183, wherein the administration of said medicament occurs about once a week, about once a month or about once every three months.

186. Use of an oligonucleotide comprising the nucleobase sequence "GCCTCAGTCTGCTTCGCACC" (SEQ ID NO: 2) for the preparation of a medicament for reducing serum LDL-cholesterol, wherein said medicament is administered during a loading period and a maintenance period.

187. The use of Paragraph 186, wherein said oligonucleotide is 20 to 30 nucleobases in length.

188. The use of Paragraph 186, wherein the loading period comprises administering the medicament once a day for up to 10 days.

189. The use of Paragraph 188, wherein said medicament is administered intravenously or subcutaneously.

190. The use of Paragraph 186, wherein the maintenance period comprises administering the medicament at least once about every 3 months.

191. The use of Paragraph 190, wherein the medicament is administered once about every month.

192. The use of Paragraph 190, wherein the medicament is administered once about every 2 weeks.

193. The use of Paragraph 190, wherein the medicament is administered once about every 7 days.

194. The use of Paragraph 190, wherein said medicament is administered intravenously or subcutaneously.

195. The use of Paragraphs 188 or 190, wherein the oligonucleotide present in the medicament is administered in a dose from about 0.1 mg/kg/day to about 5 mg/kg/day.

196. The use of Paragraphs 195, wherein the oligonucleotide present in the medicament is administered in a dose from about 0.1 mg/kg/day to about 1.2 mg/kg/day.

197. The use of Paragraphs 188 or 190, wherein the oligonucleotide present in the medicament is administered in a dose from about 50 mg per week to about 600 mg per week.

198. The use of Paragraph 197, wherein the oligonucleotide present in the medicament is administered in a dose of about 50 mg per week.

199. The use of Paragraph 197, wherein the dose is about 100 mg per week.

200. The use of Paragraph 197, wherein the dose is about 200 mg per week.

201. The use of Paragraph 197, wherein the dose is about 400 mg per week.

202. The use of Paragraph 186, wherein the oligonucleotide comprises ISIS 301012.

203. The use of Paragraph 186, wherein administration of the medicament results in a reduction in serum VLDL-cholesterol, serum triglycerides, serum lipoprotein(a) or any combination of serum VLDL-cholesterol, serum triglycerides and serum lipoprotein(a).

204. The use of Paragraph 186, wherein the medicament is administered to a human subject that exhibits at least one indication selected from the group consisting of an elevated serum total cholesterol level, an elevated serum LDL-cholesterol level, an elevated total cholesterol:HDL ratio and an elevated LDL:HDL ratio.

205. The use of Paragraph 186, wherein the medicament is administered to a human subject who has suffered from or suffers from homozygous familial hypercholesterolemia, or heterozygous familial hypercholesterolemia.

206. The use of Paragraph 186, wherein the medicament is administered to a human subject who has suffered from, suffers from or is at an increased risk for nonfamilial hypercholesterolemia.

207. The use of Paragraph 186, wherein the medicament is administered to a human subject who has serum-LDL cholesterol levels above about 70 mg/dL prior to administration.

208. The use of Paragraph 186, wherein the medicament is administered to a human subject who has serum-LDL cholesterol levels above about 100 mg/dL prior to administration.

209. The use of Paragraph 186, wherein the medicament is administered to a human subject who has serum-LDL cholesterol levels above about 130 mg/dL prior to administration.

210. The use of Paragraphs 207, 208 or 209, wherein the administering said medicament reduces the serum-LDL cholesterol levels to less than about 70 mg/dL.

211. The use of Paragraphs 208 or 209, wherein the administering said medicament reduces the serum-LDL cholesterol levels to less than about 100 mg/dL.

212. The use of Paragraph 209, wherein the administering said medicament reduces the serum LDL-cholesterol levels to less than about 130 mg/dL.

213. The method of Paragraphs 29, 33, 37, 74, 79, or 84, wherein said oligonucleotide comprises ISIS 301012.

214. The pharmaceutical composition of Paragraphs 109 or 113, wherein said oligonucleotide comprises ISIS 301012.

215. The plurality of doses of Paragraphs 132 or 144, wherein said oligonucleotide comprises ISIS 301012.

216. The use of Paragraphs 158, 170, 175, 179, 183, 186, wherein said oligonucleotide comprises ISIS 301012.

Disclosed are also methods of reducing serum cholesterol by administering an oligonuleotide targeted to apolipoprotein B, such as the antisense oligonuleotide ISIS 301012 and a second lipid-lowering agent at a dose lower than that which would be required to achieve a therapeutic or prophylactic effect if the second agent was administered alone. For example, second lipid-lowering agents can be selected from the group consisting of bile acid sequestrants (e.g.,

cholestyramine, colestipol, and colesevelam hydrochloride), fibrates (e.g., clofibrate, gemfibrozil, fenofibrate, bezafibrate, and ciprofibrate), niacin, statins (e.g., lovastatin, prevastatin, atorvastatin, simvastatin, and fluvastatin), and cholesterol absorption inhibitors (e.g., ezetimibe).

BRIEF DESCRIPTION OF THE DRAWINGS

**Figure 1** is a line graph showing the percent change from baseline of apolipoprotein B (ApoB-100), serum LDL-cholesterol (LDL) and serum total cholesterol in relating to plasma trough AUC levels in human subjects approximately 3 days following the end of the multiple dose treatment period (MD25) described in Example 3.

DETAILED DESCRIPTION OF THE INVENTION

Disclosed are compositions and methods for lowering serum LDL-cholesterol levels in a human suffering from, or at risk for, hypercholesterolemia by administering to the human an oligonucleotide targeted to apolipoprotein B. As used herein "targeting" or "targeted to" refers to an oligonucleotide capable of hybridizing with a selected nucleic acid molecule or region of a nucleic acid molecule. Such hybridization of an oligonucleotide with its target nucleic acid modulates (inhibits or stimulates) the normal function of the nucleic acid through a mechanism generally referred to as "antisense." In one embodiment, the oligonucleotide comprises the nucleobase sequence "GCCTCAGTCTGCTTCGCACC" (SEQ ID NO: 2). In other embodiments, the oligonucleotide is ISIS 301012. It was discovered that the oligonucleotide ISIS 301012 was effective at reducing the level of serum apolipoprotein B and serum LDL-cholesterol in humans for an extended period after administration. For example, treatment of humans with ISIS 301012 led to lowered LDL-cholesterol and apolipoprotein B levels for several weeks following cessation of treatment. For this reason, ISIS 301012 provides an extended durational effect on hypercholesterolemia, and thereby is a useful for the treatment for this disease.

An additional embodiment of the disclosure relates to administering an oligonucleotide targeted to apolipoprotein B to a human subject so that the plasma trough AUC is between about 2 to 20 μg·hr/mL, 2 to 10 μg·hr/mL, or 2 to 7 μg·hr/mL. In one embodiment, the oligonucleotide is administered so that the plasma trough AUC is about 4 to 6 μg·hr/mL. In yet another embodiment, the oligonucleotide is administered so that the plasma trough AUC is about 5 μg·hr/mL. In still other embodiments, the oligonucleotide is administered so that the plasma trough AUC is about 2 μg·hr/mL, about 3 μg·hr/mL, about 4 μg·hr/mL, about 5 μg·hr/mL, about 6 μg·hr/mL, about 7 μg·hr/mL, about 8 μg·hr/mL, about 9 μg·hr/mL, about 10 μg·hr/mL, about 11 μg·hr/mL, about 12 μg·hr/mL, about 13 μg·hr/mL, about 14 μg·hr/mL, about 15 μg·hr/mL, about 16 μg·hr/mL, about 17 μg·hr/mL, about 18 μg·hr/mL, about 19 μg·hr/mL or about 20 μg·hr/mL. In one embodiment, the subject is treated by weekly 200 mg maintenance doses of the oligonucleotide, and the oligonucleotide is ISIS 301012. In other embodiments, the subject is treated only once per month with a concentration of the oligonucleotide that is selected to result in the desired plasma trough AUC. Alternatively, the subject can be treated only once every two, or even every three months with a concentration of the oligonucleotide that is selected to result in the desired plasma trough AUC.

Another embodiment of the disclosure relates to the use of ISIS 301012 as a treatment for a human who suffers from, or is at an increased risk for, hypercholesterolemia, wherein the ISIS 301012 oligonucleotide is administered during a loading period and a maintenance period. The doses during the loading period are typically higher, or more frequent, than during the maintenance period, and both the amount and frequency of dosing are selected such that liver tissue levels of ISIS 301012 approach tissue levels that provide therapeutic benefits. Oligonucleotide plasma trough concentrations are in equilibrium with tissue drug concentrations and thus are used as a representation of liver tissue concentrations. For example, a 200 mg dose of ISIS 301012 administered intravenously 3 times during a 1 week loading period achieved a plasma trough concentration of approximately 18 ng/mL. Reductions in serum LDL-cholesterol, serum total cholesterol, and serum apolipoprotein B were observed. The doses during the maintenance period are typically lower or less frequent than during the loading period, and are administered once per week, once every 2 weeks, once per month or once every 3 months. This dose may be equal to or less than the dose administered during the loading period. For example, a 200 mg dose of ISIS 301012 may be administered once per week during the maintenance period.

As used herein, an oligonucleotide may provide a therapeutic benefit to a human subject when a reduction of at least 5%, 10%, 15%, 20%, 30%, 40%, 50%, 60%, 70%, 80% or 90% of serum apolipoprotein B or serum LDL-cholesterol levels is found. However, any reduction in serum apolipoprotein B or LDL-cholesterol levels may be therapeutically beneficial to a subject, so the aforementioned percentage reductions are illustrative, and not limiting, on embodiments. An oligonucleotide may provide a therapeutic benefit to a human subject when serum LDL-cholesterol level is lowered to about 70 mg/dL or less, to about 100 mg/dL or less, or to about 130 mg/dL or less.

As used herein, "suffering from hypercholesterolemia" refers to a human subject who has LDL-cholesterol or total cholesterol levels higher than the recommended LDL-cholesterol or total cholesterol levels as established by the National Cholesterol Education Panel. As used herein, "at risk for hypercholesterolemia" refers to a human subject who exhibits one or more risk factors for coronary heart disease, such as, for example, those risk factors defined by the National Cholesterol Education Panel. As used herein, "in need thereof" is interchangeable with "suffering from hypercholesterolemia" or "at risk for hypercholesterolemia". "Subject" and "human subject" are herein used interchangeably.

Another embodiment of the disclosure relates to lowering the level of apolipoprotein B-containing lipoproteins in a human subject. As used herein, "apolipoprotein B-containing lipoprotein" refers to any lipoprotein that has apolipoprotein B as its protein component, and is understood to include LDL, VLDL, IDL, and lipoprotein(a). LDL, VLDL, IDL and lipoprotein(a) each contain one molecule of apolipoprotein B, thus a serum apolipoprotein B measurement reflects the total number of these lipoproteins. As is known in the art, each of the aforementioned lipoproteins is atherogenic. Thus, lowering one or more apolipoprotein B-containing lipoproteins in serum may provide a therapeutic benefit to a human subject. Small LDL particles are considered to be particularly atherogenic relative to large LDL particles, thus lowering small LDL particles can provide a therapeutic benefit to a human subject. Following treatment with ISIS 301012, levels of serum small LDL particles, serum VLDL-cholesterol, or serum lipoprotein(a) were found to be reduced in humans.

Another embodiment of the disclosure relates to lowering additional lipid parameters in a subject. Following treatment with ISIS 301012, serum triglycerides, LDL:HDL ratio, or total cholesterol:HDL ratio were found to be reduced in humans. Reduction of total cholesterol:HDL ratio or LDL:HDL ratio is a clinically desirable improvement in cholesterol ratio. Similarly, it is clinically desirable to reduce serum triglycerides humans who exhibit elevated lipid levels.

Other embodiments of the disclosure encompass methods of reducing serum LDL-cholesterol in a human by administering a dose of an oligonucleotide that inhibits expression of apolipoprotein B. In this embodiment, the oligonucleotide is administered in a dose that provides a predetermined trough concentration in the human's plasma, wherein the predetermined plasma trough concentration results in a lowered serum LDL-cholesterol level. In one embodiment, the plasma trough concentration ranges from about 5 ng/mL to about 40 ng/mL. In one embodiment, the oligonucleotide is SEQ ID NO: 2. Preferably, the lowered serum LDL-cholesterol level provides a therapeutic benefit to the human. This embodiment encompasses reducing additional lipid parameters, such as serum total cholesterol, serum small LDL particles, serum triglycerides, serum lipoprotein(a), and serum VLDL-cholesterol.

In another embodiment, the oligonucleotide is administered in a dose that provides a predetermined concentration in the human's plasma, wherein the predetermined plasma trough concentration is measured as a plasma trough "area under the curve" (AUC), as detailed more completely below, and wherein the predetermined plasma trough AUC results in a lowered serum LDL-cholesterol level. Such plasma trough AUC range from about 2 μg·hr/mL to about 20 μg·hr/mL. In one embodiment, the oligonucleotide is SEQ ID NO: 2. This embodiment encompasses reducing additional lipid parameters, such as serum small LDL particles, serum total cholesterol, serum triglycerides, serum lipoprotein(a), and serum VLDL-cholesterol.

Still other embodiments of the present disclosure relate to a plurality of doses or one or more pharmaceutical compositions comprising a plurality of doses of an oligonucleotide targeted to apolipoprotein B for reducing serum cholesterol levels. In certain embodiments, the serum cholesterol is serum LDL-cholesterol or serum VLDL-cholesterol. Administration of such plurality of doses to a subject, such as a human, results in a plasma trough AUC for the oligonucleotide of from about 2 μg·hr/mL to about 20 μg·hr/mL, about 2 μg·hr/mL to about 10 μg·hr/mL, and about 4 μg·hr/mL to about 6 μg·hr/mL. In other embodiments, the plasma trough AUC is about 5 μg·hr/mL. In still other embodiments, the administration of the plurality of doses results in a plasma trough AUC for the oligonucleotide of about 2 μg·hr/mL, about 3 μg·hr/mL, about 4 μg·hr/mL, about 5 μg·hr/mL, about 6 μg·hr/mL, about 7 μg·hr/mL, about 8 μg·hr/mL, about 9 μg·hr/mL, about 10 μg·hr/mL, about 11 μg·hr/mL, about 12 μg·hr/mL, about 13 μg·hr/mL, about 14 μg·hr/mL, about 15 μg·hr/mL, about 16 μg-hr/mL, about 17 μg·hr/mL, about 18 μg·hr/mL, about 19 μg·hr/mL or about 20 μg·hr/mL. In any of the foregoing embodiments, the plasma trough AUC is achieved from about 3 to about 33 days after administration of at least one dose of the plurality of doses of the oligonucleotide. In some embodiments, the serum cholesterol is serum LDL-cholesterol. In some preferred embodiments, the oligonucleotide used in the plurality of doses is SEQ ID NO: 2.

In certain other embodiments, administration of a plurality of doses an oligonucleotide targeted to apolipoprotein B results in a plasma trough concentration of about 5 ng/mL to about 40 ng/mL, whereas in other embodiments the plasma trough concentration is about 5 ng/mL to about 20 ng/mL. In still other embodiments the plasma trough concentration is about about 5 ng/mL, about 6 ng/mL, about 7 ng/mL, about 8 ng/mL, about 9 ng/mL, about 10 ng/mL, about 11 ng/mL, about 12 ng/mL, about 13 ng/mL, about 14 ng/mL, about 15 ng/mL, about 16 ng/mL, 17 ng/mL, about 18 ng/mL, about 19 ng/mL, about 20 ng/mL, about 21 ng/mL, about 22 ng/mL, about 23 ng/mL, about 24 ng/mL, about 25 ng/mL, about 26 ng/mL, about 27 ng/mL, about 28 ng/mL, 29 ng/mL, about 30 ng/mL, about 31 ng/mL, about 32 ng/mL, about 33 ng/mL, about 34 ng/mL, about 35 ng/mL, about 36 ng/mL, about 37 ng/mL, about 38 ng/mL, about 39 ng/mL or about 40 ng/mL. In such embodiments, the plasma trough concentration is achieved about 7 days after administration of at least one dose of the plurality of doses of the oligonucleotide. In some preferred embodiments, the oligonucleotide used in the plurality of doses is SEQ ID NO: 2.

Embodiments described herein also relate the use of a plurality of doses of an oligonucleotide targeted to apolipoprotein B for the preparation of a medicament for reducing serum cholesterol levels. Administration such medicament to a subject, such as a human, results in a plasma trough AUC for the oligonucleotide of from about 2 μg·hr/mL to about 20 μg·hr/mL, about 2 μg·hr/mL to about 10 μg·hr/mL, and about 4 μg·hr/mL to about 6 μg·hr/mL. In other embodiments, the plasma trough AUC is about 5 μg·hr/mL. In still other embodiments, the administration of medicament results in a plasma trough AUC for the oligonucleotide of about 2 μg·hr/mL, about 3 μg·hr/mL, about 4 μg·hr/mL, about 5 μg·hr/mL, about 6 μg·hr/mL, about 7 μg·hr/mL, about 8 μg·hr/mL, about 9 μg·hr/mL, about 10 μg·hr/mL, about 11 μg·hr/mL, about 12 μg·hr/mL, about 13 μg·hr/mL, about 14 μg·hr/mL, about 15 μg·hr/mL, about 16 μg·hr/mL, about 17 μg·hr/mL, about 18 μg-hr/mL, about 19 μg-hr/mL or about 20 μg·hr/mL. In any of the foregoing embodiments, the plasma trough AUC is achieved from about 3 to about 33 days after administration of the medicament containing the oligonucleotide. In some preferred embodiments, the oligonucleotide used in the preparation of the medicament is SEQ ID NO: 2.

In certain other embodiments, administration of a plurality of doses an oligonucleotide targeted to apolipoprotein B results in a plasma trough concentration of about 5 ng/mL to about 40 ng/mL, whereas in other embodiments the plasma trough concentration is about 5 ng/mL to about 20 ng/mL. In still other embodiments the plasma trough concentration is about about 5 ng/mL, about 6 ng/mL, about 7 ng/mL, about 8 ng/mL, about 9 ng/mL, about 10 ng/mL, about 11 ng/mL, about 12 ng/mL, about 13 ng/mL, about 14 ng/mL, about 15 ng/mL, about 16 ng/mL, 17 ng/mL, about 18 ng/mL, about 19 ng/mL, about 20 ng/mL, about 21 ng/mL, about 22 ng/mL, about 23 ng/mL, about 24 ng/mL, about 25 ng/mL, about 26 ng/mL, about 27 ng/mL, about 28 ng/mL, 29 ng/mL, about 30 ng/mL, about 31 ng/mL, about 32 ng/mL, about 33 ng/mL, about 34 ng/mL, about 35 ng/mL, about 36 ng/mL, about 37 ng/mL, about 38 ng/mL, about 39 ng/mL or about 40 ng/mL. In such embodiments, the plasma trough concentration is achieved about 7 days after administration of the medicament containing the oligonucleotide. In some preferred embodiments, the oligonucleotide used in the preparation of the medicament is SEQ ID NO: 2.

Still other embodiments of the present disclosure relate to the use of the oligonucleotide of SEQ ID NO: 2 for the preparation of a medicament for reducing serum LDL-cholesterol, wherein administration of the medicament is sufficient to achieve one or a combination of the following pharmacokinetic properties: a plasma trough AUC of at least about 2 μg·hr/mL, a plasma trough concentration of at least about 5 ng/mL or an estimated liver concentration of at least about 10 μg/g of liver.

Additional embodiments described herein relate to the use of an oligonucleotide comprising SEQ ID NO: 2 for the preparation of a medicament for reducing serum LDL-cholesterol, wherein the medicament is administered during a loading period and a maintenance period. For example, in the loading period, the medicament can be administered at about 1 dose per day for up to about 10 days. The medicament may be administered either intravenously or subcutaneously. In some embodiments, the maintenance period comprises administering the medicament at least once about every 3 months. However, during the maintenance, the medicament may be administered more frequently. For example, the medicament can be administered once about every 1 month, once about every two weeks or once about every week. Maintenance doses of the medicament may be administered intravenously or subcutaneously. In some embodiments the loading doses and/or the maintenance doses of the medicament are administered at a rate of about 0.1 mg/kg/day to about 5 mg/kg/day. In certain embodiments, administration occurs at the rate of from about 0.1 mg/kg/day to about 1.2 mg/kg/day. In other embodiments, the medicament is administered in a dose from about 50 mg to about 600 mg per week.

In any of the foregoing embodiments, the oligonucleotide comprising SEQ ID NO: 2 can be ISIS 301012. ISIS 301012 is targeted to human apolipoprotein B mRNA, and is a chimeric oligonucleotide ("gapmer") 20 nucleotides in length, composed of a central "gap" region consisting of ten 2'-deoxynucleotides, which is flanked on both sides (5' and 3' directions) by five-nucleotide "wings". The wings are composed of 2'-O-methoxyethyl (2'-MOE)nucleotides. The internucleoside (backbone) linkages are phosphorothioate (P=S) throughout the oligonucleotide. All cytidine residues are 5-methylcytidines. ISIS 301012 is synthesized using methods described in U.S. Patent Application Serial No. 10/712,795, which is incorporated herein by reference in its entirety. Oligonucleotides

In the context of this invention, the term "oligonucleotide" refers to an oligomer or polymer of ribonucleic acid (RNA) or deoxyribonucleic acid (DNA) or mimetics thereof. Thus, this term includes oligonucleotides composed of naturally-occurring nucleobases, sugars and covalent internucleoside (backbone) linkages (RNA and DNA) as well as oligonucleotides having non-naturally-occurring portions which function similarly (oligonucleotide mimetics). Oligonucleotide mimetics are often preferred over native forms because of desirable properties such as, for example, enhanced cellular uptake, enhanced affinity for nucleic acid target and increased stability in the presence of nucleases. As used herein, the term "modification" includes substitution and/or any change from a starting or natural oligonucleotide.

As is known in the art, a nucleoside is a base-sugar combination. The base portion of the nucleoside is normally a heterocyclic base. The two most common classes of such heterocyclic bases are the purines and the pyrimidines. Nucleotides are nucleosides that further include a phosphate group covalently linked to the sugar portion of the nucleoside. For those nucleosides that include a pentofuranosyl sugar, the phosphate group can be linked to the 2', 3' or 5' hydroxyl moiety of the sugar. In forming oligonucleotides, the phosphate groups covalently link adjacent nucleosides to one another to form a linear polymeric compound. Within the oligonucleotide structure, the phosphate groups are commonly referred to as forming the internucleoside backbone of the oligonucleotide. The normal linkage or backbone of RNA and DNA is a 3' to 5' phosphodiester linkage.

Oligonucleotides include oligonucleotides containing modified backbones or non-natural internucleoside linkages. As used herein, an "oligonucleotide mimetic" or "mimetic" refers to any compound of the invention which is modified from the naturally occurring RNA or DNA nucleic acids.

As defined herein, oligonucleotides having modified backbones include those that retain a phosphorus atom in the backbone and those that do not have a phosphorus atom in the backbone. For the purposes of this specification, and as sometimes referenced in the art, modified oligonucleotides that do not have a phosphorus atom in their internucleoside backbone can also be considered to be oligonucleosides. Modified oligonucleotide backbones include, for example, phosphorothioates. Phosphorothioate linkers provide nuclease stability as well as plasma protein binding characteristics to the oligonucleotide. Nuclease stability is useful for increasing the *in vivo* lifetime of oligonucleotides, while plasma protein binding decreases the rate of first pass clearance of oligonucleotide via renal excretion.

In other oligonucleotide mimetics, the sugar is modified or substituted with novel groups. The base units are maintained for hybridization with an appropriate nucleic acid target compound. Sugar modifications may impart nuclease stability, binding affinity or some other beneficial biological property to the oligonucleotide. One such sugar modification includes 2'-methoxyethoxy (2-O-$CH_2CH_2OCH_3$, also known as 2'-O-(2-methoxyethyl) or 2'-MOE) (Martin et al., Helv. Chim. Acta, 1995, 78, 486-504) i.e., an alkoxyalkoxy group.

Oligonucleotide mimetics may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C). Certain nucleobase substitutions, including 5-methylcytosinse substitutions, are particularly useful for increasing the binding affinity of the oligonucleotides of the invention. For example, 5-methylcytosine substitutions have been shown to increase nucleic acid duplex stability by 0.6-1.2°C (Sanghvi, Y.S., Crooke, S.T. and Lebleu, B., eds., Antisense Research and Applications, CRC Press, Boca Raton, 1993, pp. 276-278) and are presently preferred base substitutions, even more particularly when combined with 2-O-methoxyethyl sugar modifications.

It is not necessary for all positions in a given oligonucleotide or oligonucleotide mimetic to be uniformly modified, and in fact one or more of the aforementioned modifications may be incorporated in a single oligonucleotide or even at a single nucleoside within an oligonucleotide.

Embodiments of the disclosure also include oligonucleotides or mimetics which are chimeric compounds. "Chimeric" oligonucleotides or "chimeras," in the context of this invention, are compounds, particularly oligonucleotides, which contain at least two chemically distinct regions, each made up of at least one monomer unit, i.e., a nucleotide in the case of an oligonucleotide. These oligonucleotides typically contain at least one chemically distinct region wherein the oligonucleotide is modified so as to confer upon the oligonucleotide increased resistance to nuclease degradation, increased cellular uptake, and/or increased binding affinity for the target nucleic acid. An additional chemically distinct region of the oligonucleotide may serve as a substrate for enzymes capable of cleaving RNA:DNA or RNA:RNA hybrids. By way of example, RNase H is a cellular endonuclease which cleaves the RNA strand of an RNA:DNA duplex. Modifications which activate, recruit or trigger RNase H and result in cleavage of the RNA target thereby greatly enhance the efficiency of the oligonucleotide for inhibition of gene expression. Consequently, comparable results can often be obtained with shorter oligonucleotides when chimeric oligonucleotides incorporating modifications which facilitate duplex cleavage are used, compared to phosphorothioate deoxyoligonucleotides hybridizing to the same target region. Cleavage of the RNA target can be routinely detected by gel electrophoresis and, if necessary, associated nucleic acid hybridization techniques known in the art.

Chimeric oligonucleotides of the invention may be formed as composite structures of two or more oligonucleotides, modified oligonucleotides, oligonucleosides and/or oligonucleotide mimetics as described above. Chimeric oligonucleotides can be of several different types. These include a first type wherein the "gap" segment of linked nucleosides is positioned between 5' and 3' "wing" segments of linked nucleosides and a second "open end" type wherein the "gap" segment is located at either the 3' or the 5' terminus of the chimeric oligonucleotide. Compounds of the first type are also known in the art as "gapmers" or gapped oligonucleotides. Compounds of the second type are also known in the art as "hemimers" or "wingmers". Such compounds have also been referred to in the art as hybrids.

In a gapmer that is 20 nucleotides in length, a gap or wing can be 1, 2, 3, 4, 5, 6, 7, 8, 9, 10, 11, 12, 13, 14, 15, 16, 17 or 18 nucleotides in length. In one embodiment, a 20-nucleotide gapmer is comprised of a gap 8 nucleotides in length, flanked on both the 5' and 3' sides by wings 6 nucleotides in length. In another embodiment, a 20-nucleotide gapmer is comprised of a gap 10 nucleotides in length, flanked on both the 5' and 3' sides by wings 5 nucleotides in length. In another embodiment, a 20-nucleotide gapmer is comprised of a gap 12 nucleotides in length flanked on both the 5' and 3' sides by wings 4 nucleotides in length. In a further embodiment, a 20-nucleotide gapmer is comprised of a gap 14 nucleotides in length flanked on both the 5' and 3' sides by wings 3 nucleotides in length. In another embodiment, a 20-nucleotide gapmer is comprised of a gap 16 nucleotides in length flanked on both the 5' and 3' sides by wings 2 nucleotides in length. In a further embodiment, a 20-nucleotide gapmer is comprised of a gap 18 nucleotides in length flanked on both the 5' and 3' ends by wings 1 nucleotide in length. Alternatively, the wings are of different lengths, for example, a 20-nucleotide gapmer may be comprised of a gap 10 nucleotides in length, flanked by a 6-nucleotide wing on one side (5' or 3') and a 4-nucleotide wing on the other side (5' or 3').

In a hemimer, an "open end" chimeric oligonucleotide having two chemically distinct regions, a first chemically distinct region, or the gap segment, in a compound 20 nucleotides in length can be located at the 5' terminus of the oligonucleotide and can be 1, 2, 3, 4, 5, 6, 7, 8, 9, 10, 11, 12, 13, 14, 15, 16, 17, 18 or 19 nucleotides in length. Furthermore, a second chemically distinct region in a compound 20 nucleotides in length can be located at the 3' terminus of the oligonucleotide and can be 1, 2, 3, 4, 5, 6, 7, 8, 9, 10, 11, 12, 13, 14, 15, 16, 17, 18 or 19 nucleotides in length. For example, a 20-nucleotide hemimer can have a first chemically distinct region, or a gap segment, of 10 nucleotides at the 5' end and a second chemically distinct region of 10 nucleotides at the 3' end.

Representative United States patents that teach the preparation of such hybrid structures include, but are not limited to, U.S.: 5,013,830; 5,149,797; 5,220,007; 5,256,775; 5,366,878; 5,403,711; 5,491,133; 5,565,350; 5,623,065; 5,652,355; 5,652,356; and 5,700,922, each of which is herein incorporated by reference in its entirety.

The oligonucleotides comprise from about 14 to about 30 nucleobases (i.e. from about 14 to about 30 linked nucleosides). One having ordinary skill in the art will appreciate that this embodies oligonucleotides having 14, 15, 16, 17, 18, 19, 20, 21, 22, 23, 24, 25, 26, 27, 28, 29 or 30 nucleobases. Embodiments of the invention include oligonucleotides having 1, 2 or 3 nucleobases added to or removed from either terminus of an oligonucleotide about 14 to 30 nucleobases in length, wherein the oligonucleotides do not lose their therapeutic effectiveness.

In a further embodiment, the oligonucleotides comprise from about 20 to about 30 nucleobases. One having ordinary skill in the art will appreciate that this embodies oligonucleotides having 20, 21, 22, 23, 24, 25, 26, 27, 28, 29 or 30 nucleobases. Embodiments of the disclosure include oligonucleotides having 1, 2 or 3 nucleobases added to or removed from either terminus of an oligonucleotide about 20 to 30 nucleobases in length, wherein the oligonucleotides do not lose their therapeutic effectiveness.

The oligonucleotides may be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed. It is well known to use similar techniques to prepare oligonucleotides such as the phosphorothioates and alkylated derivatives.

The oligonucleotides described herein, particularly ISIS 301012 and ISIS 301012 related oligonucleotides, encompass any pharmaceutically acceptable salts, esters, or salts of such esters, or any other compound which, upon administration to a human, is capable of providing (directly or indirectly) the biologically active metabolite or residue of the administered oligonucleotide. Accordingly, for example, the disclosure is also drawn to prodrugs and pharmaceutically acceptable salts of the oligonucleotides, pharmaceutically acceptable salts of such prodrugs, and other bioequivalents. As used herein, "bioequivalence" means the absence of a significant difference in the rate and extent to which the active ingredient in pharmaceutical equivalents becomes available at the site of drug action when administered at the same molar dose under similar conditions. As used herein, "ISIS 301012 related oligonucleotides" include oligonucleotides having the sequence of ISIS 301012 which are truncated in 1 or 2 base increments from the 5' and/or 3' end.

The term "prodrug" indicates a therapeutic agent that is prepared in an inactive form that is converted to an active form (i.e., drug) within the body, or cells thereof, by the action of endogenous enzymes or other chemicals and/or conditions. In particular, prodrug versions of the oligonucleotides of the invention are prepared as SATE [(S-acetyl-2-thioethyl)phosphate] derivatives according to the methods disclosed in WO 93/24510 to Gosselin et al., published December 9, 1993 or in WO 94/26764 and U.S. 5,770,713 to Imbach et al.

The term "pharmaceutically acceptable salts" refers to physiologically and pharmaceutically acceptable salts of the oligonucleotides of the invention: i.e., salts that retain the desired biological activity of the parent compound and do not impart undesired toxicological effects thereto.

For oligonucleotides, preferred examples of pharmaceutically acceptable salts include but are not limited to (a) salts formed with cations such as sodium, potassium, ammonium, magnesium, calcium, polyamines such as spermine and spermidine, etc.; (b) acid addition salts formed with inorganic acids, for example hydrochloric acid, hydrobromic acid, sulfuric acid, phosphoric acid, nitric acid and the like; (c) salts formed with organic acids such as, for example, capric acid, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, naphthalenesulfonic acid, methanesulfonic acid, p-toluenesulfonic acid, naphthalenedisulfonic acid, polygalacturonic acid, and the like; and (d) salts formed from elemental anions such as chlorine, bromine, and iodine.

## Clinical Indications

Disclosed are methods of treating, preventing or ameliorating conditions associated with elevated serum LDL-cholesterol, comprising administering to a human subject a therapeutically or prophylatically effective amount of an oligonucleotide that inhibits the expression of apolipoprotein B. One such compound is ISIS 301012. Such conditions include homozygous familial hypercholesterolemia, heterozygous familial hypercholesterolemia, non-familial hypercholesterolemia, familial combined hypercholesterolemia, and familial defective apolipoprotein B. As used herein, the term "therapeutically effective amount" means a dose that, when administered to a human, lowers one or more lipid parameters including serum LDL-cholesterol, serum apolipoprotein B, serum VLDL-cholesterol, serum total cholesterol, serum lipoprotein(a), serum triglycerides, serum total cholesterol:HDL ratio, or serum LDL:HDL ratio. As used herein, the term "prophylactically effective amount" means a dose that, when administered to a human, prevents an elevation in one or more lipid parameters including serum LDL-cholesterol, serum apolipoprotein B, serum VLDL-cholesterol, serum total cholesterol, serum lipoprotein(a), serum triglycerides, serum total cholesterol:HDL ratio, or serum LDL:HDL ratio.

In a further embodiment, the methods encompass methods for treating, preventing or ameliorating conditions associated with elevated serum LDL-cholesterol in a human subject previously unsuccessfully treated by lipid-lowering agents. In one embodiment, the human subject was previously unsuccessfully treated by a statin. It understood that such subjects include homozygous familial hypercholesterolemic subjects, who do not respond to a statin or exhibit a limited response to a statin, owing to insufficient LDL-receptor activity. Thus, homozygous familial hypercholesterolemics, as well as the heterozygous hypercholesterolemics, often do not achieve clinically desirable lipid levels of, for example, serum total cholesterol or serum LDL-cholesterol. One having skill in the art will further understand that subjects previously unsuccessfully treated by lipid-lowering therapeutic agents include those who are intolerant to certain lipid-lowering agents. For example, those who are experience severe side effects following treatment with a statin, e.g. rhabdomyolysis, are intolerant to a statin and consequently do not achieve clinically desirable lipid levels with statin therapy. Additional subjects include those who experience adverse effects following administration of current lipid-lowering agents, for example, myopathy, fatigue or central nervous system effects (e.g. insomnia) following treatment with a statin, and likewise do not achieve clinically desirable lipid levels with statin therapy. In one embodiment, human subjects previously unsuccessfully treated by a statin are given doses of ISIS 301012 that provide therapeutic benefits, such as lowered serum apolipoprotein B, lowered serum LDL-cholesterol or lowered serum total cholesterol.

As used herein, "previously unsuccessfully treated by a statin" is understood to include human subjects who is not achieving clinically desirable lipid levels (e.g., serum LDL-cholesterol, serum VLDL-cholesterol, serum total cholesterol) due to one or more of the following: insufficient LDL-receptor activity; intolerance to a statin; adverse effects following treatment with a statin; or poor adherence to clinically recommended statin therapy.

As used herein, a human subject who has "insufficient LDL-receptor activity" is understood to include a subject who meets one or more of the following criteria: genetic testing confirming 2 mutated LDL-receptor genes; document history of untreated serum LDL-cholesterol greater than 500 mg/dL; tendinous and/or cutaneous xanthoma prior to age 10 years; or, both parents have documented elevated serum LDL-cholesterol prior to lipid-lowering therapy consistent with heterozygous familial hypercholesterolemia. In some embodiments, the subject has been diagnosed with homozygous or heterozygous familial hypercholesterolemia.

With respect to some aspects, disclosed are methods for treating, preventing or ameliorating apolipoprotein B-related disorders. As used herein, "apolipoprotein B-related disorder" indicates conditions or diseases that are associated with elevated serum apolipoprotein B, and include familial hypercholesterolemia, familial defective apoB-100, familial combined hypercholesterolemia, nonfamilial (or polygenic) hypercholesterolemia, hypertriglyceridemia, metabolic syndrome, and Type 2 diabetes.

With respect to some aspects, disclosed are methods for treating human subjects exhibiting elevated levels of small LDL particles, such subjects having more LDL-cholesterol carried in small LDL particles as relative to large LDL particles, comprising administering to a subject a therapeutically or prophylatically effective amount of an oligonucleotide that inhibits the expression of apolipoprotein B. One such compound is ISIS 301012. The total levels of LDL-cholesterol in such subjects may or may not be elevated.

Some embodiments relates to a method of using an oligonucleotide comprising the nucleobase sequence "GCCTCAGTCTGCTTCGCACC" (SEQ ID NO: 2) in a treatment for reducing serum LDL-cholesterol in a human subject. The method comprises informing the human subject that the administration of said oligonucleotide results in a plasma trough AUC of at least about 2 μg·hr/mL, results in a plasma trough concentration of at least about 5 ng/mL or results in an estimated liver concentration of said oligonucleotide of at least about 10 μg/g. In some embodiments, the oligonucleotide comprises ISIS 301012.

As used herein, "informing" refers to providing information relating to the pharmacologic, pharmacokinetic and/or pharmacodynamic activities of an oligonucleotide. The act of informing can be performed, for example, by providing a verbal description or by providing printed matter. In instances where printed matter is used, the printed matter may provide, for example, information relating to effects of the oligonucleotide on serum LDL-cholesterol, serum VLDL-cholesterol, serum total cholesterol, serum small LDL particles, serum total cholesterol:HDL ratio or serum LDL:HDL ratio. The printed matter may further provide information relating to the pharmacokinetic profile of an oligonucleotide, such as, for example, plasma trough AUC, plasma trough concentrations, elimination half-life, estimated tissue concentrations, or $C_{max}$. In one embodiment, the printed matter provides information relating to the pharmacodynamic and pharmacokinetic effects of ISIS 301012. As used herein, "informing" does not require any more than the mere act of providing the information. It is not required that intended recipients of the information accept, acknowledge receipt of or understand the information.

As used herein, "label" refers to printed matter that is associated with a container for holding the oligonucleotides and/or pharmaceutical compositions that include the oligonucleotides described herein. By way of non-limiting example, the label and container can be placed together in a box or shrink wrap. Alternatively, the label can be attached directly to the container. In other embodiments, the label need not be physically associated with or in physical proximity with the container, however, the label should be provided at the same time or at a time reasonably near to the time of providing the container.

Pharmaceutical Compositions

Pharmaceutical compositions comprising oligonucleotides (e.g. the pharmaceutical composition comprising ISIS 301012 or ISIS 301012 related olignucleotides), may be administered in a number of ways, including parenterally. Parenteral administration is understood to include intravenous, intraarterial, subcutaneous, intraperitoneal or intramuscular injection or infusion. In one aspect, administration of oligonucleotides such as ISIS 301012 to a human subject is performed by a health professional. In another aspect, administration of oligonucleotides is performed by a trained designee, such as, for example, the subject. In one aspect, an oligonucleotide is administered subcutaneously as a single injection into the abdomen or thigh. Alternatively, the dose is divided and administered as 2 or 3 non-contiguous injections into the abdomen or thigh.

Compositions and formulations for parenteral administration may include sterile aqueous solutions, which may also contain buffers, diluents and other suitable additives such as, but not limited to, penetration enhancers, carrier compounds and other pharmaceutically acceptable carriers or excipients. Liquid pharmaceutical compositions of oligonucleotide can be prepared by combining the oligonucleotide with a suitable vehicle, for example sterile pyrogen free water, or saline solution. Sterile pyrogen free water is understood to include sterile water for injection.

The pharmaceutical formulations, which may conveniently be presented in unit dosage form, may be prepared according to conventional techniques well known in the pharmaceutical industry. Such techniques include the step of bringing into association the active ingredients with the pharmaceutical carrier(s) or excipient(s). In general the formulations are prepared by bringing into association the active ingredients with liquid carriers or finely divided solid carriers or both, and then, if necessary, shaping the product.

The pharmaceutical compositions may also be formulated as suspensions in aqueous, non-aqueous or mixed media. The pharmaceutical compositions may include physiologically compatible buffers, including, for example, phosphate-buffered saline (PBS). Aqueous suspensions may further contain substances which increase the viscosity of the suspension including, for example, sodium carboxymethylcellulose, sorbitol and/or dextran. The suspension may also contain stabilizers.

A "pharmaceutical carrier" or "excipient" is a pharmaceutically acceptable solvent, suspending agent or any other pharmacologically inert vehicle for delivering one or more oligonucleotides to an animal. The excipient may be liquid or solid and is selected, with the planned manner of administration in mind, so as to provide for the desired bulk, consistency, *etc.,* when combined with an oligonucleotide and any other components of a given pharmaceutical composition. Suitable pharmaceutically acceptable carriers include, but are not limited to, water, salt solutions, alcohols, polyethylene glycols, gelatin, lactose, amylose, magnesium stearate, talc, silicic acid, viscous paraffin, hydroxymethylcellulose, polyvinylpyrrolidone and the like.

The pharmaceutical compositions encompass several active drug products. In one aspect, an active drug product is a sterile oligonucleotide solution in water for injection that may be administered as a subcutaneous injection or as an intravenous infusion after dilution into saline. The concentration of the active drug ingredient is 250 mg/mL. This formulation comprises oligonucleotide in water for injection adjusted to pH 7.0-9.0 with acid or base during preparation. The oligonucleotide in water is packaged in a Type I, clear glass vial (ammonium sulfate treated), stoppered with a TEFLON®-coated, bromobutyl rubber closure and sealed with an aluminum FLIP-OFF® overseal. In one embodiment, the sterile oligonucleotide in water for injection solution comprises ISIS 301012.

In a further aspect, an active drug product is sterile lyophilized oligonucleotide that is reconstituted with a suitable diluent, e.g., sterile water for injection. The reconstituted product is administered as a subcutaneous injection or as an intravenous infusion after dilution into saline. The lyophilized drug product consists of the oligonucleotide which has been prepared in water for injection, adjusted to pH 7.0-9.0 with acid or base during preparation, and then lyophilized. The lyophilized drug product may be 50-125 mg of the oligonucleotide. It is understood that this encompasses 50, 75, 100 and 125 mg of lyophilized oligonucleotide. The lyophilized drug product may be packaged in a 2 mL Type I, clear glass vial (ammonium sulfate-treated), stoppered with a bromobutyl rubber closure and sealed with an aluminum FLIP-OFF® overseal. In one embodiment, the lyophilized drug product comprises ISIS 301012.

The compositions may additionally contain other adjunct components conventionally found in pharmaceutical compositions, at their art-established usage levels. Thus, for example, the compositions may contain additional, compatible, pharmaceutically-active materials such as, for example, antipruritics, astringents, local anesthetics or anti-inflammatory agents, or may contain additional materials useful in physically formulating various dosage forms of the compositions, such as dyes, flavoring agents, preservatives, antioxidants, opacifiers, thickening agents and stabilizers. However, such materials, when added, should not unduly interfere with the biological activities of the components of the compositions. The formulations can be sterilized and, if desired, mixed with auxiliary agents, e.g., lubricants, preservatives, stabilizers, wetting agents, emulsifiers, salts for influencing osmotic pressure, buffers, colorings, flavorings and/or aromatic substances and the like which do not deleteriously interact with the oligonucleotide(s) of the formulation.

Dosing

As used herein, a "dose" refers to the amount of drug given to a human subject in one day; e.g. by intravenous or subcutaneous administration, in a single administration or divided into multiple administrations. A preferred dose range for a typical 70 kg subject is about 25-800 mg. More preferred ranges include about 25-600 mg, about 25-400 mg, about 25-200 mg, about 50-600 mg, about 50-400 mg, or about 50-200 mg in a day. Additional ranges include about 0.1-5 mg/kg, about 0.5-3 mg/kg, about 0.5-8 mg/kg, about 0.25-3 mg/kg, or about 0.25-2 mg/kg. As used herein, the amount of drug given as a dose ranges from 50-600 mg per week. It is understood that doses of 50, 100, 150, 200, 250, 300, 350, 400, 450, 500, 550 and 600 mg per week all fall within the range of 50-600 mg/week. As used herein, the terms "patient" and "subject" are interchangeable.

Dosing regimens may include doses during a loading period and/or a maintenance period. During the loading period, which usually or most often occurs at the initiation of therapy and which lasts approximately one week (although it could be more or less, e.g. 3, 4, 5, 6, 7, 8, 9 or 10 days), a single administration may be given or multiple administrations may be given every day, every 2 days, every 3 days, every 4 days, every 5 days, every 6 days, or every week. Alternatively, the loading period may last about 28 days, although it could be more or less, e.g., 11, 12, 13, 14, 15, 16, 17, 18, 19, 20, 21, 22, 23, 24, 25, 26, 27, 28, 29, 30, 31, 32, 33, 34 or 35 days, and a single administration may be given every day, every 2 days, every 3 days, every 4 days, every 5 days, every 6 days, or every 7 days. During a maintenance period, which follows the loading period and may last for a number of years or the duration of the lifetime of the subject, doses may be given at a frequency ranging from every day to every 3 months, which is understood to include every day, every 2 days, every 3 days, every 4 days, every 5 days, every 6 days, every week, every 2 weeks, every 3 weeks, every 4 weeks, every month, every 2 months, or every 3 months. Maintenance period doses are typically, but not always, lower than loading period doses, and may range from 10-50 mg/day. Lowered lipid levels, such as serum apolipoprotein B, serum total cholesterol, serum LDL-cholesterol, serum VLDL-cholesterol, serum lipoprotein(a), serum LDL:HDL ratio and/or serum cholesterol:HDL ratio can be maintained for at least 2, 3, 4, 5, 6, 7 or 8 weeks (or 1, 2, 3, or 4 months) after a dose of the drug.

An alternative dosing regimen may include doses administered during a maintenance period, without a preceding loading period. Doses may be given at a frequency ranging from every day to every three months, which is understood to include every day, every 2 days, every 3 days, every 4 days, every 5 days, every 6 days, every week, every 2 weeks, every 3 weeks, every 4 weeks, every month, every 2 months, or every 3 months.

The term "bioavailability" refers to a measurement of that portion of an administered drug which reaches the circulatory system (e.g. blood, especially blood plasma) when a particular mode of administration is used to deliver the drug. For example, when a subcutaneous mode of administration is used to introduce the drug into a human subject, the bioavailability for that mode of administration may be compared to a different mode of administration (e.g. an intravenous mode of administration) and extrapolations made to facilitate determination of the proper therapy. In general, bioavailability can be assessed by measuring the area under the curve (AUC) or the maximum serum or plasma concentration ($C_{max}$) of the unchanged form of a drug following administration of the drug to a human subject. AUC is a determination of the Area Under the Curve plotting the serum or plasma concentration of a drug along the ordinate (Y-axis) against time along the abscissa (X-axis). Generally, the AUC for a particular drug can be calculated using methods known to those of ordinary skill in the art and as described in G. S. Banker, Modern Pharmaceutics, Drugs and the Pharmaceutical Sciences, 4th Ed, (May 2002). In some embodiments, the area under a drug's blood plasma concentration curve ($AUC_{sc}$) after subcutaneous administration may be divided by the area under the drug's plasma concentration curve after intravenous administration ($AUC_{iv}$) to provide a dimensionless quotient (relative bioavailability, RB) that represents fraction of drug absorbed via the subcutaneous route as compared to the intravenous route.

As used herein, "AUC" or "$AUC_{0-\infty}$"indicates the area under the concentration-time curve from time 0 (at or prior to oligonucleotide administration) to infinity. "$AUC_{0-48}$" refers to the area under the concentration-time curve from time 0 (at or prior to oligonucleotide administration) to 48 hours following oligonucleotide administration. In one embodiment, the oligonucleotide is ISIS 301012.

A trough measurement is determined at a time after dosing when drug concentrations in plasma are in equilibrium with drug concentrations in tissue, and when drug concentrations are no longer affected by absorption or clearance functions, such as distribution of an injected dose to various tissues. For example, in the case of oligonucleotides such as ISIS 301012, trough calculations are made at least 3 days following administration of the oligonucleotide to a human subject. As used herein, "$C_{trough}$" or "plasma trough concentration" refers to a minimum plasma concentration when plasma oligonucleotide concentrations are in equilibrium with tissue oligonucleotide concentrations. As used herein, "$AUC_{trough}$" or "plasma trough AUC" indicates the area under the concentration-time curve at a time when plasma oligonucleotide concentrations are in equilibrium with tissue oligonucleotide concentrations. In one embodiment, the oligonucleotide is ISIS 301012. The half-life of a drug can be calculated using methods known to those of ordinary skill in the art. Oligonucleotide half-life is determined during the distribution phase or elimination phase following administration of the oligonucleotide. The apparent distribution half-life is calculated using log-linear regression of oligonucleotide concentrations in plasma during the distribution phase. By way of example, distribution phase for an oligonucleotide may last for up to 3 days following administration of the oligonucleotide to a human subject. As used herein, "terminal elimination half-life" or "elimination half-life" represents the time at which approximately 50% of the administered oligonucleotide is cleared from tissues, and is calculated using log-linear regression of oligonucleotide concentrations in plasma during the terminal elimination phase of administration.

One having ordinary skill in the art will understand that for oligonucleotides, plasma AUC, $C_{max}$, $C_{trough}$, and related parameters are measured in the plasma fraction of blood, rather than in the serum fraction, as the plasma fraction measurement reflects the more clinically relevant amount of oligonucleotide bound to and carried by plasma proteins. Oligonucleotide concentrations in plasma may be determined by methods routine in the art, for example, by hybridization-based ELISA. It is likewise understood that lipid parameters, including apolipoprotein B, LDL-cholesterol, total cholesterol, VLDL-cholesterol, lipoprotein(a), HDL-cholesterol, or triglycerides, are measured in the serum or plasma fraction of blood.

In general, bioavailability correlates with therapeutic efficacy when a compound's therapeutic efficacy is related to the blood (or plasma) concentration achieved, even if the drug's ultimate site of action is intracellular (van Berge-Henegouwen et al., Gastroenterol., 1977, 73, 300). Therapeutic efficacy of ISIS 301012 is determined by comparing the plasma concentration of ISIS 301012 to, for example, reductions in serum LDL-cholesterol.

Organ bioavailability refers to the concentration of an oligonucleotide in an organ. Organ bioavailability may be measured in human subjects by a number of means, such as by whole-body radiography. Organ bioavailability may be modified, e.g. enhanced, by one or more modifications to an oligonucleotide, by use of one or more carrier compounds or excipients, etc. as discussed in more detail herein. In general, an increase in bioavailability will result in an increase in organ bioavailability. Oligonucleotide plasma trough concentrations, including ISIS 301012 plasma trough concentrations, are in equilibrium with tissue drug concentrations and thus are used as a representation of liver tissue concentrations.

The effects of treatments with oligonucleotides such as ISIS 301012 can be assessed following collection of tissues or fluids from a human subject receiving said treatments. It is known in the art that a biopsy sample can be procured from certain tissues without resulting in detrimental effects to a subject. In certain embodiments, a tissue and its constituent cells comprise, but are not limited to, blood (e.g., hematopoietic cells, such as human hematopoietic progenitor cells, human hematopoietic stem cells, $CD34^+$ cells $CD4^+$ cells), lymphocytes and other blood lineage cells, bone marrow, breast, cervix, colon, esophagus, lymph node, muscle, peripheral blood, oral mucosa and skin. In other embodiments, a fluid and its constituent cells comprise, but are not limited to, blood, urine, semen, synovial fluid, lymphatic fluid and cerebro-spinal fluid.

Tissues or fluids from subjects can be evaluated for expression levels of the target mRNA or protein. Additionally, the mRNA or protein expression levels of other genes known or suspected to be associated with the specific disease state, condition or phenotype can be assessed. mRNA levels can be measured or evaluated by real-time PCR, Northern blot, *in situ* hybridization or DNA array analysis. Protein levels can be measured or evaluated by ELISA, immunoblotting, quantitative protein assays, protein activity assays (for example, caspase activity assays) immunohistochemistry or immunocytochemistry.

Furthermore, the effects of treatment can be assessed by measuring lipid parameters, as described herein (cholesterol, lipoproteins and triglycerides, etc.), or biomarkers associated with the disease or condition in the aforementioned tissues and fluids, collected from a patient or subject receiving treatment, by routine clinical methods known in the art. These biomarkers include but are not limited to: glucose, free fatty acids and other markers of glucose and lipid metabolism;; liver transaminases, bilirubin, albumin, blood urea nitrogen, creatine and other markers of kidney and liver function; interleukins, tumor necrosis factors, intracellular adhesion molecules, C-reactive protein and other markers of inflammation; testosterone, estrogen and other hormones; tumor markers; vitamins, minerals and electrolytes.

In addition, nuclear magnetic resonance (NMR) spectroscopy is used to determine the concentrations of blood lipoprotein particles, including VLDL particles (total, large/chylomicron, medium and small), LDL particles (total, large, small, medium-small and very small) and HDL particles (large, medium and small). Such measurements of lipoprotein particle size allow for the determination of LDL-cholesterol, VLDL-cholesterol and HDL-cholesterol subclasses, for example the fraction of LDL-cholesterol that is carried in small LDL particles versus large LDL particles.

In situ measurements of lipid parameters in tissues and fluids may also be performed using whole body imaging techniques, including ultrasound, computed tomography (CT) scan, or magnetic resonance imaging.

While the present invention has been described with specificity in accordance with certain embodiments, the following examples serve only to illustrate the invention and are not intended to limit the same.

EXAMPLES

Example 1

Antisense inhibition of human apolipoprotein B expression by a chimeric phosphorothioate oligonucleotide having 2'-MOE wings and a deoxy gap (ISIS 301012)

An oligonucleotide was designed to target human apolipoprotein B RNA, using a published sequence (GenBank accession number NM_000384.1, incorporated herein as SEQ ID NO: 1) and is referred to hereinafter as ISIS 301012 (GCCTCAGTCTGCTTCGCACC; SEQ ID NO: 2). ISIS 301012 is a chimeric oligonucleotide synthesized using methods as described in WO 2004044181, which is incorporated herein by reference in its entirety. The chimeric ISIS 301012 oligonucleotide is a "gapmer" 20 nucleotides in length, composed of a central "gap" region consisting of ten 2'-deoxynucleotides, and flanked on both sides (5' and 3' directions) by five-nucleotide "wings". The wings are composed of 2'-O-methoxyethyl (2'-MOE) nucleotides. ISIS 301012 is shown in Table 1, where, in the nucleotide sequence 2'-deoxynucleotides (nucleobases 6-15) are indicated by plain type and 2'-MOE nucleotides (nucleobases 1-5 and 16-20) are indicated by emboldened, underlined type. The internucleoside (backbone) linkages are phosphorothioate (P=S) throughout the oligonucleotide. All cytidine residues are 5-methylcytidines.

ISIS 301012 reduced human apolipoprotein B in vitro and in vivo. In Table 1, "Target site" indicates the first (5'-most) nucleotide number on the particular target sequence to which the oligonucleotide binds.

**Table 1**

| ISIS 301012 Target Site and Sequence | | | | | |
|---|---|---|---|---|---|
| **ISIS #** | **REGION** | **TARGET SEQ ID NO** | **TARGET SITE** | **SEQUENCE** | **SEQ ID NO** |
| 301012 | Coding Region | 2 | 3249 | **GCCTC**AGTCTGCTTC**GCACC** | 2 |

Example 2

Effects of apolipoprotein B antisense inhibition in Cynomolgus monkeys

Cynomolgus monkeys (male or female) were used to evaluate antisense oligonucleotides for their potential to lower apolipoprotein B mRNA or protein levels *in vivo,* as well as to evaluate phenotypic endpoints associated with apolipoprotein B expression. As part of this example, LDL-cholesterol and total cholesterol levels of the treated monkeys were determined.

ISIS 301012 in lean Cynomolgus monkeys

The oligonucleotide ISIS 301012 was investigated in a long-term study for its effects on apolipoprotein B expression and serum lipid levels in Cynomolgus monkeys.

Male and female Cynomolgus monkeys were treated with 2, 4 or 12 mg/kg of ISIS 301012 intravenously, or 2 or 20 mg/kg subcutaneously, at a frequency of every two days for the first week, and every 4 days thereafter, for 1 and 3 month treatment periods. Saline-treated animals served as negative controls. Each treatment group included 2 to 3 animals of each sex.

At a one month interval and at the 3 month study termination, the animals were sacrificed and evaluated for apolipoprotein B expression in liver, lipid levels in serum and indicators of toxicity. RNA was isolated from liver tissue and apolipoprotein B mRNA expression was measured by real-time PCR as described in U.S. Application Serial No. 10/712,795, which is herein incorporated by reference in its entirety. Serum lipids, including total cholesterol, LDL-cholesterol, HDL-cholesterol and triglycerides, were evaluated by routine clinical analysis, e.g., using an Olympus Clinical Analyzer (Olympus America Inc., Melville, NY). Ratios of LDL-cholesterol to HDL-cholesterol and total cholesterol to HDL-cholesterol were also calculated. Analyses of serum alanine aminotransferase (ALT) and serum asparate aminotransferase (AST), inflammatory infiltrates in tissue and basophilic granules in tissue provided an assessment of toxicities related to the treatment. Hepatic steatosis, or accumulation of lipids in the liver, was assessed by routine histological analysis with oil red O stain and measurement of liver tissue triglycerides using a Triglyceride GPO Assay (Sigma-Aldrich, St. Louis, MO).

The results from the one month interval of the long term treatment are shown in Table 2 and were normalized to saline-treated animals for mRNA and to untreated baseline values for lipid levels. Total cholesterol, LDL-cholesterol, HDL-cholesterol, LDL particle concentration and triglyceride levels in serum were measured by nuclear magnetic resonance spectroscopy by Liposcience (Raleigh, NC). Additionally, the concentration of intact oligonucleotide in liver was measured by capillary gel electrophoresis and is presented as micrograms of oligonucleotide per gram of liver tissue. Each result represents the average of data from 4 animals (2 males and 2 females). Where present, "N.D." indicates "not determined."

**Table 2**

| Effects of ISIS 301012 in lean Cynomolgus monkeys after 4 weeks of treatment | | | | | | |
|---|---|---|---|---|---|---|
| | | Intravenous delivery | | | Subcutaneous injection | |
| | | 2 mg/kg | 4 mg/kg | 12 mg/kg | 3.5 mg/kg | 20 mg/kg |
| apolipoprotein B expression (% change normalized to saline) | | -45 | -76 | -96 | N.D. | -94 |
| antisense oligonucleotide concentration in liver (μg/g) | | 92 | 179 | 550 | N.D. | 855 |
| | | | | | | |
| Lipid Parameters (% change normalized to untreated baseline value) | Saline | 2 mg/kg | 4 mg/kg | 12 mg/kg | 3.5 mg/kg | 20 mg/kg |
| Total cholesterol | +1 | -6 | -2 | -2 | +5 | -5 |
| LDL-cholesterol | +17 | +15 | +9 | +3 | -4 | -16 |
| HDL-cholesterol | -11 | -23 | -15 | -8 | +13 | +5 |
| LDL/HDL | +62 | +94 | +38 | +44 | -15 | -19 |
| Total cholesterol/HDL | +30 | +44 | +22 | +21 | -7 | -10 |
| Triglyceride | +37 | +26 | +32 | +15 | +1 | -3 |
| LDL Particle concentration | +15 | +8 | +8 | -11 | -14 | -21 |

These data show that ISIS 301012 inhibited apolipoprotein B expression in a dose-dependent manner in a primate species and concomitantly lowered lipid levels at higher doses of ISIS 301012. Furthermore, these results demonstrate that ISIS 301012 accumulated in the liver in a dose-dependent manner.

Following 13 weeks of treatment with a 2 mg/kg intravenous dose of ISIS 301012 or a 20 mg/kg subcutaneous dose of ISIS 301012, total cholesterol, LDL-cholesterol, HDL-cholesterol, LDL particle concentration and triglyceride levels in serum were measured by nuclear magnetic resonance spectroscopy by LipoScience, Inc. (Raleigh, NC). These data are shown in Table 3 and are normalized to untreated baseline values. Each result represents the average of data from 4 animals (2 males and 2 females).

**Table 3**

| Effects of ISIS 301012 in lean Cynomolgus monkeys after 13 weeks of treatment | | | |
|---|---|---|---|
| Lipid parameters, % change normalized to untreated baseline value | Saline | 2 mg/kg | 20 mg/kg |
| Total cholesterol | +11 | +7 | +11 |
| LDL-cholesterol | +36 | +4 | -3 |
| HDL-cholesterol | -8 | +18 | +5 |
| LDL/HDL | +64 | -6 | -20 |
| Total cholesterol/HDL | +30 | +5 | -11 |
| Triglyceride | +36 | +5 | +10 |
| LDL Particle concentration | +31 | -3 | -7 |

These data illustrate significantly decreased LDL-cholesterol and total cholesterol/HDL and LDL/HDL ratios following 13 weeks of treatment with ISIS 301012. Furthermore, HDL-cholesterol levels were significantly increased.

Hepatic steatosis, or accumulation of lipids in the liver, was not observed following 4 weeks of treatment with the doses indicated. Expected dose-related toxicities were observed at the higher doses of 12 and 20 mg/kg, including a transient 1.2-1.3 fold increase in activated partial thromboplastin time (APTT) during the first 4 hours and basophilic granules in the liver and kidney (as assessed by routine histological examination of tissue samples). No functional changes in kidney were observed.

In a similar experiment, male and female Cynomolgus monkeys received an intravenous dose of ISIS 301012 at 4 mg/kg, every two days for the first week and every 4 days thereafter. Groups of animals were sacrificed after the first dose and the fourth dose, as well as 11, 15 and 23 days following the fourth and final dose. Liver RNA was isolated and apolipoprotein B mRNA levels were evaluated by real-time PCR as described in U.S. Application Serial No. 10/712,795, which is herein incorporated by reference in its entirety. The results of this experiment demonstrated a 40% reduction in apolipoprotein B mRNA expression after a single intravenous dose of 4 mg/kg ISIS 301012. Furthermore, after 4 doses of ISIS 301012 at 4 mg/kg, apolipoprotein B mRNA was reduced by approximately 85% and a 50% reduction in apolipoprotein B mRNA was sustained for up to 16 days following the cessation of ISIS 301012 treatment.

ISIS 326358 in high-fat fed Cynomolgus monkeys

In a further embodiment, the effects of antisense inhibition of apolipoprotein B in high-fat fed Cynomolgus monkeys were evaluated. ISIS 326358 (GCCTCAGTCTGCTTTACACC; SEQ ID NO: 3) is an oligonucleotide and the Cynomolgus monkey equivalent of ISIS 301012. The ISIS 326356 oligonucleotide differs from ISIS 301012 at 2 nucleobase positions. Like ISIS 301012, ISIS 326358 is a gapmer, having a central gap portion of 10 2'-deoxynucleotides, which is flanked on both sides (5'and 3') by wing portions of 5 2'-MOE nucleotides. All internucleoside linkages are phosphorothioate, and all cytidine residues are 5-methylcytidines.

The pharmacological activity of ISIS 326358 was characterized in Cynomolgus monkeys fed a high-fat diet (approximately 17% lard and approximately 25% cholesterol) for 3 weeks prior to treatment with ISIS 326358, and throughout the study. ISIS 326358 was administered subcutaneously at doses of 2.5, 5, or 17.5 mg/kg for 5 weeks. Doses were given on alternate days for the first 3 doses (loading doses; 7.5, 12.5 or 37.5 mg/kg/week) and twice weekly thereafter (maintenance doses; 5, 10 or 35 mg/kg/week).

The high-fat diet increased serum cholesterol approximately 400 mg/dL, relative to the approximately 150 mg/dL level observed in monkeys receiving a standard diet. Following treatment with ISIS 326358, LDL-cholesterol, liver apolipoprotein B (mRNA and protein), and total cholesterol were significantly reduced as much as 70%, 50% and 50%, respectively. Reductions in LDL-cholesterol and total cholesterol were dose- and time-dependent. No statistically significant changes in apolipoprotein B or lipoproteins were noted in the saline control group, relative to pre-dosing values. No changes in liver transaminases or liver triglyceride levels were noted in monkeys treated with ISIS 326358.

Together, these data demonstrate that antisense inhibition of apolipoprotein B using ISIS 326358 in primates receiving a high-fat diet reduces liver apolipoprotein B expression and significantly decreases serum total cholesterol and LDL-cholesterol.

Example 3

Evaluation of ISIS 301012 in a Phase I Clinical Study

As described below, ISIS 301012 was tested in a double blind, placebo-controlled, Phase I, dose-escalation study for the purpose of evaluating the safety and tolerability of single and multiple doses of ISIS 301012 administered to humans intravenously and subcutaneously. In addition, these studies evaluated the pharmacokinetic profile of single and multiple doses of ISIS 301012 administered intravenously and subcutaneously; and also evaluated the pharmacodynamics of ISIS 301012 administered intravenously and subcutaneously.

For this Example, a solution of ISIS 301012 (250 mg/mL, 0.5 mL) in sterile, unpreserved, buffered saline contained in 2-mL stoppered glass vials was provided. The study drug was stored securely at 2° C to 8°C and protected from light. The placebo was 0.9% sterile saline.

Study Design

Subjects, 18 to 65 years of age with total cholesterol between 200 and 300 mg/dL after an overnight fast and a body mass index (BMI) of less than 30 kg/m2, were randomized into Cohorts to receive ISIS 301012 or placebo in a 3:1 ratio. The dosing cohorts were as follows: Cohort A, 50 mg ISIS 301012 or placebo; Cohort B, 100 mg ISIS 301012 or placebo; Cohort C, 200 mg ISIS 301012 or placebo; Cohort D, 400 mg ISIS 301012 or placebo.

The study consisted of a single dose component (SD) followed by a multiple dose component (MD). In the single dose component, each subject received one subcutaneous dose of study drug, which was followed by a 4 week observation period. Subjects who completed the single dose component and the observation period of the study were continued in the multiple dose component of the study. Additional subjects were recruited for the multiple dose component of the study only. The multiple dose period was following by a post-treatment evaluation period (PD).

During the multiple dose component, subjects from the single dose component of the study continued to receive the study drug (ISIS 301012 or placebo) to which they had previously been randomized. During the first week of the multiple dose treatment period, subjects received three intravenous doses at their respective cohort dose levels on alternate days followed by a single weekly subcutaneous dose for three weeks. This dosing regimen resulted in estimated tissue concentrations that were approximately 70 to 80% of steady state levels.

On Day SD1 (day 1 of single dose period), blood for a lipid panel (total cholesterol, LDL-cholesterol, HDL-cholesterol, VLDL-cholesterol, apolipoprotein B, triglyceride, lipoprotein(a) and high-sensitivity CRP) was collected following an overnight fast (at least 12 hours). These measurements represent baseline measurements. Study drug was administered via a subcutaneous injection (s.c.) with the end of the injection designated as Time 0 (t = zero). Blood samples for pharmacokinetic (PK) analysis were collected at the following timepoints: 0.5, 1, 1.5, 2, 3, 4, 6, 8, and 12 hrs after study drug administration. Urine samples for PK analysis were collected over a 24 hour period, beginning at Time 0 (t = zero) on Day SD1 and ending on Day SD2. On SD4, blood samples were collected for PK analysis and lipid panel analysis.

Individual cohort treatments for the single dose administration period are summarized in Table 4. The subjects in the placebo group receive the same injection volume as in the Cohort to which they were assigned.

**Table 4**

| Single Dose Treatment Period | | |
|---|---|---|
| Total Dose | # Subjects | All SQ injections at 250 mg/mL SD1 |
| Placebo | 7 | According to cohort |
| 50 mg | 8 | 1 injection, 0.2 mL |
| 100 mg | 8 | 1 injection, 0.4 mL |
| 200 mg | 9 | 1 injection, 0.8 mL |
| 400 mg | 4 | 2 injections, 0.8 mL |

During the multiple dose component, study drug was administered intravenously as a 2-hour infusion on Days MD1, MD3 and MD5 of Week 1 and as a subcutaneous injection(s) of no more than 200 mg per injection on Days MD8, MD15 and MD22. All subjects were required to fast for at least 12 hours before the blood sampling for the lipid panel on MD1, MD8, MD15, MD22, MD25, PD14, PD30, and, if applicable, on Days PD44, PD58, PD72, and PD86.

On Day MD1 (day 1 of multiple dose period), study drug was administered via a 2-hour intravenous (i.v.) infusion with the start of the infusion designated Time 0 (t = zero). Blood samples for pharmacokinetic (PK) analysis were collected 0.5, 1, 2, 2.25, 2.5, 3, 4, 6, and 8 hrs after start of study drug infusion. The 2 hour PK sampling was collected just prior to the end of study drug infusion. In addition, a 24-hour urine collection was performed beginning at Time 0 (t = zero) for PK analysis.

On Days MD3 and Day MD5, study drug was administered via intravenous infusion and blood samples for PK analysis were collected 5 minutes prior to the start of study drug infusion and 2 hours after the start of study drug infusion.

On Days MD2, MD4, MD6, blood samples for PK were collected 24 hours after the start of study drug infusion.

On Days MD8, MD15, study drug was administered via subcutaneous injection. Blood samples for PK analysis and urine samples for urinalysis were collected.

On Day MD22, study drug was administered via subcutaneous injection. Blood samples for PK analysis were collected prior to and 0.5, 1, 1.5, 2, 3, 4, 6, 8, and 12 hours after study drug administration. A urine sample for urinalysis was collected over a 24 hour period, beginning at time of dosing on Day MD22 and ending on Day MD23.

On Day MD23, blood samples for PK were collected 24 hours after dosing of the study drug on Day MD22.

On Day MD25, 3 days after the final dose on Day MD22, blood samples were collected for PK analysis.

Shown in Table 5 is a summary of the dosing schedule for the multiple dose period. The 50 mg and 200 mg groups each had one less subject than during the single dose period. The subjects in the placebo group receive the same injection volume as in the Cohort to which they were assigned.

**Table 5**

| Multiple Dose Treatment Period | | | | | | | |
|---|---|---|---|---|---|---|---|
| | | Loading Week- All 2 hour I.V. infusions | | | Once a Week Dosing- SQ Injections at 250 mg/mL | | |
| Dose | # Subjects | MD1 | MD3 | MD5 | MD8 | MD15 | MD22 |
| Placebo | 7 | N/A | N/A | N/A | According to cohort | | |
| 50 mg | 7 | 50 mg | 50 mg | 50 mg | 1 inj, 0.2 mL | 1 inj, 0.2 mL | 1 inj, 0.2 mL |
| 100 mg | 8 | 100 mg | 100 mg | 100 mg | 1 inj, 0.4 mL | 1 inj, 0.4 mL | 1 inj, 0.4 mL |
| 200 mg | 8 | 200 mg | 200 mg | 200 mg | 1 inj, 0.8 mL | 1 inj, 0.8 mL | 1 inj, 0.8 mL |
| 400 mg | 2 | 400 mg | 400 mg | 400 mg | 2 inj, 0.8 mL | 2 inj, 0.8 mL | 2 inj, 0.8 mL |

During the post-treatment evaluation period, on Days PD14 (or PD39, 39 days since MD1), and PD30 (or PD55, 55 days since MD1) blood samples were collected for lipid panel and PK analysis. All subjects who had fasting total serum cholesterol levels less than or equal to 90% of their baseline values on PD30 continued in an extended follow-up period. Fasting lipid panel measurements were made every 2 weeks until PD86 (or PD111, 111 days past MD1) or until total serum cholesterol levels returned to greater than 90% of baseline. On Days PD44, PD58, PD72, and PD 86 (or PD69, PD83, PD97 and PD111, respectively), blood samples were collected for lipid panel and PK analysis.

Pharmacodynamic Analysis

The pharmacodynamic effects of ISIS 301012 were assessed by comparing lipid parameter levels at the start of treatment to those following multiple doses of ISIS 301012; these data are shown in the following tables. Data are presented as mean percent change from baseline, where the baseline is either the respective lipid parameter measurement made on the first day of the first dose of study drug administered, which was either the first day of the single dose treatment period (SD1) or the first day of the multiple dose treatment period (MD1).

Total cholesterol, LDL-cholesterol, HDL-cholesterol and triglycerides were measured by routine clinical procedures at MDS Pharma Services (Belfast, Ireland). Apolipoprotein B and Lipoprotein(a) levels were measured by routine clinical laboratory procedures at MDS Pharma Services (Belfast, Ireland). The data are presented in the Tables below as the mean percent change relative to the baseline values of SD1 (Tables 6, 8, 10, 12, 14, 16, 18, and 19) or MD1 (Tables 7, 9, 11, 13, 15, 17, and 21). The mean represents the average of data from 5 subjects in the placebo cohort, from 3 subjects in the 50 mg cohort, from 3 subjects in the 100 mg cohort, from 6 to 7 subjects in the 200 mg cohort and from 2 subjects in the 400 mg cohort.

The baseline value to which the data were normalized is indicated for each Table. The baseline values were set at 100%, and values above or below 100% indicate an increase or decrease, respectively, in the lipid parameter measured. Data are presented for lipid parameter measurements made during the multiple dose periods, for example, MD8 indicates a measurement made 8 days following administration of the first dose during the multiple dose periods. Also shown are data from lipid parameter measurements made during the post-treatment evaluation period, for example, a measurement on day PD39 was made on the $14^{th}$ day of the post-treatment evaluation period, which is equivalent to 39 days following the first administration of the first dose during the multiple dose period. Where present, "ND" indicates that the particular measurement is "not determined". Analyses of other serum biomarkers revealed no clinical adverse event trends, including no changes in white blood cell count, platelet count or renal function. Furthermore, no toxicities were observed following administration of ISIS 301012.

**LDL-cholesterol.** Measurements of LDL-cholesterol, shown in Tables 6 and 7, revealed reductions in this lipoprotein in the 100 mg, 200 mg and 400 mg cohorts throughout the multiple dose period. For example, in Table 6, on MD25, mean LDL-cholesterol levels were 73%, 66% and 60% of baseline values in the 100 mg, 200 mg and 400 mg cohorts, respectively. Effects were seen in the 400 mg cohort, where LDL-cholesterol levels were reduced by as much as 39% (61% of MD1 baseline on day MD22) or 45% (55% of SD1 baseline value on day MD15). Furthermore, a reduction in LDL-cholesterol was observed out to day PD83 in the 100 mg cohort, approximately 2 months following administration of the final dose of ISIS 301012.

**Table 6**

| LDL-Cholesterol Mean Percent of Baseline (SD1) | | | | | |
|---|---|---|---|---|---|
| | **Placebo** | **50 mg** | **100 mg** | **200 mg** | **400 mg** |
| **MD1** | 99 | 120 | 89 | 83 | 91 |
| **MD8** | 99 | 99 | 75 | 83 | 79 |
| **MD15** | 100 | 106 | 63 | 68 | 61 |
| **MD22** | 94 | 109 | 69 | 65 | 55 |
| **MD25** | 92 | 108 | 73 | 66 | 60 |
| **PD39** | 87 | ND | 68 | 58 | ND |
| **PD55** | 96 | 95 | 69 | 58 | ND |
| **PD83** | ND | ND | 78 | ND | ND |

**Table 7**

| LDL-Cholesterol Mean Percent of Baseline (MD1) | | | | | |
|---|---|---|---|---|---|
| | **Placebo** | **50 mg** | **100 mg** | **200 mg** | **400 mg** |
| **MD8** | 100 | 83 | 84 | 92 | 87 |
| **MD15** | 102 | 89 | 70 | 75 | 67 |
| **MD22** | 96 | 92 | 78 | 72 | 61 |
| **MD25** | 93 | 90 | 82 | 73 | 66 |
| **PD39** | 88 | ND | 76 | 64 | ND |
| **PD55** | 97 | 79 | 78 | 64 | ND |
| **PD83** | ND | ND | 87 | ND | ND |

**Apolipoprotein B.** Measurements of serum apolipoprotein B, shown in Tables 8 and 9, revealed a dose-dependent reduction in this apolipoprotein, particularly in the 100 mg, 200 mg and 400 mg cohorts. Reductions in serum apolipoprotein B were maintained out to day PD111 in the 100 mg and 200 mg cohorts, approximately 3 months following administration of the final dose of ISIS 301012. Reductions were as great as 56% or 52% (or 44% of SD1 baseline value on day MD22 or 48% of MD1 baseline value on day MD22, respectively) in the 400 mg cohort; a similar reduction in the mean percent relative to SD1 baseline was observed out to day PD83. Additionally, prolonged effects were also observed in the 100 mg and 200 mg cohorts, where serum apolipoprotein B levels were 90% and 79%, respectively, of SD1 baseline values on day PD111, approximately 3 months following administration of the final dose of ISIS 301012.

**Table 8**

| Apolipoprotein B Mean Percent of Baseline (SD1) | | | | | |
|---|---|---|---|---|---|
| | **Placebo** | **50 mg** | **100 mg** | **200 mg** | **400 mg** |
| **MD1** | 111 | 111 | 90 | 99 | 92 |
| **MD8** | 113 | 86 | 80 | 94 | 76 |
| **MD15** | 109 | 100 | 67 | 66 | 56 |
| **MD22** | 102 | 89 | 80 | 63 | 44 |
| **MD25** | 98 | 89 | 90 | 44 | 52 |
| **PD39** | 115 | 89 | 80 | 58 | 48 |
| **PD55** | 104 | 86 | 80 | 53 | 52 |
| **PD69** | ND | ND | ND | 61 | 52 |

(continued)

| Apolipoprotein B Mean Percent of Baseline (SD1) | | | | | |
|---|---|---|---|---|---|
| | **Placebo** | **50 mg** | **100 mg** | **200 mg** | **400 mg** |
| **PD83** | ND | ND | 90 | 69 | 56 |
| **PD97** | ND | ND | 90 | 72 | ND |
| **PD111** | ND | ND | 90 | 79 | ND |

**Table 9**

| Apolipoprotein B Mean Percent of Baseline (MD1) | | | | | |
|---|---|---|---|---|---|
| | **Placebo** | **50 mg** | **100 mg** | **200 mg** | **400 mg** |
| **MD8** | 102 | 77 | 89 | 96 | 83 |
| **MD15** | 98 | 90 | 74 | 67 | 61 |
| **MD22** | 92 | 81 | 89 | 64 | 48 |
| **MD25** | 88 | 81 | 100 | 45 | 57 |
| **PD39** | 104 | 81 | 89 | 58 | 52 |
| **PD55** | 94 | 77 | 89 | 53 | 57 |
| **PD69** | ND | 62 | ND | 62 | 57 |
| **PD83** | ND | 70 | 100 | 70 | 61 |
| **PD97** | ND | 73 | 100 | 73 | ND |
| **PD111** | ND | 80 | 100 | 80 | ND |

**Total Cholesterol.** Analysis of total cholesterol revealed a reduction in the mean percent change from baseline, whether the data were normalized to SD1 (Table 10) or MD1 (Table 11). For example, on MD1, MD8, MD15, MD22 and MD25, mean total cholesterol in the 200 mg cohort was reduced to 97%, 89%, 74%, 71% and 76% of SD1 baseline values. Furthermore, these effects were sustained following cessation of dosing on MD 22. For example, in the 200 mg cohort, the mean total cholesterol was 88% of SD1 baseline values on PD111, approximately 3 months following administration of the final dose of ISIS 301012.

**Table 10**

| Total Cholesterol Mean Percent of Baseline (SD1) | | | | | |
|---|---|---|---|---|---|
| | **Placebo** | **50 mg** | **100 mg** | **200 mg** | **400 mg** |
| **MD1** | 103 | 113 | 87 | 97 | 88 |
| **MD8** | 103 | 93 | 88 | 89 | 79 |
| **MD15** | 104 | 96 | 75 | 74 | 63 |
| **MD22** | 99 | 103 | 76 | 71 | 60 |
| **MD25** | 98 | 97 | 89 | 76 | 63 |
| **PD39** | 109 | 100 | 85 | 70 | 61 |
| **PD55** | 114 | 105 | 90 | 74 | 68 |
| **PD69** | ND | ND | 84 | 69 | 62 |
| **PD83** | ND | ND | 95 | 78 | ND |
| **PD97** | ND | ND | 96 | 78 | ND |
| **PD111** | ND | ND | 105 | 88 | ND |

**Table 11**

| Total Cholesterol Mean Percent of Baseline (MD1) | | | | | |
|---|---|---|---|---|---|
| | **Placebo** | **50 mg** | **100 mg** | **200 mg** | **400 mg** |
| **MD8** | 101 | 83 | 101 | 91 | 90 |
| **MD15** | 102 | 85 | 86 | 76 | 72 |
| **MD22** | 97 | 91 | 88 | 73 | 68 |
| **MD25** | 96 | 86 | 102 | 78 | 72 |
| **PD39** | 106 | 89 | 98 | 72 | 69 |
| **PD55** | 111 | 93 | 103 | 75 | 78 |
| **PD69** | ND | ND | 97 | 71 | 70 |
| **PD83** | ND | ND | 109 | 80 | ND |
| **PD97** | ND | ND | 110 | 80 | ND |
| **PD111** | ND | ND | 120 | 91 | ND |

**HDL-Cholesterol.** As shown in Tables 12 and 13, these data reveal some changes in HDL-cholesterol in the 400 mg cohort, however, in the 50, 100 and 200 mg cohorts, HDL-cholesterol changes were not markedly changed. HDL-cholesterol levels were increased in the 100 mg cohort, relative to baseline day MD1.

**Table 12**

| HDL-Cholesterol Mean Percent of Baseline (SD1) | | | | | |
|---|---|---|---|---|---|
| | **Placebo** | **50 mg** | **100 mg** | **200 mg** | **400 mg** |
| **MD1** | 101 | 102 | 92 | 102 | 87 |
| **MD8** | 94 | 78 | 102 | 91 | 71 |
| **MD15** | 95 | 92 | 91 | 96 | 67 |
| **MD22** | 103 | 99 | 102 | 104 | 87 |
| **MD25** | 103 | 100 | 109 | 102 | 94 |
| **PD39** | 103 | ND | 99 | 102 | ND |
| **PD55** | 99 | 98 | 101 | 106 | ND |

**Table 13**

| HDL-Cholesterol Mean Percent of Baseline (MD1) | | | | | |
|---|---|---|---|---|---|
| | **Placebo** | **50 mg** | **100 mg** | **200 mg** | **400 mg** |
| **MD8** | 93 | 76 | 110 | 89 | 82 |
| **MD15** | 94 | 90 | 98 | 94 | 77 |
| **MD22** | 102 | 96 | 110 | 102 | 100 |
| **MD25** | 102 | 98 | 118 | 100 | 108 |
| **PD39** | 114 | ND | 108 | 99 | ND |
| **PD55** | 98 | 96 | 109 | 104 | ND |

**Triglyceride.** Triglyceride levels, shown in Tables 14 and 15, were reduced by administration of ISIS 301012 in the 100 mg, 200 mg and 400 mg cohorts. As shown in Table 14, in the 100 mg and 200 mg cohorts, triglyceride reduction was achieved by MD8 and MD15, respectively, and was maintained out to day PD55, approximately 1 month following administration of the final dose of ISIS 301012.

**Table 14**

| Triglyceride Mean Percent of Baseline (SD1) | | | | | |
|---|---|---|---|---|---|
| | **Placebo** | **50 mg** | **100 mg** | **200 mg** | **400 mg** |
| **MD1** | 148 | 100 | 110 | 121 | 103 |
| **MD8** | 153 | 180 | 81 | 107 | 105 |
| **MD15** | 157 | 103 | 91 | 94 | 87 |
| **MD22** | 122 | 89 | 87 | 90 | 91 |
| **MD25** | 109 | 100 | 83 | 91 | 64 |
| **PD39** | 129 | ND | 70 | 83 | ND |
| **PD55** | 96 | 115 | 92 | 66 | ND |

**Table 15**

| Triglyceride Mean Percent of Baseline (MD1) | | | | | |
|---|---|---|---|---|---|
| | **Placebo** | **50 mg** | **100 mg** | **200 mg** | **400 mg** |
| **MD8** | 103 | 180 | 73 | 89 | 102 |
| **MD15** | 106 | 103 | 82 | 78 | 85 |
| **MD22** | 82 | 89 | 79 | 75 | 89 |
| **MD25** | 74 | 100 | 75 | 75 | 62 |
| **PD39** | 87 | ND | 64 | 69 | ND |
| **PD55** | 65 | 115 | 83 | 55 | ND |

**Lipoprotein(a).** Lipoprotein(a) levels in serum, shown in Tables 16 and 17, were reduced following treatment with ISIS 301012. For example, in the 400 mg cohort, lipoprotein(a) was reduced during the multiple dose period, to 71%, 83%, 76% and 84% of SD1 baseline or 66%, 85%, 70% and 72% of MD1 baseline on days MD8, MD15, MD22 and MD25, respectively. Reductions of approximately 12% to 15% (normalized to baseline SD1 or MD1) were sustained out to day PD55 in the 100 mg and 200 mg cohorts, approximately one month following administration of the final dose of ISIS 301012.

**Table 16**

| Lipoprotein(a) Mean Percent of Baseline (SD1) | | | | | |
|---|---|---|---|---|---|
| | **Placebo** | **50 mg** | **100 mg** | **200 mg** | **400 mg** |
| **MD1** | 104 | 130 | 99 | 97 | 108 |
| **MD8** | 93 | 99 | 107 | 109 | 71 |
| **MD15** | 100 | 103 | 84 | 89 | 93 |
| **MD22** | 104 | 95 | 75 | 82 | 76 |
| **MD25** | 111 | 109 | 90 | 84 | 78 |
| **PD39** | 140 | ND | 93 | 88 | ND |
| **PD55** | 122 | 97 | 88 | 85 | ND |

**Table 17**

| Lipoprotein(a) Mean Percent of Baseline (MD1) | | | | | |
|---|---|---|---|---|---|
| | **Placebo** | **50 mg** | **100 mg** | **200 mg** | **400 mg** |
| **MD8** | 89 | 76 | 108 | 112 | 66 |
| **MD15** | 96 | 79 | 84 | 91 | 85 |
| **MD22** | 100 | 74 | 75 | 84 | 70 |
| **MD25** | 106 | 84 | 91 | 86 | 72 |
| **PD39** | 134 | ND | 94 | 91 | ND |
| **PD55** | 118 | 75 | 88 | 87 | ND |

**Total Cholesterol:HDL Ratio.** As shown in Table 18, the ratios of total cholesterol to HDL-cholesterol were calculated, revealing an improvement in these ratios, which is a clinically-desirable effect. A reduction in the ratios relative to baseline values was observed, for example, in the 200 mg cohort, beginning on MD8 and persisting out to PD55. Improvements in the ratio of total cholesterol to HDL-cholesterol were also seen in the 100 mg and 400 mg cohorts.

**Table 18**

| Total Cholesterol:HDL Ratio, Mean Percent of Baseline (SD1) | | | | | |
|---|---|---|---|---|---|
| | **Placebo** | **50 mg** | **100 mg** | **200 mg** | **400 mg** |
| **MD8** | 109 | 123 | 91 | 97 | 106 |
| **MD15** | 112 | 101 | 86 | 80 | 91 |
| **MD22** | 100 | 93 | 87 | 74 | 63 |
| **MD25** | 95 | 91 | 85 | 75 | 64 |
| **PD39** | 95 | ND | 91 | 69 | ND |
| **PD55** | 108 | 97 | 90 | 68 | ND |

**LDL-cholesterol versus HDL-cholesterol.** As shown in Table 19, the clinically-desirable reduction in LDL:HDL ratio was observed in the 100 mg, 200 mg and 400 mg cohorts during the multiple dose treatment period. These reductions persisted out to day PD55 in the 100 mg and 200 mg cohorts, on which day the ratios were 68% and 55%, respectively, of SD1 baseline values.

**Table 19**

| LDL: HDL Ratio, Mean Percent of Baseline (SD1) | | | | | |
|---|---|---|---|---|---|
| | **Placebo** | **50 mg** | **100 mg** | **200 mg** | **400 mg** |
| **MD8** | 102 | 130 | 74 | 88 | 100 |
| **MD15** | 109 | 116 | 68 | 68 | 82 |
| **MD22** | 95 | 103 | 70 | 62 | 54 |
| **MD25** | 88 | 101 | 67 | 63 | 55 |
| **PD39** | 81 | ND | 69 | 55 | ND |
| **PD55** | 93 | 97 | 68 | 55 | ND |

**VLDL-Cholesterol.** As shown in Tables 20 and 21, VLDL-cholesterol levels were shown to be reduced, in all cohorts relative to baseline day MD1 and in the 100 mg, 200 mg and 400 mg cohorts relative to baseline day SD1. For example, by day MD25, VLDL-cholesterol levels in the 200 mg cohort were reduced to 63% of baseline MD1, and these effects persisted out to study day PD55, at which time VLDL-cholesterol levels were 49% of baseline day MD1. Sustained reductions in VLDL-cholesterol were observed out to PD55 in the 100 and 200 mg cohorts, normalized to baseline days SD1 or MD1.

**Table 20**

| VLDL-Cholesterol Mean Percent of Baseline (SD1) | | | | | |
|---|---|---|---|---|---|
| | **Placebo** | **50 mg** | **100 mg** | **200 mg** | **400 mg** |
| **MD1** | 144 | 125 | 106 | 122 | 84 |
| **MD8** | 149 | 222 | 79 | 101 | 88 |
| **MD15** | 153 | 118 | 90 | 87 | 62 |
| **MD22** | 122 | 103 | 91 | 83 | 70 |
| **MD25** | 106 | 121 | 87 | 77 | 44 |
| **PD39** | 126 | ND | 83 | 77 | ND |
| **PD55** | 87 | 127 | 86 | 60 | ND |

**Table 21**

| VLDL-Cholesterol Mean Percent of Baseline (MD1) | | | | | |
|---|---|---|---|---|---|
| | **Placebo** | **50 mg** | **100 mg** | **200 mg** | **400 mg** |
| **MD8** | 104 | 178 | 74 | 83 | 106 |
| **MD15** | 107 | 95 | 85 | 71 | 74 |
| **MD22** | 85 | 82 | 86 | 68 | 83 |
| **MD25** | 74 | 97 | 82 | 63 | 52 |
| **PD39** | 88 | ND | 78 | 63 | ND |
| **PD55** | 60 | 102 | 81 | 49 | ND |

Pharmacodynamic Effects with Statistical Analysis

Shown in Tables 22 through 43 are summaries of the lipid parameters evaluated in 7 subjects who received the placebo, and 8, 8, 9, and 4 subjects who received 50 mg, 100 mg, 200 mg and 400 mg ISIS 301012, respectively. Presented in the Summary tables are the mean levels of each lipid parameter, e.g. mg/dL LDL-cholesterol, per dose cohort. The second table shown for each lipid parameter is the Percent Change table, and represents the mean percent change from baseline for each cohort, e.g. mean percent change at MD25 relative to baseline. "Study day" indicates the day of the study when the lipid parameter, e.g. LDL-cholesterol, was measured. The "baseline" measurement is the level of the lipid parameter, e.g. LDL-cholesterol, prior to the initial dose of ISIS 301012 on study day SD1. If a measurement from SD1 was not available, a MD 1 measurement was used in its place. "MD" indicates a measurement taken during the multiple dose period, for example, MD15 is day 15 of the multiple dose period. The multiple dose period was 25 days in length, thus the MD25 measurement is from a sample taken on the day of the final dose of study drug. "PD" is a measurement taken during the post-treatment evaluation period. For example, PD39 is a measurement taken 14 days following the final dose, which is equivalent to 39 days following the first dose of the multiple dose period. "N" indicates the number of samples used in calculating the mean and median levels; N for measurements made during the MD or PD periods may be less than N at baseline. "Std" is the standard deviation. P-values apply to the difference between means in dosing groups and means in the placebo group, and were determined using the Wilcoxon Rank-sum test. "NC" indicates a lipid parameter level or P-value that was not calculated.

**LDL-Cholesterol.** Table 22 contains the LDL-cholesterol level summary, where levels are shown in mg/dL. The percent mean and median changes in LDL-cholesterol, relative to baseline values, are presented in Table 23. Prolonged and dose-dependent reductions in serum LDL-cholesterol levels were observed. The maximum percent reduction of LDL-cholesterol was approximately 35% for the 200 mg group and approximately 48% for the 400 mg group (Table 23). LDL-cholesterol reduction persisted, remaining below baseline for 90 days in 50% of the subjects in the 200 mg group.

**Table 22**

| LDL-Cholesterol Level Summary, mg/dL | | | | | | |
|---|---|---|---|---|---|---|
| **Study Day** | | **Placebo** | **50 mg** | **100 mg** | **200 mg** | **400 mg** |
| **Baseline** | N | 7 | 8 | 8 | 9 | 4 |
| | Mean | 131 | 126 | 131 | 123 | 133 |
| | Median | 123 | 118 | 136 | 121 | 130 |
| | Std | 27 | 24 | 24 | 16 | 23 |
| | Min - Max | 95 - 171 | 100 - 173 | 91 - 160 | 103 - 153 | 109 - 164 |
| | P-value | | 0.7789 | 1 | 0.6597 | 0.7879 |
| **MD8** | N | 7 | 7 | 8 | 8 | 2 |
| | Mean | 126 | 118 | 119 | 105 | 105 |
| | Median | 121 | 116 | 111 | 102 | 105 |
| | Std | 19 | 17 | 25 | 14 | 14 |
| | Min - Max | 107 - 160 | 104 - 155 | 94 - 164 | 88 - 126 | 95 - 115 |
| | P-value | | 0.197 | 0.414 | 0.0401 | 0.2222 |
| **MD15** | N | 7 | 8 | 8 | 8 | 2 |
| | Mean | 132 | 124 | 101 | 85 | 82 |
| | Median | 116 | 121 | 99 | 86 | 82 |
| | Std | 29 | 20 | 29 | 14 | 21 |
| | Min - Max | 109 - 175 | 99 - 166 | 59 - 151 | 67 - 107 | 67 - 96 |
| | P-value | | 0.9302 | 0.0774 | 0.0003 | 0.0556 |
| **MD22** | N | 7 | 8 | 8 | 8 | 2 |
| | Mean | 124 | 125 | 102 | 82 | 74 |
| | Median | 112 | 125 | 98 | 85 | 74 |
| | Std | 29 | 15 | 20 | 11 | 5 |
| | Min - Max | 93 - 167 | 96 - 149 | 79 - 144 | 58 - 92 | 70 - 77 |
| | P-value | | 0.4443 | 0.0881 | 0.0003 | 0.0556 |
| **MD25** | N | 7 | 8 | 8 | 8 | 2 |
| | Mean | 122 | 128 | 105 | 83 | 80 |
| | Median | 115 | 121 | 102 | 86 | 80 |
| | Std | 32 | 16 | 16 | 13 | 6 |
| | Min - Max | 76 - 170 | 117 - 163 | 84 - 139 | 54 - 95 | 76 - 84 |
| | P-value | | 0.143 | 0.128 | 0.0126 | 0.1389 |
| **PD39** | N | 7 | 8 | 8 | 8 | 2 |
| | Mean | 128 | 119 | 106 | 77 | 79 |
| | Median | 118 | 115 | 98 | 81 | 79 |
| | Std | 22 | 17 | 24 | 18 | 1 |
| | Min - Max | 107 - 160 | 103 - 155 | 90-158 | 51 - 107 | 78 - 80 |
| | P-value | | 0.4797 | 0.0362 | 0.0005 | 0.0556 |

(continued)

| LDL-Cholesterol Level Summary, mg/dL | | | | | | |
|---|---|---|---|---|---|---|
| **Study Day** | | **Placebo** | **50 mg** | **100 mg** | **200 mg** | **400 mg** |
| **PD55** | N | 7 | 8 | 8 | 8 | 2 |
| | Mean | 127 | 117 | 103 | 79 | 85 |
| | Median | 120 | 114 | 96 | 82 | 85 |
| | Std | 25 | 16 | 25 | 17 | 13 |
| | Min - Max | 103 - 179 | 100 - 151 | 72 - 154 | 59 - 114 | 76 - 94 |
| | P-value | | 0.3357 | 0.0376 | 0.0009 | 0.0556 |
| **PD69** | N | 2 | 2 | 5 | 7 | 2 |
| | Mean | 127 | 111 | 106 | 85 | 84 |
| | Median | 127 | 111 | 105 | 87 | 84 |
| | Std | 24 | 25 | 23 | 7 | 7 |
| | Min-Max | 110 - 144 | 93 - 128 | 81 - 141 | 73 - 93 | 79 - 89 |
| | P-value | | 0.6667 | 0.381 | 0.0278 | 0.3333 |
| **PD83** | N | 2 | 3 | 7 | 8 | 2 |
| | Mean | 144 | 115 | 108 | 92 | 99 |
| | Median | 144 | 111 | 106 | 92 | 99 |
| | Std | 40 | 25 | 23 | 12 | 2 |
| | Min - Max | 116 - 172 | 92 - 141 | 73 - 150 | 72 - 115 | 97 - 100 |
| | P-value | | 0.4 | 0.2222 | 0.0444 | 0.3333 |
| **PD97** | N | 0 | 2 | 5 | 8 | 0 |
| | Mean | NC | 114 | 111 | 95 | NC |
| | Median | NC | 114 | 116 | 95 | NC |
| | Std | NC | 21 | 36 | 10 | NC |
| | Min - Max | NC | 99 - 128 | 65 - 160 | 75 - 109 | NC |
| **PD111** | N | 0 | 1 | 2 | 6 | 0 |
| | Mean | NC | 90 | 121 | 101 | NC |
| | Median | NC | 90 | 121 | 100 | NC |
| | Std | NC | NC | 13 | 11 | NC |
| | Min - Max | NC | 90 - 90 | 112 - 130 | 85 - 119 | NC |

**Table 23**

| LDL-Cholesterol Level Summary, % Change from Baseline | | | | | | |
|---|---|---|---|---|---|---|
| **Study Day** | | **Placebo** | **50 mg** | **100 mg** | **200 mg** | **400 mg** |
| **MD8** | N | 7 | 7 | 8 | 8 | 2 |
| | Mean | -2 | -8 | -8 | -14 | -27 |
| | Median | 1 | -8 | -14 | -14 | -27 |
| | Std | 16 | 7 | 15 | 10 | 5 |
| | Min - Max | -20 - 27 | -19 - 2 | -23 - 20 | -27 - 3 | -30 - -23 |
| | P-value | | 0.6200 | 0.3357 | 0.1893 | 0.0556 |
| **MD15** | N | 7 | 8 | 8 | 8 | 2 |
| | Mean | 1 | -0.2 | -23 | -29 | -44 |
| | Median | 4 | -3 | -24 | -26 | -44 |
| | Std | 11 | 13 | 19 | 17 | 3 |
| | Min - Max | -19 - 16 | -14 - 29 | -46 - 9 | -50 - -7 | -46 - -43 |
| | P-value | | 0.5358 | 0.0205 | 0.0022 | 0.0556 |
| **MD22** | N | 7 | 8 | 8 | 8 | 2 |
| | Mean | -5 | 1 | -21 | -32 | -48 |
| | Median | -2 | -2 | -23 | -28 | -48 |
| | Std | 12 | 15 | 12 | 15 | 7 |
| | Min - Max | -31 - 7 | -14 - 25 | -33 - 0.0 | -52 - -11 | -53 - -44 |
| | P-value | | 0.8665 | 0.0289 | 0.0037 | 0.0556 |
| **MD25** | N | 7 | 8 | 8 | 8 | 2 |
| | Mean | -6 | 3 | -19 | -31 | -44 |
| | Median | -4 | 1 | -20 | -26 | -44 |
| | Std | 19 | 16 | 11 | 16 | 7 |
| | Min - Max | -43 - 21 | -15 - 37 | -32 - 2 | -55 - -12 | -49 - -39 |
| | P-value | | 0.6126 | 0.0939 | 0.0093 | 0.1111 |
| **PD39** | N | 7 | 8 | 8 | 8 | 2 |
| | Mean | -1 | -4 | -19 | -35 | -44 |
| | Median | -3 | -8 | -18 | -35 | -44 |
| | Std | 13 | 16 | 14 | 19 | 10 |
| | Min - Max | -19 - 20 | -21 - 30 | -36 - -1 | -67 - -7 | -51 - -37 |
| | P-value | | 0.6126 | 0.0721 | 0.0022 | 0.0556 |
| **PD55** | N | 7 | 8 | 8 | 8 | 2 |
| | Mean | -1 | -6 | -20 | -34 | -41 |
| | Median | 5 | -7 | -23 | -37 | -41 |
| | Std | 15 | 8 | 17 | 19 | 3 |
| | Min - Max | -18 - 21 | -15 - 9 | -40 - 10 | -58 - -1 | -43 - -39 |
| | P-value | | 0.8665 | 0.0939 | 0.0022 | 0.0556 |

(continued)

| LDL-Cholesterol Level Summary, % Change from Baseline | | | | | | |
|---|---|---|---|---|---|---|
| Study Day | | Placebo | 50 mg | 100 mg | 200 mg | 400 mg |
| PD69 | N | 2 | 2 | 5 | 7 | 2 |
| | Mean | 4 | -13 | -18 | -29 | -41 |
| | Median | 4 | -13 | -17 | -24 | -41 |
| | Std | 18 | 14 | 9 | 12 | 7 |
| | Min - Max | -8 - 17 | -23 - -3 | -33 - -11 | -48 - -16 | -46 - -36 |
| | P-value | | 0.6667 | 0.0952 | 0.0556 | 0.3333 |
| PD83 | N | 2 | 3 | 7 | 8 | 2 |
| | Mean | -1 | -1 | -19 | -24 | -30 |
| | Median | -1 | -4 | -20 | -18 | -30 |
| | Std | 3 | 16 | 8 | 15 | 12 |
| | Min - Max | -3 - 0.6 | -15 - 17 | -26 - -6 | -45 - -6 | -39 - -22 |
| | P-value | | 0.8000 | 0.0556 | 0.0444 | 0.3333 |
| PD97 | N | 0 | 2 | 5 | 8 | 0 |
| | Mean | NC | 2 | -15 | -21 | NC |
| | Median | NC | 2 | -18 | -16 | NC |
| | Std | NC | 14 | 11 | 13 | NC |
| | Min - Max | NC | -8 - 11 | -29 - 0 | -46 - -11 | NC |
| | P-value | 0 | 1 | 2 | 6 | 0 |
| PD111 | N | NC | -17 | 7 | -19 | NC |
| | Mean | NC | -17 | 7 | -19 | NC |
| | Median | NC | 0 | 23 | 8 | NC |
| | Std | NC | -17 - -17 | -9 - 23 | -30 - -6 | NC |
| | Min - Max | 7 | 7 | 8 | 8 | 2 |
| | P-value | -2 | -8 | -8 | -14 | -27 |

**Apolipoprotein B.** Shown in Tables 24 and 25 are the mean serum apolipoprotein B levels per cohort and mean percent of baseline per cohort, respectively. Administration of ISIS 301012 resulted in a dose-dependent, prolonged reduction of serum apolipoprotein B levels. The 200 mg dose group showed a maximum reduction of 50%, relative to baseline (Table 25), on MD25, 72 hours following the final dose. The reduced apolipoprotein B levels remained below baseline for 90 days following treatment in 75% of the 200 mg subjects. Due to the prolonged effect of ISIS 301012, the levels of serum apolipoprotein B were measured at PD125 and PD139 for 2 subjects and 1 subject, respectively. At Day PD125, the mean and median serum apolipoprotein B levels for 2 subjects from the 200 mg cohort was 90 mg/dL (std = 28), representing a -17% change relative to baseline (std = 12). At Day PD139, total serum apolipoprotein B in 1 subject was 86 mg/dL, representing a -28% change relative to baseline.

**Table 24**

| Apolipoprotein B Level Summary, mg/dL | | | | | | |
|---|---|---|---|---|---|---|
| Study Day | | Placebo | 50 mg | 100 mg | 200 mg | 400 mg |
| Baseline | N | 7 | 8 | 8 | 9 | 4 |
| | Mean | 102 | 101 | 105 | 101 | 113 |

(continued)

| Apolipoprotein B Level Summary, mg/dL | | | | | | |
|---|---|---|---|---|---|---|
| Study Day | | Placebo | 50 mg | 100 mg | 200 mg | 400 mg |
| | Median | 93 | 101 | 99 | 98 | 110 |
| | Std | 22 | 20 | 22 | 17 | 27 |
| | Min - Max | 84 - 147 | 77 - 142 | 68 - 141 | 76 - 133 | 86 - 145 |
| | P-value | | 0.9312 | 0.2671 | 0.4684 | 0.4939 |
| **MD8** | N | 7 | 8 | 8 | 8 | 2 |
| | Mean | 107 | 86 | 95 | 93 | 90 |
| | Median | 105 | 83 | 83 | 89 | 90 |
| | Std | 18 | 24 | 24 | 15 | 21 |
| | Min - Max | 86 - 132 | 56 - 136 | 75 - 142 | 76 - 117 | 75 - 105 |
| | P-value | | 0.058 | 0.1807 | 0.152 | 0.3889 |
| **MD15** | N | 7 | 8 | 8 | 8 | 3 |
| | Mean | 103 | 94 | 78 | 67 | 70 |
| | Median | 94 | 89 | 71 | 69 | 70 |
| | Std | 28 | 20 | 21 | 13 | 16 |
| | Min - Max | 68 - 143 | 72-138 | 57 - 122 | 47 - 84 | 54 - 86 |
| | P-value | | 0.2939 | 0.0323 | 0.0034 | 0.0583 |
| **MD22** | N | 7 | 8 | 8 | 8 | 2 |
| | Mean | 99 | 91 | 82 | 66 | 56 |
| | Median | 93 | 89 | 77 | 70 | 56 |
| | Std | 23 | 16 | 20 | 11 | 6 |
| | Min - Max | 76 - 143 | 65 - 118 | 63 - 124 | 41 - 76 | 52 - 60 |
| | P-value | | 0.6943 | 0.113 | 0.0005 | 0.0556 |
| **MD25** | N | 7 | 8 | 8 | 8 | 2 |
| | Mean | 94 | 87 | 87 | 50 | 66 |
| | Median | 95 | 84 | 87 | 51 | 66 |
| | Std | 22 | 10 | 18 | 16 | 6 |
| | Min - Max | 67 - 123 | 74 - 106 | 61 - 122 | 21 - 73 | 61 - 70 |
| | P-value | | 0.4634 | 0.5358 | 0.0012 | 0.1111 |
| **PD39** | N | 7 | 8 | 8 | 8 | 2 |
| | Mean | 102 | 86 | 81 | 61 | 59 |
| | Median | 101 | 82 | 79 | 62 | 59 |
| | Std | 19 | 12 | 23 | 13 | 4 |
| | Min - Max | 66 - 120 | 78 - 113 | 51 - 126 | 43 - 81 | 56 - 61 |
| | P-value | | 0.0503 | 0.0774 | 0.002 | 0.0556 |
| **PD55** | N | 7 | 8 | 8 | 8 | 2 |
| | Mean | 86 | 86 | 76 | 57 | 62 |
| | Median | 95 | 81 | 72 | 58 | 62 |

(continued)

| Apolipoprotein B Level Summary, mg/dL | | | | | | |
|---|---|---|---|---|---|---|
| Study Day | | Placebo | 50 mg | 100 mg | 200 mg | 400 mg |
| | Std | 21 | 19 | 25 | 9 | 8 |
| | Min - Max | 44 - 100 | 59 - 113 | 53 - 131 | 41 - 72 | 56 - 67 |
| | P-value | | 0.7573 | 0.2319 | 0.0166 | 0.2222 |
| PD69 | N | 3 | 5 | 5 | 7 | 2 |
| | Mean | 109 | 90 | 74 | 62 | 64 |
| | Median | 112 | 86 | 75 | 61 | 64 |
| | Std | 20 | 26 | 24 | 7 | 10 |
| | Min - Max | 88 -127 | 70 - 133 | 39 - 106 | 50 - 70 | 57 - 71 |
| | P-value | | 0.2857 | 0.0714 | 0.0083 | 0.2 |
| PD83 | N | 2 | 4 | 7 | 8 | 2 |
| | Mean | 120 | 96 | 72 | 67 | 70 |
| | Median | 120 | 88 | 76 | 68 | 70 |
| | Std | 28 | 25 | 28 | 11 | 10 |
| | Min - Max | 100 - 139 | 76 - 131 | 30 - 104 | 47 - 85 | 63 - 77 |
| | P-value | | 0.2667 | 0.1111 | 0.0444 | 0.3333 |
| PD97 | N | 0 | 2 | 4 | 8 | 1 |
| | Mean | NC | 96 | 90 | 72 | 81 |
| | Median | NC | 96 | 88 | 74 | 81 |
| | Std | NC | 8 | 42 | 12 | NC |
| | Min - Max | NC | 90 - 101 | 45 - 139 | 56 - 85 | 81 - 81 |
| PD111 | N | 0 | 1 | 3 | 6 | 2 |
| | Mean | NC | 76 | 81 | 77 | 95 |
| | Median | NC | 76 | 87 | 74 | 95 |
| | Std | NC | NC | 14 | 14 | 21 |
| | Min - Max | NC | 76 - 76 | 65 - 90 | 66 - 105 | 80 - 110 |

**Table 25**

| Apolipoprotein B, % Change from Baseline | | | | | | |
|---|---|---|---|---|---|---|
| Study Day | | Placebo | 50 mg | 100 mg | 200 mg | 400 mg |
| MD8 | N | 7 | 8 | 8 | 8 | 2 |
| | Mean | 7 | -14 | -9 | -8 | -24 |
| | Median | 11 | -13 | -11 | -7 | -24 |
| | Std | 12 | 22 | 14 | 12 | 5 |
| | Min - Max | -10 - 24 | -46 - 17 | -24 - 19 | -27 - 10 | -28 - -21 |
| | P-value | | 0.0721 | 0.0401 | 0.0401 | 0.0556 |
| MD15 | N | 7 | 8 | 8 | 8 | 3 |

(continued)

| Apolipoprotein B, % Change from Baseline | | | | | | |
|---|---|---|---|---|---|---|
| **Study Day** | | **Placebo** | **50 mg** | **100 mg** | **200 mg** | **400 mg** |
| | Mean | 1 | -6 | -25 | -32 | -43 |
| | Median | 1 | -4 | -28 | -32 | -43 |
| | Std | 14 | 14 | 13 | 18 | 2 |
| | Min - Max | -24 - 21 | -27 - 14 | -41 - -2 | -61 - -14 | -44 - -41 |
| | P-value | | 0.3969 | 0.0059 | 0.0022 | 0.0167 |
| **MD22** | N | 7 | 8 | 8 | 8 | 2 |
| | Mean | -3 | -9 | -22 | -34 | -52 |
| | Median | 0 | -10 | -20 | -33 | -52 |
| | Std | 10 | 11 | 11 | 16 | 9 |
| | Min - Max | -16 - 10 | -23 - 7 | -43 - -7 | -56 - -11 | -59 - -45 |
| | P-value | | 0.1893 | 0.0037 | 0.0012 | 0.0556 |
| **MD25** | N | 7 | 8 | 8 | 8 | 2 |
| | Mean | -7 | -13 | -16 | -50 | -44 |
| | Median | -3 | -11 | -12 | -50 | -44 |
| | Std | 16 | 12 | 13 | 17 | 11 |
| | Min - Max | -28 - 14 | -32 - 8 | -37 - 3 | -78 - -26 | -52 - -36 |
| | P-value | | 0.4634 | 0.281 | 0.0006 | 0.0556 |
| **PD39** | N | 7 | 8 | 8 | 8 | 2 |
| | Mean | 2 | -14 | -22 | -39 | -50 |
| | Median | 4 | -16 | -19 | -37 | -50 |
| | Std | 21 | 9 | 15 | 18 | 12 |
| | Min - Max | -26 - 37 | -26 - 3 | -57 - -10 | -61 - -17 | -58 - -41 |
| | P-value | | 0.0939 | 0.0541 | 0.0059 | 0.0556 |
| **PD55** | N | 7 | 8 | 8 | 8 | 2 |
| | Mean | -11 | -14 | -28 | -42 | -47 |
| | Median | 4 | -16 | -22 | -45 | -47 |
| | Std | 29 | 13 | 15 | 14 | 9 |
| | Min - Max | -70 - 12 | -33 - 4 | -49 - -7 | -59 - -21 | -54 - -41 |
| | P-value | | 0.1893 | 0.0541 | 0.0205 | 0.2222 |
| **PD69** | N | 3 | 5 | 5 | 7 | 2 |
| | Mean | 6 | -16 | -34 | -38 | -46 |
| | Median | -1 | -11 | -34 | -40 | -46 |
| | Std | 24 | 16 | 7 | 12 | 8 |
| | Min - Max | -14 - 33 | -33 - 4 | -43 - -25 | -55 - -20 | -51 - -40 |
| | P-value | | 0.3929 | 0.0357 | 0.0167 | 0.2 |
| **PD83** | N | 2 | 4 | 7 | 8 | 2 |
| | Mean | 4 | -12 | -31 | -33 | -40 |

(continued)

| Apolipoprotein B, % Change from Baseline | | | | | | |
|---|---|---|---|---|---|---|
| Study Day | | Placebo | 50 mg | 100 mg | 200 mg | 400 mg |
| | Median | 4 | -9 | -21 | -34 | -40 |
| | Std | 13 | 13 | 26 | 16 | 9 |
| | Min - Max | -5 - 12 | -30 - 0 | -67 - -5 | -52 - -8 | -47 - -34 |
| | P-value | | 0.2667 | 0.1111 | 0.0444 | 0.3333 |
| **PD97** | N | 0 | 2 | 4 | 8 | 1 |
| | Mean | NC | 3 | -19 | -28 | -15 |
| | Median | NC | 3 | -20 | -25 | -15 |
| | Std | NC | 27 | 15 | 14 | |
| | Min - Max | NC | -17 - 22 | -34 - -1 | -58 - -13 | -15 - -15 |
| **PD111** | N | 0 | 1 | 3 | 6 | 2 |
| | Mean | NC | -30 | -3 | -23 | -20 |
| | Median | NC | -30 | -8 | -25 | -20 |
| | Std | NC | | 33 | 16 | 6 |
| | Min - Max | NC | -30 - -30 | -32 - 32 | -48 - -3 | -24 - -16 |

**Total Cholesterol.** Total cholesterol mean levels and mean percent of baseline are shown in Tables 26 and 27, respectively. The maximum mean reductions observed were 27% in the 200 mg group and 40% in the 400 mg group (Table 27). Due to the prolonged effects of ISIS 301012, the levels of serum total cholesterol were measured at Day PD125 for 2 subjects and Day PD139 for 1 subject. At Day PD125, the mean and median serum total cholesterol level for 2 subjects from the 200 mg cohort was 170 mg/dL (std = 16), representing a -17% change relative to baseline (std = 6). At Day PD139, total serum cholesterol in 1 subject was 193 mg/dL, representing a -7% change relative to baseline.

**Table 26**

| Total Cholesterol Level Summary, mg/dL | | | | | | |
|---|---|---|---|---|---|---|
| Study Day | | Placebo | 50 mg | 100 mg | 200 mg | 400 mg |
| **Baseline** | N | 7 | 8 | 8 | 9 | 4 |
| | Mean | 216 | 221 | 218 | 211 | 239 |
| | Median | 213 | 217 | 220 | 201 | 240 |
| | Std | 33 | 33 | 24 | 18 | 30 |
| | Min - Max | 174 - 271 | 186 - 290 | 182 - 251 | 193 - 244 | 201 - 275 |
| | P-value | | 0.9324 | 0.8056 | 0.7378 | 0.2182 |
| **MD8** | N | 7 | 8 | 8 | 8 | 2 |
| | Mean | 214 | 205 | 201 | 187 | 203 |
| | Median | 209 | 195 | 199 | 182 | 203 |
| | Std | 18 | 29 | 29 | 22 | 14 |
| | Min - Max | 190 - 240 | 166 - 255 | 155 - 251 | 166 - 232 | 193 - 213 |
| | P-value | | 0.285 | 0.1961 | 0.0126 | 0.6389 |
| **MD15** | N | 7 | 8 | 8 | 8 | 2 |
| | Mean | 220 | 212 | 177 | 160 | 174 |

(continued)

| Total Cholesterol Level Summary, mg/dL | | | | | | |
|---|---|---|---|---|---|---|
| Study Day | | Placebo | 50 mg | 100 mg | 200 mg | 400 mg |
| | Median | 217 | 211 | 182 | 159 | 174 |
| | Std | 24 | 35 | 33 | 27 | 16 |
| | Min - Max | 193 - 259 | 170 - 282 | 135 - 240 | 120 - 197 | 162 - 186 |
| | P-value | | 0.4848 | 0.0071 | 0.0006 | 0.0556 |
| **MD22** | N | 7 | 8 | 8 | 8 | 2 |
| | Mean | 211 | 214 | 173 | 151 | 155 |
| | Median | 201 | 219 | 172 | 153 | 155 |
| | Std | 37 | 23 | 25 | 20 | 6 |
| | Min - Max | 159 - 263 | 166 - 240 | 139 - 220 | 124 -178 | 151 - 159 |
| | P-value | | 0.8005 | 0.0266 | 0.0022 | 0.0833 |
| **MD25** | N | 7 | 8 | 8 | 8 | 2 |
| | Mean | 211 | 217 | 188 | 160 | 162 |
| | Median | 209 | 209 | 191 | 157 | 162 |
| | Std | 39 | 26 | 24 | 22 | 6 |
| | Min - Max | 143 - 271 | 186 - 267 | 143 - 224 | 120 - 190 | 159 - 166 |
| | P-value | | 0.8838 | 0.1125 | 0.0099 | 0.2222 |
| **PD39** | N | 7 | 8 | 8 | 8 | 2 |
| | Mean | 230 | 218 | 190 | 153 | 157 |
| | Median | 224 | 213 | 190 | 164 | 157 |
| | Std | 27 | 28 | 25 | 27 | 14 |
| | Min - Max | 197 - 263 | 186 - 275 | 162 - 244 | 104 - 178 | 147 - 166 |
| | P-value | | 0.4127 | 0.0047 | 0.0002 | 0.0556 |
| **PD55** | N | 7 | 8 | 8 | 8 | 2 |
| | Mean | 235 | 224 | 185 | 161 | 176 |
| | Median | 228 | 220 | 186 | 168 | 176 |
| | Std | 31 | 26 | 28 | 26 | 3 |
| | Min - Max | 201 - 298 | 193 - 275 | 132 - 232 | 128 - 190 | 174 - 178 |
| | P-value | | 0.3804 | 0.0044 | 0.0003 | 0.0556 |
| **PD69** | N | 3 | 5 | 7 | 7 | 2 |
| | Mean | 241 | 222 | 193 | 154 | 159 |
| | Median | 248 | 209 | 186 | 155 | 159 |
| | Std | 26 | 58 | 24 | 21 | 11 |
| | Min - Max | 213 - 263 | 166 - 313 | 162 - 240 | 128 - 178 | 151 - 166 |
| | P-value | | 0.3929 | 0.025 | 0.0167 | 0.2 |
| **PD83** | N | 2 | 4 | 7 | 8 | 2 |
| | Mean | 259 | 226 | 201 | 168 | 182 |
| | Median | 259 | 219 | 201 | 172 | 182 |

(continued)

| Total Cholesterol Level Summary, mg/dL | | | | | | |
|---|---|---|---|---|---|---|
| **Study Day** | | **Placebo** | **50 mg** | **100 mg** | **200 mg** | **400 mg** |
| | Std | 33 | 50 | 27 | 25 | 16 |
| | Min - Max | 236 - 282 | 174 - 294 | 170 - 251 | 132 - 209 | 170 - 193 |
| | P-value | | 0.5333 | 0.1111 | 0.0444 | 0.3333 |
| **PD97** | N | 0 | 2 | 5 | 8 | 1 |
| | Mean | NC | 205 | 201 | 166 | 190 |
| | Median | NC | 205 | 209 | 166 | 190 |
| | Std | NC | 27 | 49 | 20 | NC |
| | Min - Max | NC | 186 - 224 | 155 - 271 | 143 - 201 | 190 - 190 |
| **PD111** | N | 0 | 1 | 3 | 6 | 2 |
| | Mean | NC | 162 | 209 | 186 | 203 |
| | Median | NC | 162 | 228 | 188 | 203 |
| | Std | NC | NC | 34 | 10 | 14 |
| | Min - Max | NC | 162 - 162 | 170 - 228 | 170 - 197 | 193 - 213 |

**Table 27**

| Total Cholesterol, % Change from Baseline | | | | | | |
|---|---|---|---|---|---|---|
| **Study Day** | | **Placebo** | **50 mg** | **100 mg** | **200 mg** | **400 mg** |
| **MD8** | N | 7 | 8 | 8 | 8 | 2 |
| | Mean | 1 | -7 | -8 | -11 | -21 |
| | Median | -6 | -10 | -11 | -12 | -21 |
| | Std | 13 | 7 | 8 | 6 | 2 |
| | Min - Max | -13 - 20 | -16-5 | -16 - 4 | -21 - -2 | -23 - -19 |
| | P-value | | 0.281 | 0.127 | 0.0721 | 0.0556 |
| **MD15** | N | 7 | 8 | 8 | 8 | 2 |
| | Mean | 3 | -4 | -19 | -23 | -32 |
| | Median | 0 | -2 | -21 | -22 | -32 |
| | Std | 11 | 7 | 12 | 15 | 0 |
| | Min - Max | -9 - 19 | -14 - 4 | -35 - 0 | -51 - -8 | -32 - -32 |
| | P-value | | 0.281 | 0.0061 | 0.0012 | 0.0556 |
| **MD22** | N | 7 | 8 | 8 | 8 | 2 |
| | Mean | -2 | -2 | -21 | -27 | -40 |
| | Median | 2 | -2 | -21 | -26 | -40 |
| | Std | 12 | 11 | 9 | 12 | 8 |
| | Min - Max | -26 - 11 | -17 - 19 | -34 - -6 | -46 - -12 | -45 - -34 |
| | P-value | | 0.6943 | 0.014 | 0.0037 | 0.0556 |
| **MD25** | N | 7 | 8 | 8 | 8 | 2 |

(continued)

| Total Cholesterol, % Change from Baseline | | | | | | |
|---|---|---|---|---|---|---|
| Study Day | | Placebo | 50 mg | 100 mg | 200 mg | 400 mg |
| | Mean | -2 | -2 | -14 | -24 | -37 |
| | Median | 0 | -1 | -13 | -23 | -37 |
| | Std | 17 | 7 | 8 | 12 | 8 |
| | Min - Max | -33 - 18 | -14 - 8 | -29 - -2 | -40 - -6 | -42 - -31 |
| | P-value | | 0.9554 | 0.0502 | 0.0132 | 0.0833 |
| **PD39** | N | 7 | 8 | 8 | 8 | 2 |
| | Mean | 7 | -1 | -12 | -27 | -39 |
| | Median | 9 | 0 | -13 | -28 | -39 |
| | Std | 10 | 9 | 8 | 14 | 11 |
| | Min - Max | -7 - 20 | -11 - 8 | -24 - -2 | -50 - -12 | -47 - -31 |
| | P-value | | 0.0721 | 0.0025 | 0.0003 | 0.0556 |
| **PD55** | N | 7 | 8 | 8 | 8 | 2 |
| | Mean | 10 | 2 | -15 | -23 | -31 |
| | Median | 5 | 4 | -13 | -27 | -31 |
| | Std | 14 | 7 | 8 | 14 | 6 |
| | Min - Max | -6 - 31 | -11 - 9 | -28 - -7 | -39 - -4 | -35 - -27 |
| | P-value | | 0.4634 | 0.0003 | 0.0006 | 0.0556 |
| **PD69** | N | 3 | 5 | 7 | 7 | 2 |
| | Mean | 1 | -3 | -12 | -27 | -38 |
| | Median | 0 | 0 | -12 | -25 | -38 |
| | Std | 5 | 11 | 6 | 11 | 10 |
| | Min - Max | -3 - 7 | -20 - 8 | -20 - -4 | -39 - -10 | -45 - -31 |
| | P-value | | 0.8571 | 0.0167 | 0.0167 | 0.2 |
| **PD83** | N | 2 | 4 | 7 | 8 | 2 |
| | Mean | 8 | 1 | -8 | -20 | -29 |
| | Median | 8 | 3 | -7 | -15 | -29 |
| | Std | 5 | 8 | 6 | 12 | 13 |
| | Min - Max | 4 - 11 | -10 - 7 | -17 - 0 | -35 - -6 | -38 - -19 |
| | P-value | | 0.2667 | 0.0556 | 0.0444 | 0.3333 |
| **PD97** | N | 0 | 2 | 5 | 8 | 1 |
| | Mean | NC | 3 | -8 | -20 | -21 |
| | Median | NC | 3 | -9 | -17 | -21 |
| | Std | NC | 10 | 10 | 11 | |
| | Min - Max | NC | -4 - 9 | -18 - 8 | -41 - -4 | -21 - -21 |
| **PD111** | N | 0 | 1 | 3 | 6 | 2 |
| | Mean | NC | -16 | 5 | -13 | -21 |
| | Median | NC | -16 | 0 | -12 | -21 |

(continued)

| Total Cholesterol, % Change from Baseline | | | | | | |
|---|---|---|---|---|---|---|
| **Study Day** | | **Placebo** | **50 mg** | **100 mg** | **200 mg** | **400 mg** |
| | Std | NC | | 14 | 8 | 2 |
| | Min - Max | NC | -16 - -16 | -6 - 20 | -25 - -4 | -23 - -19 |

**HDL-Cholesterol.** Presented in Tables 28 and 29 are the mean HDL-cholesterol levels and mean percent change relative to baseline HDL-cholesterol, respectively. No significant changes in HDL-cholesterol levels were observed, as would be expected since HDL does not include an apolipoprotein B component. Just as elevated LDL-cholesterol is a risk factor for cardiovascular disease, reduced HDL-cholesterol is also a risk factor that is considered when determining whether an individual is in need of lipid-lowering therapy. Thus, that ISIS 301012 did not adversely affect HDL-cholesterol levels is a positive therapeutic outcome.

**Table 28**

| HDL-Cholesterol Level Summary, mg/dL | | | | | | |
|---|---|---|---|---|---|---|
| **Study Day** | | **Placebo** | **50 mg** | **100 mg** | **200 mg** | **400 mg** |
| **Baseline** | N | 7 | 8 | 8 | 9 | 4 |
| | Mean | 51 | 59 | 49 | 53 | 59 |
| | Median | 44 | 52 | 45 | 48 | 57 |
| | Std | 19 | 18 | 11 | 13 | 17 |
| | Min - Max | 29 - 83 | 44 - 98 | 39 - 72 | 33 - 70 | 41 - 81 |
| | P-value | | 0.1893 | 0.7789 | 0.4869 | 0.6485 |
| **MD8** | N | 7 | 7 | 8 | 8 | 2 |
| | Mean | 48 | 49 | 47 | 47 | 44 |
| | Median | 53 | 52 | 44 | 45 | 44 |
| | Std | 13 | 13 | 10 | 10 | 21 |
| | Min-Max | 29 - 68 | 28 - 68 | 36 - 64 | 35 - 62 | 29 - 59 |
| | P-value | | 0.9015 | 0.7789 | 0.8665 | 1 |
| **MD15** | N | 7 | 8 | 8 | 8 | 2 |
| | Mean | 48 | 56 | 45 | 48 | 42 |
| | Median | 53 | 53 | 43 | 43 | 42 |
| | Std | 15 | 18 | 8 | 13 | 18 |
| | Min - Max | 29 - 67 | 36 - 94 | 36 - 64 | 35 - 68 | 29 - 54 |
| | P-value | | 0.5358 | 0.8429 | 0.7789 | 0.5 |
| **MD22** | N | 7 | 8 | 8 | 8 | 2 |
| | Mean | 51 | 57 | 46 | 52 | 54 |
| | Median | 52 | 52 | 42 | 48 | 54 |
| | Std | 18 | 14 | 11 | 15 | 21 |
| | Min - Max | 28 - 78 | 45 - 87 | 37 - 65 | 34 - 74 | 39 - 69 |
| | P-value | | 0.7789 | 0.7542 | 1 | 0.8889 |
| **MD25** | N | 7 | 8 | 8 | 8 | 2 |
| | Mean | 52 | 60 | 49 | 52 | 58 |

(continued)

| HDL-Cholesterol Level Summary, mg/dL | | | | | | |
|---|---|---|---|---|---|---|
| **Study Day** | | **Placebo** | **50 mg** | **100 mg** | **200 mg** | **400 mg** |
| | Median | 55 | 55 | 46 | 46 | 58 |
| | Std | 17 | 15 | 11 | 14 | 23 |
| | Min - Max | 28 - 75 | 48 - 94 | 37 - 69 | 34 - 73 | 42 - 74 |
| | P-value | | 0.6126 | 0.8665 | 0.9551 | 0.8889 |
| **PD39** | N | 7 | 8 | 8 | 8 | 2 |
| | Mean | 55 | 62 | 50 | 53 | 63 |
| | Median | 56 | 58 | 49 | 52 | 63 |
| | Std | 19 | 17 | 11 | 14 | 25 |
| | Min - Max | 27 - 82 | 43 - 99 | 38 - 73 | 29 - 71 | 45 - 80 |
| | P-value | | 0.5358 | 0.5358 | 1 | 0.6667 |
| **PD55** | N | 7 | 8 | 8 | 8 | 2 |
| | Mean | 55 | 65 | 50 | 56 | 72 |
| | Median | 56 | 63 | 49 | 54 | 72 |
| | Std | 18 | 19 | 11 | 16 | 17 |
| | Min - Max | 33 - 86 | 41 - 100 | 38 - 74 | 33 - 80 | 60 - 84 |
| | P-value | | 0.3357 | 0.4634 | 0.9551 | 0.5 |
| **PD69** | N | 3 | 4 | 5 | 7 | 2 |
| | Mean | 61 | 67 | 49 | 52 | 66 |
| | Median | 73 | 65 | 46 | 54 | 66 |
| | Std | 26 | 12 | 13 | 15 | 27 |
| | Min - Max | 32 - 79 | 57 - 83 | 35 - 68 | 30 - 73 | 47 - 85 |
| | P-value | | 1 | 0.5714 | 0.3833 | 0.8 |
| **PD83** | N | 2 | 4 | 7 | 8 | 2 |
| | Mean | 56 | 65 | 49 | 53 | 66 |
| | Median | 56 | 63 | 44 | 54 | 66 |
| | Std | 33 | 6 | 13 | 15 | 31 |
| | Min - Max | 32 - 79 | 62 - 75 | 38 - 75 | 31 - 77 | 45 - 88 |
| | P-value | | 1 | 1 | 0.8889 | 0.6667 |
| **PD97** | N | 0 | 2 | 5 | 8 | 1 |
| | Mean | NC | 62 | 52 | 52 | 87 |
| | Median | NC | 62 | 53 | 54 | 87 |
| | Std | NC | 3 | 16 | 13 | |
| | Min - Max | NC | 59 - 64 | 37 - 77 | 32 - 71 | 87 - 87 |
| **PD111** | N | 0 | 1 | 2 | 6 | 2 |
| | Mean | NC | 55 | 63 | 56 | 65 |
| | Median | NC | 55 | 63 | 61 | 65 |
| | Std | NC | . | 11 | 13 | 27 |

(continued)

| HDL-Cholesterol Level Summary, mg/dL | | | | | | |
|---|---|---|---|---|---|---|
| Study Day | | Placebo | 50 mg | 100 mg | 200 mg | 400 mg |
| | Min - Max | NC | 55 - 55 | 56 - 71 | 31 - 68 | 46 - 84 |

**Table 29**

| HDL-Cholesterol, % Change from Baseline | | | | | | |
|---|---|---|---|---|---|---|
| **Study Day** | | **Placebo** | **50 mg** | **100 mg** | **200 mg** | **400 mg** |
| **MD8** | N | 7 | 7 | 8 | 8 | 2 |
| | Mean | -2 | -9 | -5 | -7 | -28 |
| | Median | -5 | -5 | -5 | -8 | -28 |
| | Std | 19 | 16 | 10 | 11 | 2 |
| | Min - Max | -31 - 22 | -35 - 9 | -19 - 11 | -27 - 8 | -29 - -27 |
| | P-value | | 0.7104 | 0.7789 | 0.7789 | 0.2222 |
| **MD15** | N | 7 | 8 | 8 | 8 | 2 |
| | Mean | -3 | -6 | -8 | -6 | -31 |
| | Median | -10 | -7 | -9 | -6 | -31 |
| | Std | 20 | 8 | 10 | 8 | 3 |
| | Min - Max | -22 - 24 | -17 - 5 | -22 - 3 | -23 - 6 | -33 - -29 |
| | P-value | | 0.6943 | 0.9551 | 0.9551 | 0.0556 |
| **MD22** | N | 7 | 8 | 8 | 8 | 2 |
| | Mean | 1 | -2 | -6 | -1 | -10 |
| | Median | -7 | -5 | -6 | 3 | -10 |
| | Std | 19 | 10 | 12 | 10 | 7 |
| | Min - Max | -21 - 37 | -11 - 16 | -27 - 9 | -21 - 10 | -15 - -5 |
| | P-value | | 0.8665 | 0.6126 | 0.7789 | 0.6667 |
| **MD25** | N | 7 | 8 | 8 | 8 | 2 |
| | Mean | 2 | 3 | 0 | 0 | -3 |
| | Median | -5 | 2 | -2 | 2 | -3 |
| | Std | 19 | 6 | 13 | 5 | 7 |
| | Min-Max | -17 - 30 | -4 - 11 | -22 - 15 | -10 - 5 | -8 - 2 |
| | P-value | | 0.1893 | 0.8665 | 0.3969 | 1.0000 |
| **PD39** | N | 7 | 8 | 8 | 8 | 2 |
| | Mean | 7 | 6 | 1 | 1 | 5 |
| | Median | -2 | 6 | -1 | 1 | 5 |
| | Std | 20 | 10 | 13 | 10 | 8 |
| | Min - Max | -9 - 42 | -12 - 19 | -17 - 21 | -12 - 20 | 0 - 11 |
| | P-value | | 0.4634 | 0.7789 | 0.9551 | 0.5000 |
| **PD55** | N | 7 | 8 | 8 | 8 | 2 |

(continued)

| HDL-Cholesterol, % Change from Baseline | | | | | | |
|---|---|---|---|---|---|---|
| **Study Day** | | **Placebo** | **50 mg** | **100 mg** | **200 mg** | **400 mg** |
| | Mean | 10 | 11 | 1 | 7 | 25 |
| | Median | 3 | 7 | 3 | 11 | 25 |
| | Std | 18 | 17 | 9 | 9 | 30 |
| | Min - Max | -11 - 40 | -7 - 45 | -13 - 15 | -12 - 15 | 4 - 46 |
| | P-value | | 0.8421 | 0.4448 | 0.9294 | 0.3056 |
| **PD69** | N | 3 | 4 | 5 | 7 | 2 |
| | Mean | 2 | 19 | -2 | -1 | 10 |
| | Median | 2 | 19 | -5 | 2 | 10 |
| | Std | 7 | 7 | 19 | 9 | 6 |
| | Min - Max | -5 - 9 | 12 - 28 | -20 - 29 | -15 - 13 | 5 - 14 |
| | P-value | | 0.0571 | 0.3929 | 0.5167 | 0.4000 |
| **PD83** | N | 2 | 4 | 7 | 8 | 2 |
| | Mean | 10 | 26 | 0 | 2 | 9 |
| | Median | 10 | 26 | 2 | 2 | 9 |
| | Std | 0 | 11 | 15 | 13 | 0 |
| | Min - Max | 10 - 10 | 12 - 38 | -22 - 28 | -15 - 27 | 9 - 9 |
| **PD97** | P-value | | 0.1333 | 0.2222 | 0.1778 | 0.3333 |
| | N | 0 | 2 | 5 | 8 | 1 |
| | Mean | NC | 18 | 5 | 1 | 8 |
| | Median | NC | 18 | -2 | 0 | 8 |
| | Std | NC | 0 | 14 | 10 | NC |
| **PD111** | Min - Max | NC | 18 - 18 | -5 - 29 | -9 - 17 | 8 - 8 |
| | N | 0 | 1 | 2 | 6 | 2 |
| | Mean | NC | 9 | -1 | 3 | 8 |
| | Median | NC | 9 | -1 | -1 | 8 |
| | Std | NC | NC | 1 | 15 | 5 |
| | Min - Max | NC | 9 - 9 | -2 - 0 | -10 - 31 | 5 - 12 |

**Triglyceride.** Triglyceride level summaries and percent changes are presented in Tables 30 and 31, respectively. Dose-dependent reductions in triglyceride levels were observed, with maximum reductions of 27% in the 200 mg group and 43% in the 400 mg group (Table 31). Elevated serum triglyceride levels may be considered an independent risk factor for coronary heart disease, thus a reduction in triglyceride levels is a therapeutically desirable outcome.

**Table 30**

| Triglyceride Level Summary, mg/dl | | | | | | |
|---|---|---|---|---|---|---|
| **Study Day** | | **Placebo** | **50 mg** | **100 mg** | **200 mg** | **400 mg** |
| **Baseline** | N | 7 | 8 | 8 | 9 | 4 |
| | Mean | 104 | 95 | 105 | 103 | 127 |
| | Median | 76 | 94 | 88 | 88 | 108 |

(continued)

| Triglyceride Level Summary, mg/dl | | | | | | |
|---|---|---|---|---|---|---|
| **Study Day** | | **Placebo** | **50 mg** | **100 mg** | **200 mg** | **400 mg** |
| | Std | 90 | 29 | 47 | 44 | 57 |
| | Min - Max | 56 - 307 | 55 - 139 | 65 - 212 | 51 - 179 | 82 - 209 |
| | P-value | | 0.3512 | 0.0875 | 0.3652 | 0.0727 |
| **MD8** | N | 7 | 7 | 8 | 8 | 2 |
| | Mean | 127 | 114 | 116 | 112 | 149 |
| | Median | 99 | 69 | 112 | 88 | 149 |
| | Std | 82 | 71 | 48 | 63 | 86 |
| | Min - Max | 41 - 286 | 51 - 239 | 37 - 209 | 57 - 232 | 88 - 209 |
| | P-value | | 0.6200 | 0.8906 | 0.9551 | 0.6667 |
| **MD15** | N | 7 | 8 | 8 | 8 | 2 |
| | Mean | 146 | 109 | 126 | 100 | 123 |
| | Median | 108 | 98 | 117 | 84 | 123 |
| | Std | 113 | 46 | 61 | 39 | 4 |
| | Min - Max | 50 - 371 | 46 - 178 | 34 - 231 | 72 - 172 | 120 - 126 |
| | P-value | | 0.6943 | 1.0000 | 0.6733 | 0.8889 |
| **MD22** | N | 7 | 8 | 8 | 8 | 2 |
| | Mean | 118 | 104 | 122 | 99 | 129 |
| | Median | 78 | 101 | 137 | 77 | 129 |
| | Std | 84 | 20 | 48 | 51 | 28 |
| | Min - Max | 56 - 291 | 80 - 133 | 56 - 204 | 50 - 199 | 109 - 149 |
| | P-value | | 0.5142 | 0.6319 | 1.0000 | 0.6111 |
| **MD25** | N | 7 | 8 | 8 | 8 | 2 |
| | Mean | 110 | 95 | 114 | 101 | 91 |
| | Median | 90 | 86 | 108 | 97 | 91 |
| | Std | 83 | 24 | 47 | 47 | 32 |
| | Min - Max | 45 - 288 | 72 - 136 | 69 - 218 | 48 - 196 | 68-113 |
| | P-value | | 1.0000 | 0.4634 | 0.7167 | 0.9444 |
| **PD39** | N | 7 | 8 | 8 | 8 | 2 |
| | Mean | 113 | 116 | 114 | 91 | 83 |
| | Median | 63 | 99 | 98 | 84 | 83 |
| | Std | 89 | 46 | 61 | 36 | 26 |
| | Min - Max | 56 - 297 | 75 - 207 | 45 - 240 | 49 - 138 | 64 - 101 |
| | P-value | | 0.3201 | 0.5894 | 0.9336 | 0.8333 |
| **PD55** | N | 7 | 8 | 8 | 8 | 2 |
| | Mean | 97 | 93 | 146 | 77 | 103 |
| | Median | 59 | 81 | 115 | 77 | 103 |
| | Std | 65 | 41 | 103 | 23 | 51 |

(continued)

| Triglyceride Level Summary, mg/dl | | | | | | |
|---|---|---|---|---|---|---|
| **Study Day** | | **Placebo** | **50 mg** | **100 mg** | **200 mg** | **400 mg** |
| | Min - Max | 52 - 226 | 49 - 168 | 56 - 389 | 41 - 109 | 67 - 139 |
| | P-value | | 0.8026 | 0.1520 | 0.8665 | 0.5000 |
| **PD69** | N | 3 | 4 | 5 | 7 | 2 |
| | Mean | 219 | 110 | 197 | 103 | 106 |
| | Median | 92 | 110 | 135 | 90 | 106 |
| | Std | 248 | 36 | 146 | 57 | 6 |
| | Min - Max | 61 - 505 | 67 - 152 | 98 - 451 | 41 - 222 | 101 - 110 |
| | P-value | | 0.8571 | 0.5714 | 0.8333 | 0.8000 |
| **PD83** | N | 2 | 4 | 7 | 8 | 2 |
| | Mean | 237 | 94 | 131 | 134 | 148 |
| | Median | 237 | 96 | 108 | 107 | 148 |
| | Std | 199 | 36 | 72 | 74 | 59 |
| | Min - Max | 96 - 378 | 47 - 135 | 38 - 243 | 55 - 262 | 106 - 190 |
| | P-value | | 0.4667 | 0.6667 | 0.5333 | 1.0000 |
| **PD97** | N | 0 | 2 | 5 | 8 | 1 |
| | Mean | NC | 147 | 149 | 99 | 83 |
| | Median | NC | 147 | 128 | 85 | 83 |
| | Std | NC | 50 | 112 | 56 | NC |
| | Min - Max | NC | 111 - 182 | 59 - 337 | 60 - 232 | 83 - 83 |
| **PD111** | N | 0 | 1 | 2 | 6 | 2 |
| | Mean | NC | 151 | 96 | 118 | 109 |
| | Median | NC | 151 | 96 | 80 | 109 |
| | Std | NC | NC | 68 | 106 | 34 |
| | Min - Max | NC | 151 - 151 | 48 - 144 | 46 - 326 | 85 - 133 |

**Table 31**

| Triglyceride Mean, % from Baseline | | | | | | |
|---|---|---|---|---|---|---|
| **Study Day** | | **Placebo** | **50 mg** | **100 mg** | **200 mg** | **400 mg** |
| **MD8** | N | 7 | 7 | 8 | 8 | 2 |
| | Mean | 32 | 8 | 17 | -1 | -5 |
| | Median | 17 | 0 | 11 | -4 | -5 |
| | Std | 53 | 44 | 49 | 27 | 7 |
| | Min - Max | -27 - 108 | -41 - 72 | -54 - 117 | -34 - 52 | -9 - 0 |
| | P-value | | 0.3829 | 0.9551 | 0.3357 | 0.5 |
| **MD15** | N | 7 | 8 | 8 | 8 | 2 |
| | Mean | 45 | 17 | 34 | -6 | -6 |

(continued)

| Triglyceride Mean, % from Baseline | | | | | | |
|---|---|---|---|---|---|---|
| **Study Day** | | **Placebo** | **50 mg** | **100 mg** | **200 mg** | **400 mg** |
| | Median | 21 | 25 | 8 | 0 | -6 |
| | Std | 64 | 37 | 97 | 20 | 51 |
| | Min - Max | -24 - 166 | -47 - 50 | -58 - 255 | -42 - 22 | -43 - 30 |
| | P-value | | 0.9551 | 0.6126 | 0.0289 | 0.5 |
| **MD22** | N | 7 | 8 | 8 | 8 | 2 |
| | Mean | 21 | 18 | 21 | -8 | -8 |
| | Median | 0 | 13 | 8 | -6 | -8 |
| | Std | 41 | 38 | 47 | 28 | 29 |
| | Min - Max | -13 - 101 | -35 - 91 | -30 - 117 | -64 - 30 | -29 - 12 |
| | P-value | | 1 | 0.9551 | 0.1206 | 0.5 |
| **MD25** | N | 7 | 8 | 8 | 8 | 2 |
| | Mean | 10 | 4 | 12 | -7 | -38 |
| | Median | 17 | 4 | 1 | -7 | -38 |
| | Std | 32 | 26 | 29 | 26 | 11 |
| | Min - Max | -38 - 47 | -34 - 35 | -14 - 69 | -46 - 28 | -46 - -30 |
| | P-value | | 0.6943 | 1 | 0.281 | 0.1111 |
| **PD39** | N | 7 | 8 | 8 | 8 | 2 |
| | Mean | 12 | 23 | 10 | -15 | -43 |
| | Median | -3 | 16 | 4 | -20 | -43 |
| | Std | 43 | 35 | 44 | 23 | 13 |
| | Min - Max | -18 - 99 | -13 - 92 | -44 - 106 | -44 - 30 | -52 - -34 |
| | P-value | | 0.281 | 0.7789 | 0.152 | 0.0556 |
| **PD55** | N | 7 | 8 | 8 | 8 | 2 |
| | Mean | 1 | -1 | 37 | -27 | -32 |
| | Median | -11 | -6 | 20 | -34 | -32 |
| | Std | 38 | 30 | 60 | 20 | 2 |
| | Min - Max | -30 - 70 | -52 - 56 | -30 - 160 | -43 - 18 | -34 - -31 |
| | P-value | | 0.7789 | 0.2319 | 0.0541 | 0.0556 |
| **PD69** | N | 3 | 4 | 5 | 7 | 2 |
| | Mean | 30 | 12 | 99 | -9 | -19 |
| | Median | 17 | 17 | 14 | -11 | -19 |
| | Std | 30 | 21 | 176 | 34 | 46 |
| | Min - Max | 9 - 65 | -16 - 29 | -14 - 407 | -54 - 45 | -52 - 13 |
| | P-value | | 0.6286 | 1 | 0.1833 | 0.4 |
| **PD83** | N | 2 | 4 | 7 | 8 | 2 |
| | Mean | 22 | -2 | 31 | 17 | 23 |
| | Median | 22 | -1 | 21 | 22 | 23 |

(continued)

| Triglyceride Mean, % from Baseline | | | | | | |
|---|---|---|---|---|---|---|
| Study Day | | Placebo | 50 mg | 100 mg | 200 mg | 400 mg |
| | Std | 1 | 26 | 70 | 30 | 103 |
| | Min - Max | 22 - 23 | -32 - 25 | -53 - 170 | -25 - 71 | -49 - 96 |
| | P-value | | 0.5333 | 0.8889 | 1 | 1 |
| **PD97** | N | 0 | 2 | 5 | 8 | 1 |
| | Mean | NC | 57 | 24 | -9 | -14 |
| | Median | NC | 57 | 6 | -16 | -14 |
| | Std | NC | 6 | 43 | 31 | NC |
| | Min - Max | NC | 53 - 61 | -26 - 78 | -39 - 52 | -14 - -14 |
| **PD111** | N | 0 | 1 | 2 | 6 | 2 |
| | Mean | NC | 27 | 41 | 6 | -24 |
| | Median | NC | 27 | 41 | -16 | -24 |
| | Std | NC | NC | 114 | 57 | 17 |
| | Min - Max | NC | 27 - 27 | -40 - 122 | -45 - 113 | -36 - -12 |

**Lipoprotein(a).** Tables 32 and 33 show mean lipoprotein(a) levels and mean lipoprotein(a) changes relative to baseline, respectively. Lipoprotein(a) has been linked with the development and progression of atherosclerosis, thus its reduction can be a goal of lipid-lowering therapies. A maximal reduction of approximately 22% was observed was in the 200 mg group at MD22, however, no other dose-dependent statistically significant reductions in lipoprotein(a) were observed.

**Table 32**

| Lipoprotein(a) Level Summary, mg/dL | | | | | | |
|---|---|---|---|---|---|---|
| Study Day | | Placebo | 50 mg | 100 mg | 200 mg | 400 mg |
| **Baseline** | N | 7 | 8 | 8 | 9 | 4 |
| | Mean | 12 | 16 | 10 | 24 | 6 |
| | Median | 4 | 7 | 7 | 11 | 5 |
| | Std | 16 | 24 | 11 | 27 | 4 |
| | Min - Max | 1 - 45 | 1 - 71 | 1 - 29 | 3 - 73 | 1- 10 |
| | P-value | | 0.6876 | 0.9549 | 0.2507 | 0.8242 |
| **MD8** | N | 7 | 7 | 8 | 8 | 2 |
| | Mean | 12 | 6 | 11 | 25 | 4 |
| | Median | 6 | 3 | 6 | 10 | 4 |
| | Std | 15 | 7 | 13 | 29 | 4 |
| | Min - Max | 1 - 43 | 1 - 21 | 1 - 32 | 4 - 84 | 1 - 7 |
| | P-value | | 0.5973 | 0.8291 | 0.2213 | 0.6389 |
| **MD15** | N | 7 | 8 | 8 | 8 | 2 |
| | Mean | 13 | 17 | 10 | 22 | 5 |
| | Median | 5 | 5 | 7 | 8 | 5 |
| | Std | 15 | 27 | 11 | 26 | 6 |

(continued)

| Lipoprotein(a) Level Summary, mg/dL | | | | | | |
|---|---|---|---|---|---|---|
| **Study Day** | | **Placebo** | **50 mg** | **100 mg** | **200 mg** | **400 mg** |
| | Min - Max | 1 - 41 | 1 - 81 | 1 - 30 | 6 - 67 | 1 - 9 |
| | P-value | | 0.8379 | 0.7739 | 0.2681 | 0.6389 |
| **MD22** | N | 7 | 8 | 8 | 8 | 2 |
| | Mean | 13 | 15 | 8 | 21 | 4 |
| | Median | 3 | 3 | 3 | 8 | 4 |
| | Std | 16 | 26 | 11 | 26 | 4 |
| | Min - Max | 1 - 44 | 1 - 75 | 1 - 31 | 3 - 63 | 1 - 7 |
| | P-value | | 0.8632 | 0.6061 | 0.2674 | 0.6389 |
| **MD25** | N | 7 | 8 | 8 | 8 | 2 |
| | Mean | 14 | 17 | 10 | 22 | 4 |
| | Median | 5 | 6 | 4 | 9 | 4 |
| | Std | 18 | 30 | 12 | 26 | 5 |
| | Min - Max | 1 - 50 | 1 - 89 | 1 - 31 | 2 - 65 | 1 - 7 |
| | P-value | | 0.9549 | 0.7629 | 0.3340 | 0.7500 |
| **PD39** | N | 7 | 8 | 8 | 8 | 2 |
| | Mean | 14 | 14 | 8 | 23 | 3 |
| | Median | 9 | 3 | 4 | 11 | 3 |
| | Std | 15 | 27 | 9 | 27 | 3 |
| | Min - Max | 1 - 38 | 1 - 80 | 1 - 21 | 2 - 70 | 1 - 6 |
| | P-value | | 0.6876 | 0.5820 | 0.4434 | 0.5000 |
| **PD55** | N | 7 | 8 | 8 | 8 | 2 |
| | Mean | 13 | 14 | 8 | 21 | 1 |
| | Median | 4 | 3 | 1 | 8 | 1 |
| | Std | 15 | 25 | 11 | 24 | 0 |
| | Min - Max | 1 - 42 | 1 - 74 | 1 - 28 | 4 - 60 | 1 - 1 |
| | P-value | | 0.6061 | 0.2211 | 0.2810 | 0.1389 |
| **PD69** | N | 3 | 4 | 5 | 7 | 2 |
| | Mean | 2 | 4 | 9 | 23 | 3 |
| | Median | 1 | 3 | 6 | 13 | 3 |
| | Std | 2 | 3 | 11 | 25 | 2 |
| | Min - Max | 1 - 4 | 1 - 8 | 1 - 27 | 3 - 61 | 1 - 4 |
| | P-value | | 0.5429 | 0.3214 | 0.0333 | 0.7000 |
| **PD83** | N | 2 | 4 | 7 | 8 | 2 |
| | Mean | 3 | 3 | 9 | 21 | 4 |
| | Median | 3 | 1 | 4 | 13 | 4 |
| | Std | 2 | 4 | 10 | 26 | 4 |
| | Min - Max | 1 - 4 | 1 - 9 | 1 - 23 | 1 - 65 | 1 - 7 |

(continued)

| Lipoprotein(a) Level Summary, mg/dL | | | | | | |
|---|---|---|---|---|---|---|
| Study Day | | Placebo | 50 mg | 100 mg | 200 mg | 400 mg |
| | P-value | | 1.0000 | 0.5000 | 0.4000 | 1.0000 |
| PD97 | N | 0 | 2 | 5 | 8 | 1 |
| | Mean | NC | 1 | 14 | 22 | 1 |
| | Median | NC | 1 | 10 | 12 | 1 |
| | Std | NC | 0 | 11 | 25 | |
| | Min - Max | NC | 1 - 1 | 1 - 27 | 1 - 66 | 1 - 1 |
| PD111 | N | 0 | 1 | 2 | 6 | 2 |
| | Mean | NC | 3 | 28 | 23 | 5 |
| | Median | NC | 3 | 28 | 10 | 5 |
| | Std | NC | | 2 | 33 | 5 |
| | Min - Max | NC | 3 - 3 | 27 - 30 | 4 - 90 | 1 - 9 |

**Table 33**

| Lipoprotein(a), % Change from Baseline | | | | | | |
|---|---|---|---|---|---|---|
| Study Day | | Placebo | 50 mg | 100 mg | 200 mg | 400 mg |
| MD8 | N | 7 | 7 | 8 | 8 | 2 |
| | Mean | 32 | -12 | -6 | 1 | -17 |
| | Median | 0 | -21 | 0 | -7 | -17 |
| | Std | 75 | 61 | 31 | 22 | 25 |
| | Min - Max | -21 - 190 | -82 - 110 | -60 - 41 | -24 - 40 | -35 - 0 |
| | P-value | | 0.2063 | 0.8432 | 0.4448 | 0.4722 |
| MD15 | N | 7 | 8 | 8 | 8 | 2 |
| | Mean | 26 | 29 | -5 | -8 | -5 |
| | Median | 0 | 27 | 0 | -15 | -5 |
| | Std | 56 | 77 | 20 | 34 | 6 |
| | Min - Max | -9 - 150 | -80 - 140 | -41 - 28 | -37 - 74 | -9 - 0 |
| | P-value | | 1 | 0.3893 | 0.0138 | 0.4444 |
| MD22 | N | 7 | 8 | 8 | 8 | 2 |
| | Mean | 25 | 6 | -24 | -22 | -14 |
| | Median | 0 | -9 | -20 | -23 | -14 |
| | Std | 65 | 110 | 27 | 15 | 20 |
| | Min - Max | -18 - 170 | -89 - 250 | -66 - 5 | -49 - -3 | -29 - 0 |
| | P-value | | 0.4625 | 0.0476 | 0.0034 | 0.3333 |
| MD25 | N | 7 | 8 | 8 | 8 | 2 |
| | Mean | 11 | -9 | -14 | -20 | -13 |
| | Median | 9 | -6 | -6 | -15 | -13 |

(continued)

| Lipoprotein(a), % Change from Baseline | | | | | | |
|---|---|---|---|---|---|---|
| Study Day | | Placebo | 50 mg | 100 mg | 200 mg | 400 mg |
| | Std | 14 | 32 | 28 | 13 | 19 |
| | Min - Max | 0 - 39 | -50 - 38 | -60 - 24 | -41 - -4 | -27 - 0 |
| | P-value | | 0.1377 | 0.0659 | 0.0003 | 0.1667 |
| **PD39** | N | 7 | 8 | 8 | 8 | 2 |
| | Mean | 46 | -31 | -19 | -15 | -22 |
| | Median | 7 | -30 | -17 | -9 | -22 |
| | Std | 65 | 35 | 23 | 27 | 32 |
| | Min - Max | -17 - 139 | -78 - 13 | -59 - 8 | -75 - 9 | -45 - 0 |
| | P-value | | 0.0176 | 0.0253 | 0.0499 | 0.2222 |
| **PD55** | N | 7 | 8 | 8 | 8 | 2 |
| | Mean | 41 | -26 | -30 | -14 | -45 |
| | Median | 8 | -4 | -22 | -16 | -45 |
| | Std | 65 | 41 | 33 | 28 | 64 |
| | Min - Max | -7 - 140 | -89 - 16 | -91 - 0 | -52 - 41 | -90 - 0 |
| | P-value | | 0.039 | 0.0034 | 0.014 | 0.1389 |
| **PD69** | N | 3 | 4 | 5 | 7 | 2 |
| | Mean | 3 | -16 | -12 | -5 | -29 |
| | Median | 0 | -20 | -5 | -10 | -29 |
| | Std | 4 | 66 | 20 | 38 | 41 |
| | Min - Max | 0 - 8 | -89 - 67 | -48 - 0 | -68 - 43 | -57 - 0 |
| | P-value | | 0.6286 | 0.125 | 0.7833 | 0.6 |
| **PD83** | N | 2 | 4 | 7 | 8 | 2 |
| | Mean | 4 | -22 | -28 | -20 | -18 |
| | Median | 4 | 0 | -20 | -17 | -18 |
| | Std | 5 | 45 | 26 | 55 | 25 |
| | Min - Max | 0 - 8 | -89 - 2 | -60 - 0 | -91 - 81 | -36 - 0 |
| | P-value | | 0.4667 | 0.1389 | 0.4 | 0.6667 |
| **PD97** | N | 0 | 2 | 5 | 8 | 1 |
| | Mean | NC | -45 | -15 | 4 | 0 |
| | Median | NC | -45 | -20 | -13 | 0 |
| | Std | NC | 63 | 20 | 69 | |
| | Min - Max | NC | -89 - 0 | -38 - 12 | -91 - 141 | 0.0 - 0.0 |
| **PD111** | N | 0 | 1 | 2 | 6 | 2 |
| | Mean | NC | -70 | 8 | 24 | -7 |
| | Median | NC | -70 | 8 | 5 | -7 |
| | Std | NC | | 23 | 80 | 11 |
| | Min - Max | NC | -70 - -70 | -8 - 24 | -63 - 156 | -15 - 0 |

**Total Cholesterol versus HDL-Cholesterol.** Improvements in lipoprotein levels can be assessed by comparing the ratio of total cholesterol to HDL-cholesterol. A decrease in this ratio indicates an improvement in a lipoprotein profile, and such a decrease can occur either through reduction in total cholesterol, increase in HDL-cholesterol, or a combination of changes in both parameters. As would be expected following a decrease in total cholesterol and no change in HDL-cholesterol, this ratio was improved following ISIS 301012 treatment. For example, in the 200 mg group, a maximal reduction of approximately 29% was observed at PD55 (Table 35).

**Table 34**

| Total CholesterolHDL-Cholesterol Ratio, Summary | | | | | | |
|---|---|---|---|---|---|---|
| **Study Day** | | **Placebo** | **50 mg** | **100 mg** | **200 mg** | **400 mg** |
| **Baseline** | N | 7 | 8 | 8 | 9 | 4 |
| | Mean | 5 | 4 | 4 | 4 | 4 |
| | Median | 4 | 4 | 4 | 4 | 4 |
| | Std | 2 | 1 | 1 | 1 | 2 |
| | Min - Max | 3 - 9 | 2 - 5 | 2 - 6 | 3 - 6 | 3 - 7 |
| | P-value | | 0.8665 | 0.9551 | 0.7577 | 1 |
| **MD8** | N | 7 | 7 | 8 | 8 | 2 |
| | Mean | 5 | 4 | 4 | 4 | 5 |
| | Median | 4 | 4 | 4 | 4 | 5 |
| | Std | 2 | 1 | 1 | 1 | 2 |
| | Min-Max | 3 - 8 | 3 - 6 | 3 - 7 | 3 - 6 | 3 - 7 |
| | P-value | | 0.8316 | 0.6943 | 0.3357 | 1 |
| **MD15** | N | 7 | 8 | 8 | 8 | 2 |
| | Mean | 5 | 4 | 4 | 3 | 4 |
| | Median | 4 | 4 | 4 | 3 | 4 |
| | Std | 2 | 1 | 1 | 0 | 2 |
| | Min - Max | 3 - 9 | 2 - 5 | 3 - 6 | 3 - 4 | 3 - 6 |
| | P-value | | 0.6126 | 0.3514 | 0.0289 | 0.6667 |
| **MD22** | N | 7 | 8 | 8 | 8 | 2 |
| | Mean | 5 | 4 | 4 | 3 | 3 |
| | Median | 4 | 4 | 4 | 3 | 3 |
| | Std | 2 | 1 | 1 | 1 | 1 |
| | Min - Max | 3 - 9 | 2 - 5 | 3 - 6 | 2 - 4 | 2 - 4 |
| | P-value | | 0.843 | 0.8665 | 0.0939 | 0.5 |
| **MD25** | N | 7 | 8 | 8 | 8 | 2 |
| | Mean | 5 | 4 | 4 | 3 | 3 |
| | Median | 4 | 4 | 4 | 3 | 3 |
| | Std | 2 | 1 | 1 | 1 | 1 |
| | Min - Max | 3 - 10 | 3 - 5 | 3 - 6 | 2 - 4 | 2 - 4 |
| | P-value | | 0.7559 | 0.9312 | 0.0876 | 0.6389 |
| **PD39** | N | 7 | 8 | 8 | 8 | 2 |
| | Mean | 5 | 4 | 4 | 3 | 3 |

(continued)

| Total CholesterolHDL-Cholesterol Ratio, Summary | | | | | | |
|---|---|---|---|---|---|---|
| **Study Day** | | **Placebo** | **50 mg** | **100 mg** | **200 mg** | **400 mg** |
| | Median | 4 | 4 | 4 | 3 | 3 |
| | Std | 2 | 1 | 1 | 1 | 1 |
| | Min - Max | 3 - 9 | 3 - 5 | 3 - 6 | 2 - 4 | 2 - 3 |
| | P-value | | 0.2949 | 0.7169 | 0.0541 | 0.0556 |
| **PD55** | N | 7 | 8 | 8 | 8 | 2 |
| v | Mean | 5 | 4 | 4 | 3 | 2 |
| | Median | 4 | 3 | 4 | 3 | 2 |
| | Std | 2 | 1 | 1 | 1 | 1 |
| | Min - Max | 3 - 8 | 3 - 5 | 3 - 6 | 2 - 4 | 2 - 3 |
| | P-value | | 0.2438 | 0.4634 | 0.0225 | 0.1111 |
| **PD69** | N | 3 | 4 | 5 | 7 | 2 |
| | Mean | 5 | 3 | 4 | 3 | 3 |
| | Median | 3 | 3 | 4 | 3 | 3 |
| | Std | 3 | 1 | 2 | 1 | 1 |
| | Min - Max | 3 - 7 | 3 - 4 | 3 - 6 | 2 - 4 | 2 - 3 |
| | P-value | | 0.4 | 0.7857 | 0.35 | 0.8 |
| **PD83** | N | 2 | 4 | 7 | 8 | 2 |
| | Mean | 5 | 3 | 4 | 3 | 3 |
| | Median | 5 | 3 | 4 | 3 | 3 |
| | Std | 3 | 1 | 1 | 1 | 1 |
| | Min - Max | 3 - 8 | 3 - 4 | 2 - 6 | 2 - 4 | 2 - 4 |
| | P-value | | 0.5333 | 0.8889 | 0.4 | 0.6667 |
| **PD97** | N | 0 | 2 | 5 | 8 | 1 |
| | Mean | NC | 3 | 4 | 3 | 2 |
| | Median | NC | 3 | 4 | 3 | 2 |
| | Std | NC | 0 | 2 | 1 | NC |
| | Min - Max | NC | 3 - 3 | 2 - 6 | 2 - 5 | 2 - 2 |
| **PD111** | N | 0 | 1 | 2 | 6 | 2 |
| | Mean | NC | 3 | 4 | 3 | 3 |
| | Median | NC | 3 | 4 | 3 | 3 |
| | Std | NC | NC | 1 | 1 | 1 |
| | Min - Max | NC | 3 - 3 | 3 - 4 | 3 - 6 | 2 - 4 |

**Table 35**

| Total Cholesterol:HDL Ratio, % Change from Baseline | | | | | | |
|---|---|---|---|---|---|---|
| **Study Day** | | **Placebo** | **50 mg** | **100 mg** | **200 mg** | **400 mg** |
| **MD8** | N | 7 | 7 | 8 | 8 | 2 |
| | Mean | 6 | 3 | 2 | -2 | 6 |
| | Median | 2 | -3 | -0.7 | -7 | 6 |
| | Std | 14 | 16 | 8 | 13 | 5 |
| | Min - Max | -12 - 29 | -10 - 35 | -6 - 20 | -14 - 23 | 3 - 10 |
| | P-value | | 0.7104 | 0.6943 | 0.2319 | 0.6667 |
| **MD15** | N | 7 | 8 | 8 | 8 | 2 |
| | Mean | 10 | -1 | -6 | -18 | -9 |
| | Median | 11 | -2 | -6 | -19 | -9 |
| | Std | 11 | 6 | 14 | 14 | 1 |
| | Min - Max | -3 - 28 | -9-9 | -28 - 17 | -37 - 1 | -10 - -9 |
| | P-value | | 0.0541 | 0.0721 | 0.0022 | 0.0556 |
| **MD22** | N | 7 | 8 | 8 | 8 | 2 |
| | Mean | 0.5 | -3 | -8 | -24 | -34 |
| | Median | -1 | -3 | -10 | -25 | -34 |
| | Std | 12 | 7 | 8 | 11 | 13 |
| | Min - Max | -14 - 21 | -14 - 8 | -18 - 5 | -38 - -10 | -43 - -25 |
| | P-value | | 0.7789 | 0.1206 | 0.0012 | 0.0556 |
| **MD25** | N | 7 | 8 | 8 | 8 | 2 |
| | Mean | -3 | -6 | -10 | -24 | -33 |
| | Median | -2 | -4 | -10 | -21 | -33 |
| | Std | 9 | 7 | 9 | 11 | 12 |
| | Min - Max | -16 - 7 | -15 - 7 | -23 - 5 | -39 - -11 | -42 - -25 |
| | P-value | | 0.5358 | 0.1206 | 0.0022 | 0.0556 |
| **PD39** | N | 7 | 8 | 8 | 8 | 2 |
| | Mean | 0.8 | -9 | -10 | -27 | -43 |
| | Median | 5 | -8 | -6 | -26 | -43 |
| | Std | 10 | 14 | 12 | 14 | 17 |
| | Min - Max | -15 - 11 | -34 - 11 | -35 - 3 | -45 - -8 | -55 - -31 |
| | P-value | | 0.1893 | 0.0939 | 0.0037 | 0.0556 |
| **PD55** | N | 7 | 8 | 8 | 8 | 2 |
| | Mean | -1 | -9 | -11 | -29 | -45 |
| | Median | -0.7 | -12 | -7 | -28 | -45 |
| | Std | 6 | 18 | 15 | 12 | 17 |
| | Min - Max | -10-6 | -38 - 11 | -44 - 7 | -42 - -9 | -57 - -33 |
| | P-value | | 0.5358 | 0.152 | 0.0006 | 0.0556 |
| **PD69** | N | 3 | 4 | 5 | 7 | 2 |

(continued)

| Total Cholesterol:HDL Ratio, % Change from Baseline | | | | | | |
|---|---|---|---|---|---|---|
| Study Day | | Placebo | 50 mg | 100 mg | 200 mg | 400 mg |
| | Mean | -2 | -27 | -6 | -26 | -41 |
| | Median | -0.7 | -25 | -0.4 | -26 | -41 |
| | Std | 14.2 | 7.3 | 17.4 | 15.5 | 12 |
| | Min - Max | -17 - 11 | -38 - -22 | -37 - 5 | -53 - -6 | -50 - -33 |
| | P-value | | 0.0571 | 1 | 0.0667 | 0.2 |
| **PD83** | N | 2 | 4 | 7 | 8 | 2 |
| | Mean | -8 | -26 | -5 | -20 | -35 |
| | Median | -8 | -27 | -5 | -19 | -35 |
| | Std | 10 | 4 | 14 | 18 | 12 |
| | Min - Max | -15 - -1 | -29 - -20 | -31 - 11 | -54 - 2 | -43 - -26 |
| | P-value | | 0.1333 | 0.6667 | 0.4 | 0.3333 |
| **PD97** | N | 0 | 2 | 5 | 8 | 1 |
| | Mean | NC | -15 | -9 | -17 | -31 |
| | Median | NC | -15 | -4 | -13 | -31 |
| | Std | NC | 6 | 17 | 18 | 0 |
| | Min - Max | NC | -19 - -11 | -36 - 5 | -56 - 2 | -31 - -31 |
| **PD111** | N | 0 | 1 | 2 | 6 | 2 |
| | Mean | NC | -22 | 15 | -18 | -32 |
| | Median | NC | -22 | 15 | -13 | -32 |
| | Std | NC | NC | 16 | 15 | 10 |
| | Min - Max | NC | -22 - -22 | 4 - 26 | -48 - -6 | -39 - -25 |

**LDL Cholesterol versus HDL-cholesterol.** Lipid profiles were further evaluated by comparing the LDL-cholesterol level to the HDL-cholesterol level. A decrease in this ratio is a positive outcome and indicates a reduction in LDL-cholesterol, an elevation in HDL-cholesterol, or a combination of changes in both parameters. As would be expected with decreases in LDL-cholesterol and no change in HDL-cholesterol following treatment with ISIS 301012, this ratio was lowered. For example, the mean ratio in the 200 mg group was approximately 38% less than baseline levels at PD39.

**Table 36**

| LDL:HDL Ratio, Summary | | | | | | |
|---|---|---|---|---|---|---|
| Study Day | | Placebo | 50 mg | 100 mg | 200 mg | 400 mg |
| **Baseline** | N | 7 | 8 | 8 | 9 | 4 |
| | Mean | 3 | 2 | 3 | 3 | 3 |
| | Median | 3 | 2 | 3 | 3 | 2 |
| | Std | 2 | 1 | 1 | 1 | 1 |
| | Min - Max | 2 - 6 | 1 - 3 | 1 - 4 | 2 - 5 | 2 - 4 |
| | P-value | | 0.4634 | 0.9315 | 0.5913 | 0.6848 |
| **MD8** | N | 7 | 7 | 8 | 8 | 2 |
| | Mean | 3 | 3 | 3 | 2 | 3 |

(continued)

| LDL:HDL Ratio, Summary | | | | | | |
|---|---|---|---|---|---|---|
| **Study Day** | | **Placebo** | **50 mg** | **100 mg** | **200 mg** | **400 mg** |
| | Median | 2 | 2 | 3 | 2 | 3 |
| | Std | 1 | 1 | 1 | 1 | 2 |
| | Min - Max | 2 - 5 | 2 - 4 | 2 - 5 | 2 - 3 | 2 - 4 |
| | P-value | | 0.8048 | 0.9551 | 0.5565 | 0.6667 |
| **MD15** | N | 7 | 8 | 8 | 8 | 2 |
| | Mean | 3 | 2 | 2 | 2 | 2 |
| | Median | 2 | 2 | 2 | 2 | 2 |
| | Std | 2 | 1 | 1 | 0 | 2 |
| | Min - Max | 2 - 6 | 1 - 3 | 2 - 4 | 1 - 3 | 1 - 3 |
| | P-value | | 0.7789 | 0.3357 | 0.0401 | 0.6667 |
| **MD22** | N | 7 | 8 | 8 | 8 | 2 |
| | Mean | 3 | 2 | 2 | 2 | 2 |
| | Median | 2 | 2 | 2 | 2 | 2 |
| | Std | 2 | 1 | 1 | 1 | 1 |
| | Min - Max | 2 - 6 | 1 - 3 | 1 - 4 | 1 - 2 | 1 - 2 |
| | P-value | | 0.8019 | 0.9551 | 0.1284 | 0.5 |
| **MD25** | N | 7 | 8 | 8 | 8 | 2 |
| | Mean | 3 | 2 | 2 | 2 | 2 |
| | Median | 2 | 2 | 2 | 2 | 2 |
| | Std | 2 | 1 | 1 | 0 | 1 |
| | Min - Max | 2 - 6 | 2 - 3 | 1 - 4 | 1 - 2 | 1 - 2 |
| | P-value | | 0.7789 | 0.9551 | 0.152 | 0.5 |
| **PD39** | N | 7 | 8 | 8 | 8 | 2 |
| | Mean | 3 | 2 | 2 | 2 | 1 |
| | Median | 2 | 2 | 2 | 2 | 1 |
| | Std | 2 | 1 | 1 | 0 | 1 |
| | Min - Max | 2 - 6 | 1 - 3 | 1 - 4 | 1 - 2 | 1 - 2 |
| | P-value | | 0.5542 | 0.841 | 0.0135 | 0.2222 |
| **PD55** | N | 7 | 8 | 8 | 8 | 2 |
| | Mean | 3 | 2 | 2 | 2 | 1 |
| | Median | 2 | 2 | 2 | 1 | 1 |
| | Std | 1 | 1 | 1 | 1 | 1 |
| | Min-Max | 2 - 6 | 1 - 3 | 1 - 4 | 1 - 2 | 1 - 2 |
| | P-value | | 0.3201 | 0.5358 | 0.0401 | 0.1111 |
| **PD69** | N | 2 | 2 | 5 | 7 | 2 |
| | Mean | 2 | 2 | 2 | 2 | 1 |
| | Median | 2 | 2 | 2 | 2 | 1 |

(continued)

| LDL:HDL Ratio, Summary | | | | | | |
|---|---|---|---|---|---|---|
| Study Day | | Placebo | 50 mg | 100 mg | 200 mg | 400 mg |
| | Std | 0 | 0 | 1 | 1 | 1 |
| | Min - Max | 2 - 2 | 2 - 2 | 1 - 4 | 1 - 3 | 1 - 2 |
| | P-value | | 1 | 0.5714 | 0.8889 | 1 |
| **PD83** | N | 2 | 3 | 7 | 8 | 2 |
| | Mean | 3 | 2 | 2 | 2 | 2 |
| | Median | 3 | 2 | 3 | 2 | 2 |
| | Std | 3 | 0 | 1 | 1 | 1 |
| | Min - Max | 2 - 5 | 2 - 2 | 1 - 4 | 1 - 3 | 1 - 2 |
| | P-value | | 1 | 0.8889 | 0.8889 | 0.6667 |
| **PD97** | N | 0 | 2 | 5 | 8 | 0 |
| | Mean | NC | 2 | 2 | 2 | NC |
| | Median | NC | 2 | 2 | 2 | NC |
| | Std | NC | 0 | 1 | 1 | NC |
| | Min - Max | NC | 2 - 2 | 1 - 4 | 1 - 3 | NC |
| **PD111** | N | 0 | 1 | 2 | 6 | 0 |
| | Mean | NC | 2 | 2 | 2 | NC |
| | Median | NC | 2 | 2 | 2 | NC |
| | Std | NC | | 1 | 1 | NC |
| | Min - Max | NC | 2 - 2 | 2 - 2 | 1 - 4 | NC |

**Table 37**

| LDL: HDL Ratio, % Change from Baseline | | | | | | |
|---|---|---|---|---|---|---|
| Study Day | | Placebo | 50 mg | 100 mg | 200 mg | 400 mg |
| **MD8** | N | 7 | 7 | 8 | 8 | 2 |
| | Mean | 2 | 3 | -3 | -5 | 1 |
| | Median | 5 | -3 | -3 | -6 | 1 |
| | Std | 17 | 19 | 20 | 19 | 4 |
| | Min - Max | -15 - 28 | -12 - 44 | -25 - 34 | -30 - 28 | -2 - 4 |
| | P-value | 0 | 0.8048 | 0.5358 | 0.637 | 0.8889 |
| **MD15** | N | 7 | 8 | 8 | 8 | 2 |
| | Mean | 7 | 6 | -16 | -24 | -19 |
| | Median | 2 | 0 | -18 | -24 | -19 |
| | Std | 19 | 15 | 21 | 18 | 2 |
| | Min - Max | -16 - 41 | -8 - 33 | -44 - 22 | -50 - 0 | -20 - -18 |
| | P-value | 0 | 0.8665 | 0.0289 | 0.0093 | 0.0556 |
| **MD22** | N | 7 | 8 | 8 | 8 | 2 |

(continued)

| LDL: HDL Ratio, % Change from Baseline | | | | | | |
|---|---|---|---|---|---|---|
| Study Day | | Placebo | 50 mg | 100 mg | 200 mg | 400 mg |
| | Mean | -4 | 3 | -16 | -31 | -42 |
| | Median | 1 | 2 | -19 | -28 | -42 |
| | Std | 19 | 16 | 14 | 16 | 12 |
| | Min - Max | -27 - 27 | -13 - 39 | -36 - 11 | -56 - -12 | -51 - -34 |
| | P-value | 0 | 0.6126 | 0.3969 | 0.0103 | 0.0556 |
| **MD25** | N | 7 | 8 | 8 | 8 | 2 |
| | Mean | -7 | 0.6 | -18 | -31 | -42 |
| | Median | 0 | -4 | -19 | -26 | -42 |
| | Std | 15 | 18 | 13 | 15 | 12 |
| | Min - Max | -32 - 4 | -14 - 42 | -35 - 6 | -56 - -15 | -50 - -34 |
| | P-value | 0 | 0.9551 | 0.2319 | 0.0205 | 0.0556 |
| **PD39** | N | 7 | 8 | 8 | 8 | 2 |
| | Mean | -6 | -9 | -19 | -36 | -47 |
| | Median | -8 | -13 | -23 | -34 | -47 |
| | Std | 16 | 16 | 12 | 20 | 13 |
| | Min - Max | -25 - 22 | -28 - 28 | -33 - 2 | -62 - -8 | -56 - -37 |
| | P-value | 0 | 0.8665 | 0.0721 | 0.0093 | 0.0556 |
| **PD55** | N | 7 | 8 | 8 | 8 | 2 |
| | Mean | -10 | -14 | -21 | -38 | -51 |
| | Median | -14 | -17 | -27 | -35 | -51 |
| | Std | 8 | 12 | 17 | 16 | 14 |
| | Min - Max | -18 - 2 | -32 - 6 | -42 - 6 | -59 - -10 | -61 - -41 |
| | P-value | 0 | 0.3969 | 0.1893 | 0.0037 | 0.0556 |
| **PD69** | N | 2 | 2 | 5 | 7 | 2 |
| | Mean | 6 | -26 | -15 | -28 | -46 |
| | Median | 6 | -26 | -15 | -24 | -46 |
| | Std | 23 | 15 | 13 | 14 | 9 |
| | Min - Max | -10 - 23 | -37 - -16 | -36 - -2 | -50 - -10 | -53 - -40 |
| | P-value | 0 | 0.3333 | 0.381 | 0.1111 | 0.3333 |
| **PD83** | N | 2 | 3 | 7 | 8 | 2 |
| | Mean | -10 | -24 | -18 | -24 | -36 |
| | Median | -10 | -30 | -23 | -17 | -36 |
| | Std | 2 | 12 | 9 | 19 | 11 |
| | Min - Max | -12 - -9 | -30 - -10 | -28 - -4 | -52 - 5 | -44 - -28 |
| | P-value | 0 | 0.4 | 0.5 | 0.2667 | 0.3333 |
| **PD97** | N | 0 | 2 | 5 | 8 | 0 |
| | Mean | NC | -14 | -18 | -21 | NC |

(continued)

| LDL: HDL Ratio, % Change from Baseline | | | | | | |
|---|---|---|---|---|---|---|
| Study Day | | Placebo | 50 mg | 100 mg | 200 mg | 400 mg |
| | Median | NC | -14 | -16 | -13 | NC |
| | Std | NC | 12 | 18 | 18 | NC |
| | Min - Max | NC | -22 - -6 | -37 - 5 | -54 - -3 | NC |
| | N | 0 | 1 | 2 | 6 | 0 |
| | Mean | NC | -24 | 7 | -20 | NC |
| | Median | NC | -24 | 7 | -16 | NC |
| | Std | NC | 0 | 24 | 14 | NC |
| | Min - Max | NC | -24 - -24 | -10 - 24 | -46 - -7 | NC |

**VLDL-Cholesterol.** Reductions in VLDL-cholesterol were observed, as demonstrated in Tables 38 and 39. Maximal reductions were 30% in the 200 mg group and 60% in the 400 mg group (Table 39).

**Table 38**

| Total VLDL Level Summary, mg/dL | | | | | | |
|---|---|---|---|---|---|---|
| **Study Day** | | **Placebo** | **50 mg** | **100 mg** | **200 mg** | **400 mg** |
| **Baseline** | N | 7 | 8 | 8 | 9 | 4 |
| | Mean | 77 | 59 | 78 | 71 | 107 |
| | Median | 47 | 66 | 66 | 59 | 88 |
| | Std | 83 | 31 | 43 | 36 | 48 |
| | Min - Max | 36 - 264 | 16- 95 | 45 - 179 | 34 - 136 | 73 - 177 |
| | P-value | | 0.9807 | 0.1604 | 0.5177 | 0.0727 |
| **MD8** | N | 7 | 7 | 8 | 8 | 2 |
| | Mean | 91 | 83 | 89 | 75 | 118 |
| | Median | 56 | 54 | 81 | 56 | 118 |
| | Std | 75 | 68 | 42 | 48 | 81 |
| | Min - Max | 24 - 242 | 18 - 210 | 34 - 174 | 37 - 177 | 61 - 175 |
| | P-value | | 0.8048 | 0.6126 | 0.9551 | 0.5 |
| **MD15** | N | 7 | 8 | 8 | 8 | 2 |
| | Mean | 108 | 78 | 100 | 65 | 83 |
| **Study Day** | | **Placebo** | **50 mg** | **100 mg** | **200 mg** | **400 mg** |
| | Median | 72 | 63 | 97 | 53 | 83 |
| | Std | 104 | 48 | 58 | 25 | 10 |
| | Min - Max | 25 - 326 | 29-146 | 21 - 190 | 41 - 105 | 76 - 90 |
| | P-value | | 0.8665 | 0.7167 | 0.5913 | 0.8889 |
| **MD22** | N | 7 | 8 | 8 | 8 | 2 |
| | Mean | 86 | 79 | 95 | 66 | 93 |
| | Median | 52 | 74 | 100 | 57 | 93 |
| | Std | 75 | 40 | 43 | 31 | 35 |

(continued)

| Study Day | | Placebo | 50 mg | 100 mg | 200 mg | 400 mg |
|---|---|---|---|---|---|---|
| | Min - Max | 30 - 244 | 28 - 141 | 43 - 166 | 29 - 129 | 68 - 118 |
| | P-value | | 0.7789 | 0.3969 | 0.9551 | 0.5 |
| **MD25** | N | 7 | 8 | 8 | 8 | 2 |
| | Mean | 79 | 65 | 89 | 63 | 59 |
| | Median | 61 | 60 | 86 | 54 | 59 |
| | Std | 78 | 33 | 34 | 39 | 46 |
| | Min - Max | 20 - 246 | 19 - 121 | 60 - 163 | 29 - 153 | 26 - 91 |
| | P-value | | 0.843 | 0.2303 | 0.8887 | 0.8611 |
| **PD39** | N | 7 | 8 | 8 | 8 | 2 |
| | Mean | 83 | 85 | 94 | 62 | 66 |
| | Median | 46 | 77 | 81 | 60 | 66 |
| | Std | 76 | 43 | 49 | 28 | 34 |
| | Min - Max | 28 - 239 | 24 - 157 | 50 - 195 | 23 - 97 | 42 - 90 |
| | P-value | | 0.5358 | 0.281 | 1 | 0.9444 |
| **PD55** | N | 7 | 8 | 8 | 8 | 2 |
| | Mean | 67 | 58 | 105 | 50 | 66 |
| | Median | 41 | 47 | 85 | 51 | 66 |
| | Std | 58 | 35 | 74 | 21 | 50 |
| | Min - Max | 23 - 191 | 22 - 115 | 42 - 279 | 18 - 77 | 30 - 101 |
| | P-value | | 1 | 0.0671 | 0.7789 | 1 |
| **PD69** | N | 3 | 4 | 5 | 7 | 2 |
| | Mean | 150 | 66 | 130 | 70 | 83 |
| | Median | 48 | 59 | 104 | 61 | 83 |
| | Std | 184 | 27 | 73 | 43 | 23 |
| | Min - Max | 40 - 363 | 44 - 102 | 67 - 255 | 21 - 156 | 66 - 99 |
| | P-value | | 1 | 0.5714 | 1 | 0.8 |
| **PD83** | N | 2 | 4 | 7 | 8 | 2 |
| | Mean | 190 | 50 | 90 | 89 | 109 |
| | Median | 190 | 52 | 80 | 82 | 109 |
| | Std | 187 | 25 | 54 | 54 | 55 |
| | Min - Max | 57 - 322 | 21 - 74 | 23 - 176 | 29 - 185 | 70 - 148 |
| | P-value | | 0.5333 | 0.6667 | 0.5333 | 1 |
| **PD97** | N | 0 | 2 | 5 | 8 | 1 |
| | Mean | NC | 82 | 112 | 67 | 43 |
| | Median | NC | 82 | 95 | 52 | 43 |
| | Std | NC | 45 | 87 | 46 | NC |
| | Min - Max | NC | 50 - 114 | 42 - 260 | 34 - 176 | 43 - 43 |
| **PD111** | N | 0 | 1 | 2 | 6 | 2 |

(continued)

| Study Day | | Placebo | 50 mg | 100 mg | 200 mg | 400 mg |
|---|---|---|---|---|---|---|
| | Mean | NC | 94 | 76 | 78 | 82 |
| | Median | NC | 94 | 76 | 48 | 82 |
| | Std | NC | . | 60 | 77 | 48 |
| | Min - Max | NC | 94 - 94 | 33 - 118 | 19 - 225 | 48 - 116 |

**Table 39**

| Total VLDL, % Change from Baseline | | | | | | |
|---|---|---|---|---|---|---|
| **Study Day** | | **Placebo** | **50 mg** | **100 mg** | **200 mg** | **400 mg** |
| **MD8** | N | 7 | 7 | 8 | 8 | 2 |
| | Mean | 37 | 18 | 23 | -2 | -17 |
| | Median | 18 | 0 | 12 | -10 | -17 |
| | Std | 69 | 57 | 52 | 29 | 22 |
| | Min - Max | -58 - 149 | -45 - 121 | -37 - 135 | -41 - 55 | -32 - -1 |
| | P-value | | 0.535 | 0.7789 | 0.2319 | 0.3333 |
| **MD15** | N | 7 | 8 | 8 | 8 | 2 |
| | Mean | 47 | 39 | 45 | -8 | -32 |
| | Median | 51 | 56 | 16 | -15 | -32 |
| | Std | 72 | 48 | 106 | 29 | 24 |
| | Min - Max | -44 - 155 | -36 - 88 | -68 - 273 | -47 - 50 | -49 - -16 |
| | P-value | | 0.9551 | 0.8665 | 0.152 | 0.2222 |
| **MD22** | N | 7 | 8 | 8 | 8 | 2 |
| | Mean | 27 | 53 | 30 | -6 | -29 |
| | Median | 11 | 40 | 15 | 3 | -29 |
| | Std | 59 | 68 | 54 | 32 | 6 |
| | Min - Max | -47 - 134 | -30 - 171 | -35 - 128 | -70 - 35 | -33 - -24 |
| | P-value | | 0.6126 | 1 | 0.3969 | 0.2222 |
| **MD25** | N | 7 | 8 | 8 | 8 | 2 |
| | Mean | 14 | 25 | 23 | -13 | -60 |
| | Median | 14 | 6 | 17 | -7 | -60 |
| | Std | 60 | 57 | 33 | 34 | 16 |
| | Min - Max | -64 - 102 | -37 - 125 | -9 - 84 | -57 - 34 | -71 - -49 |
| | P-value | | 0.7789 | 0.8023 | 0.3969 | 0.2222 |
| **PD39** | N | 7 | 8 | 8 | 8 | 2 |
| | Mean | 16 | 61 | 25 | -12 | -51 |
| | Median | -11 | 26 | 14 | -15 | -51 |
| | Std | 55 | 75 | 41 | 36 | 3 |
| | Min - Max | -22 - 113 | 5 - 225 | -9 - 120 | -56 - 51 | -53 - -49 |

(continued)

| Total VLDL, % Change from Baseline | | | | | | |
|---|---|---|---|---|---|---|
| Study Day | | Placebo | 50 mg | 100 mg | 200 mg | 400 mg |
| | P-value | | 0.0721 | 0.1206 | 0.4634 | 0.0556 |
| **PD55** | N | 7 | 8 | 8 | 8 | 2 |
| | Mean | -1 | 5 | 33 | -30 | -55 |
| | Median | -26 | 20 | 20 | -44 | -55 |
| | Std | 50 | 34 | 45 | 31 | 17 |
| | Min - Max | -36 - 76 | -57 - 38 | -22 - 124 | -51 - 42 | -67 - -43 |
| | P-value | | 0.6126 | 0.1206 | 0.0721 | 0.0556 |
| **PD69** | N | 3 | 4 | 5 | 7 | 2 |
| | Mean | 4 | 35 | 83 | -8 | -35 |
| | Median | -11 | 20 | 49 | -4 | -35 |
| | Std | 30 | 55 | 124 | 39 | 12 |
| | Min - Max | -16 - 38 | -15 - 114 | -27 - 292 | -69 - 39 | -44 - -27 |
| | P-value | | 0.6286 | 0.3929 | 0.8333 | 0.2 |
| **PD83** | N | 2 | 4 | 7 | 8 | 2 |
| | Mean | 24 | -7 | 21 | 13 | 2 |
| | Median | 24 | -16 | 6 | 9 | 2 |
| | Std | 3 | 52 | 63 | 36 | 88 |
| | Min - Max | 22 - 27 | -60 - 64 | -57 - 126 | -31 - 62 | -61 - 64 |
| | P-value | | 0.5333 | 0.8889 | 0.7111 | 1 |
| **PD97** | N | 0 | 2 | 5 | 8 | 1 |
| | Mean | NC | 14 | 35 | -8 | -52 |
| | Median | NC | 14 | 8 | -10 | -52 |
| | Std | NC | 25 | 62 | 37 | |
| | Min - Max | NC | -4- 31 | -18 - 138 | -52 - 54 | -52 - -52 |
| **PD111** | N | 0 | 1 | 2 | 6 | 2 |
| | Mean | NC | 8 | 46 | -3 | -41 |
| | Median | NC | 8 | 46 | -16 | -41 |
| | Std | NC | | 120 | 53 | 9 |
| | Min - Max | NC | 8 - 8 | -39 - 131 | -44 - 97 | -47 - -35 |

**LDL Particle Size.** Evidence exists that high concentrations of small, dense LDL particles can be reliable predictors of cardiovascular risk. To evaluate the effects of ISIS 301012 on LDL particle size in study participants, total LDL particle concentration, small and large LDL subclass particles, and mean LDL particle size were determined by NMR (Liposcience, Raleigh, NC). As apolipoprotein B is a component of LDL-cholesterol, plasma apolipoprotein B levels provided an independent measure of LDL particle concentration. In addition to reductions in serum apolipoprotein B and LDL-cholesterol in the 200 mg dose group, significant reductions in LDL particle number, predominantly small, dense LDL particles were observed. Reductions were seen in the concentration of small LDL particles (Tables 40 and 41), as well as in the fraction of LDL-cholesterol that small LDL-cholesterol (Tables 42 and 43). The duration of response was consistent with the long hepatic tissue half-life of ISIS 301012. These results indicate that LDL-cholesterol reduction following antisense inhibition of apolipoprotein B is in part due to reduction in small, dense, atherogenic LDL particles.

**Table 40**

| Small LDL Particle Concentration, nmol/L | | | | | | |
|---|---|---|---|---|---|---|
| **Study Dav** | | Placebo | 50 mg | 100 mg | 200 mg | 400 mg |
| **Baseline** | N | 7 | 8 | 8 | 9 | 4 |
| | Mean | 913 | 401 | 848 | 821 | 869 |
| | Median | 593 | 454 | 646 | 856 | 758 |
| | Std | 859 | 306 | 583 | 277 | 511 |
| | Min - Max | 409 - 2824 | 0-859 | 243 - 2134 | 446 - 1363 | 389 - 1569 |
| | P-value | | 0.152 | 0.6943 | 0.351 | 0.7879 |
| **MD8** | N | 7 | 7 | 8 | 8 | 2 |
| | Mean | 1157 | 463 | 946 | 780 | 695 |
| | Median | 989 | 484 | 828 | 687 | 695 |
| | Std | 662 | 252 | 565 | 409 | 809 |
| | Min - Max | 656 - 2582 | 8 - 775 | 416 - 2177 | 450 - 1709 | 123 - 1267 |
| | P-value | | 0.0023 | 0.3969 | 0.1206 | 0.8889 |
| **MD15** | N | 7 | 8 | 8 | 8 | 2 |
| | Mean | 1181 | 465 | 825 | 582 | 390 |
| | Median | 876 | 426 | 618 | 594 | 390 |
| | Std | 844 | 391 | 545 | 212 | 465 |
| | Min - Max | 245 - 2699 | 2 - 1107 | 397 - 2089 | 322 - 914 | 61 - 719 |
| | P-value | | 0.0939 | 0.281 | 0.0939 | 0.2222 |
| **MD22** | N | 7 | 8 | 8 | 8 | 2 |
| | Mean | 979 | 449 | 816 | 520 | 265 |
| | Median | 849 | 423 | 726 | 461 | 265 |
| | Std | 693 | 367 | 607 | 246 | 375 |
| | Min - Max | 93 - 2310 | 0 - 935 | 217 - 2134 | 236 - 1023 | 0 - 530 |
| | P-value | | 0.152 | 0.6126 | 0.1206 | 0.1111 |
| **MD25** | N | 7 | 8 | 8 | 8 | 2 |
| | Mean | 993 | 434 | 862 | 514 | 272 |
| | Median | 634 | 416 | 723 | 471 | 272 |
| | Std | 861 | 273 | 597 | 210 | 343 |
| | Min - Max | 327 - 2846 | 15 - 825 | 244 - 2193 | 308 - 900 | 29 - 514 |
| | P-value | | 0.0721 | 0.7789 | 0.1893 | 0.1111 |
| **PD39** | N | 7 | 8 | 8 | 8 | 2 |
| | Mean | 887 | 507 | 688 | 344 | 172 |
| | Median | 646 | 487 | 644 | 344 | 172 |
| | Std | 652 | 387 | 564 | 148 | 155 |
| | Min - Max | 346 - 2226 | 19 - 1112 | 6 - 1948 | 98 - 571 | 62 - 281 |
| | P-value | | 0.281 | 0.5911 | 0.014 | 0.0556 |

(continued)

| Small LDL Particle Concentration, nmol/L | | | | | | |
|---|---|---|---|---|---|---|
| Study Dav | | Placebo | 50 mg | 100 mg | 200 mg | 400 mg |
| **PD55** | N | 7 | 8 | 8 | 8 | 2 |
| | Mean | 876 | 479 | 791 | 289 | 280 |
| | Median | 646 | 474 | 580 | 281 | 280 |
| | Std | 668 | 361 | 540 | 231 | 339 |
| | Min - Max | 124 - 2205 | 7 - 1032 | 458 - 2061 | 6 - 657 | 40 - 519 |
| | P-value | | 0.2319 | 0.6943 | 0.0401 | 0.2222 |
| **PD69** | N | 3 | 4 | 5 | 7 | 2 |
| | Mean | 1096 | 345 | 881 | 589 | 232 |
| | Median | 590 | 393 | 869 | 551 | 232 |
| | Std | 897 | 234 | 576 | 281 | 242 |
| | Min - Max | 566 - 2131 | 19 - 576 | 286 - 1809 | 352 - 1189 | 61 - 403 |
| | P-value | | 0.1143 | 0.7857 | 0.1833 | 0.2 |
| **PD83** | N | 2 | 4 | 7 | 8 | 2 |
| | Mean | 1478 | 522 | 919 | 509 | 196 |
| | Median | 1478 | 636 | 912 | 422 | 196 |
| | Std | 1600 | 358 | 621 | 292 | 277 |
| | Min - Max | 346 - 2609 | 0 - 816 | 291 - 2185 | 100 - 1060 | 0 - 392 |
| | P-value | | 0.8 | 0.8889 | 0.6889 | 0.6667 |
| **PD97** | N | 0 | 2 | 5 | 8 | 1 |
| | Mean | NC | 1051 | 879 | 592 | 0 |
| | Median | NC | 1051 | 685 | 455 | 0 |
| | Std | NC | 292 | 671 | 395 | NC |
| | Min - Max | NC | 844 - 1257 | 301 - 1891 | 232 - 1376 | 0 - 0 |
| **PD111** | N | 0 | 1 | 2 | 6 | 2 |
| | Mean | NC | 320 | 448 | 694 | 386 |
| | Median | NC | 320 | 448 | 541 | 386 |
| | Std | NC | NC | 54 | 470 | 506 |
| | Min - Max | NC | 320 - 320 | 409 - 486 | 382 - 1642 | 28 - 743 |

**Table 41**

| Small LDL Particles, Mean % Change | | | | | | |
|---|---|---|---|---|---|---|
| **Study Day** | | **Placebo** | **50 mg** | **100 mg** | **200 mg** | **400 mg** |
| **MD8** | N | 7 | 7 | 8 | 8 | 2 |
| | Mean | 50 | 11 | 20 | -9 | -50 |
| | Median | 60 | -11 | 17 | -21 | -50 |
| | Std | 42 | 49 | 29 | 39 | 43 |

(continued)

| Small LDL Particles, Mean % Change | | | | | | |
|---|---|---|---|---|---|---|
| Study Day | | Placebo | 50 mg | 100 mg | 200 mg | 400 mg |
| | Min - Max | -9 - 115 | -44 - 90 | -22 - 71 | -48 - 80 | -80 - -19 |
| | P-value | 0 | 0.1282 | 0.1893 | 0.0205 | 0.0556 |
| **MD15** | N | 7 | 7 | 8 | 8 | 2 |
| | Mean | 48 | 28 | 8 | -30 | -72 |
| | Median | 25 | 29 | 0.6 | -22 | -72 |
| | Std | 78 | 41 | 43 | 26 | 26 |
| | Min - Max | -40 - 161 | -27 - 89 | -34 - 100 | -63 - 2 | -90 - -54 |
| | P-value | 0 | 0.9015 | 0.5358 | 0.0541 | 0.0556 |
| **MD22** | N | 7 | 7 | 8 | 8 | 2 |
| | Mean | 18 | 5 | -6 | -39 | -83 |
| | Median | 25 | 9 | -3 | -45 | -83 |
| | Std | 55 | 35 | 18 | 24 | 24 |
| | Min - Max | -77 - 79 | -44 - 50 | -45 - 13 | -74 - 8 | -100 - -66 |
| | P-value | 0 | 0.62 | 0.3357 | 0.0401 | 0.1111 |
| **MD25** | N | 7 | 7 | 8 | 8 | 2 |
| | Mean | 11 | 18 | 6 | -40 | -81 |
| | Median | 13 | 6 | 2 | -39 | -81 |
| | Std | 24 | 41 | 33 | 20 | 20 |
| | Min - Max | -20 - 49 | -24 - 77 | -36 - 67 | -66 - -5 | -95 - -67 |
| | P-value | 0 | 1 | 0.6126 | 0.0012 | 0.0556 |
| **PD39** | N | 7 | 7 | 8 | 8 | 2 |
| | Mean | 9 | 40 | -25 | -58 | -86 |
| | Median | -15 | 12 | -23 | -60 | -86 |
| | Std | 48 | 45 | 38 | 22 | 6 |
| | Min - Max | -27 - 99 | -5 - 111 | -98 - 17 | -89 - -15 | -90 - -82 |
| | P-value | 0 | 0.1282 | 0.3357 | 0.0037 | 0.0556 |
| **PD55** | N | 7 | 7 | 8 | 8 | 2 |
| | Mean | 2 | 16 | 3 | -63 | -80 |
| | Median | 17 | 19 | -13 | -65 | -80 |
| | Std | 42 | 24 | 42 | 32 | 19 |
| | Min - Max | -70 - 43 | -22 - 55 | -25 - 103 | -99 - -2 | -94 - -67 |
| | P-value | 0 | 0.8048 | 0.7789 | 0.0093 | 0.1111 |
| **PD69** | N | 3 | 4 | 5 | 7 | 2 |
| | Mean | 13 | 40 | -5 | -27 | -82 |
| | Median | 26 | 41 | -8 | -41 | -82 |
| | Std | 33 | 66 | 16 | 35 | 11 |
| | Min - Max | -25 - 38 | -35 - 111 | -23 - 18 | -62 - 25 | -90 - -74 |

(continued)

| Small LDL Particles, Mean % Change | | | | | | |
|---|---|---|---|---|---|---|
| **Study Day** | | **Placebo** | **50 mg** | **100 mg** | **200 mg** | **400 mg** |
| | P-value | 0 | 0.8571 | 0.5714 | 0.1167 | 0.2 |
| **PD83** | N | 2 | 4 | 7 | 8 | 2 |
| | Mean | -17 | 7 | 9 | -39 | -88 |
| | Median | -17 | 30 | 2 | -49 | -88 |
| | Std | 13 | 74 | 26 | 33 | 18 |
| | Min - Max | -26 - -8 | -100 - 67 | -22 - 59 | -89 - 12 | -100 - -75 |
| | P-value | 0 | 0.5333 | 0.1111 | 0.5333 | 0.3333 |
| **PD97** | N | 0 | 2 | 5 | 8 | 1 |
| | Mean | NC | 131 | -0.2 | -33 | -100 |
| | Median | NC | 131 | 2 | -46 | -100 |
| | Std | NC | 4 | 28 | 40 | 0 |
| | Min - Max | NC | 128 - 134 | -41 - 34 | -69 - 45 | -100 - -100 |
| **PD111** | N | 0 | 1 | 2 | 6 | 2 |
| | Mean | NC | -40 | 32 | -15 | -74 |
| | Median | NC | -40 | 32 | -38 | -74 |
| | Std | NC | 0 | 51 | 48 | 30 |
| | Min - Max | NC | -40 - -40 | -4 - 68 | -48 - 73 | -96 - -53 |

**Table 42**

| Small LDL Subclass of LDL-Cholesterol Summary, mg/dL | | | | | | |
|---|---|---|---|---|---|---|
| **Study Day** | | **Placebo** | **50 mg** | **100 mg** | **200 mg** | **400 mg** |
| **Baseline** | N | 7 | 8 | 8 | 9 | 4 |
| | Mean | 54 | 24 | 51 | 50 | 52 |
| | Median | 35 | 28 | 39 | 51 | 45 |
| | Std | 51 | 18 | 35 | 17 | 31 |
| | Min - Max | 23 - 168 | 0- 52 | 15 - 127 | 27 - 83 | 24 - 94 |
| | P-value | | 0.2319 | 0.7167 | 0.351 | 0.6485 |
| **MD8** | N | 7 | 7 | 8 | 8 | 2 |
| | Mean | 70 | 28 | 57 | 47 | 42 |
| | Median | 60 | 30 | 51 | 41 | 42 |
| | Std | 40 | 15 | 34 | 25 | 50 |
| | Min - Max | 39 - 157 | 1 - 47 | 25 - 132 | 29 - 105 | 7 - 77 |
| | P-value | | 0.0023 | 0.3969 | 0.1206 | 0.8889 |
| **MD15** | N | 7 | 8 | 8 | 8 | 2 |
| | Mean | 71 | 28 | 50 | 35 | 23 |
| | Median | 53 | 26 | 37 | 36 | 23 |

(continued)

| Small LDL Subclass of LDL-Cholesterol Summary, mg/dL | | | | | | |
|---|---|---|---|---|---|---|
| Study Day | | Placebo | 50 mg | 100 mg | 200 mg | 400 mg |
| | Std | 52 | 23 | 32 | 13 | 28 |
| | Min - Max | 14 - 166 | 0 - 67 | 24 - 124 | 20 - 57 | 3 - 44 |
| | P-value | | 0.0939 | 0.281 | 0.1206 | 0.2222 |
| **MD22** | N | 7 | 8 | 8 | 8 | 2 |
| | Mean | 59 | 27 | 49 | 31 | 16 |
| | Median | 51 | 26 | 44 | 28 | 16 |
| | Std | 41 | 22 | 35 | 15 | 23 |
| | Min - Max | 6 - 136 | 0 - 57 | 12 - 122 | 14 - 62 | 0- 32 |
| | P-value | | 0.152 | 0.6126 | 0.1206 | 0.1111 |
| **MD25** | N | 7 | 8 | 8 | 8 | 2 |
| | Mean | 59 | 26 | 52 | 31 | 17 |
| | Median | 38 | 24 | 44 | 30 | 17 |
| | Std | 52 | 17 | 35 | 13 | 21 |
| | Min - Max | 19 - 170 | 2 - 49 | 15 - 130 | 19 - 55 | 2 - 32 |
| | P-value | 0 | 0.0721 | 0.9551 | 0.2319 | 0.3333 |
| **PD39** | N | 7 | 8 | 8 | 8 | 2 |
| | Mean | 54 | 31 | 42 | 21 | 10 |
| | Median | 38 | 30 | 38 | 21 | 10 |
| | Std | 42 | 24 | 34 | 9 | 10 |
| | Min - Max | 20 - 141 | 2 - 70 | 0 - 119 | 6 - 36 | 3 - 17 |
| | P-value | | 0.3357 | 0.7789 | 0.0154 | 0.0556 |
| **PD55** | N | 7 | 8 | 8 | 8 | 2 |
| | Mean | 53 | 29 | 49 | 18 | 17 |
| | Median | 39 | 28 | 35 | 17 | 17 |
| | Std | 42 | 22 | 35 | 14 | 20 |
| | Min - Max | 7 - 136 | 1 - 62 | 28 - 132 | 1 - 41 | 2 - 31 |
| | P-value | | 0.2319 | 0.6943 | 0.0541 | 0.2222 |
| **PD69** | N | 3 | 4 | 5 | 7 | 2 |
| | Mean | 67 | 21 | 54 | 35 | 15 |
| | Median | 35 | 23 | 54 | 33 | 15 |
| | Std | 58 | 14 | 34 | 16 | 14 |
| | Min - Max | 33 - 134 | 2 - 35 | 17 - 108 | 21 - 67 | 6 - 25 |
| | P-value | | 0.2286 | 1 | 0.4167 | 0.2 |
| **PD83** | N | 2 | 4 | 7 | 8 | 2 |
| | Mean | 92 | 32 | 56 | 31 | 12 |
| | Median | 92 | 39 | 56 | 25 | 12 |
| | Std | 102 | 22 | 37 | 17 | 17 |

(continued)

| Small LDL Subclass of LDL-Cholesterol Summary, mg/dL | | | | | | |
|---|---|---|---|---|---|---|
| Study Day | | Placebo | 50 mg | 100 mg | 200 mg | 400 mg |
| | Min - Max | 20 - 164 | 0 - 51 | 18 - 131 | 6 - 63 | 0 - 24 |
| | P-value | | 0.8 | 0.8889 | 0.7111 | 0.6667 |
| **PD97** | N | 0 | 2 | 5 | 8 | 1 |
| | Mean | NC | 63 | 55 | 36 | 0 |
| | Median | NC | 63 | 43 | 27 | 0 |
| | Std | NC | 17 | 43 | 23 | NC |
| | Min - Max | NC | 51 - 75 | 19 - 121 | 14 - 81 | 0 - 0 |
| **PD111** | N | 0 | 1 | 2 | 6 | 2 |
| | Mean | NC | 19 | 28 | 42 | 24 |
| | Median | NC | 19 | 28 | 33 | 24 |
| | Std | NC | . | 3 | 28 | 31 |
| | Min - Max | NC | 19 - 19 | 26 - 30 | 24 - 99 | 2 - 45 |

Table 43

| Small LDL Subclass of LDL-Cholesterol, % of Baseline | | | | | | |
|---|---|---|---|---|---|---|
| Study Day | | Placebo | 50 mg | 100 mg | 200 mg | 400 mg |
| **MD8** | N | 7 | 7 | 8 | 8 | 2 |
| | Mean | 54 | 13 | 21 | -8 | -50 |
| | Median | 70 | 0 | 16 | -21 | -50 |
| | Std | 43 | 51 | 27 | 41 | 45 |
| | Min - Max | -7 - 117 | -48 - 92 | -16 - 70 | -50 - 85 | -82 - -18 |
| | P-value | | 0.1282 | 0.152 | 0.0205 | 0.0556 |
| **MD15** | N | 7 | 7 | 8 | 8 | 2 |
| | Mean | 49 | 30 | 8 | -30 | -72 |
| | Median | 27 | 30 | -0.7 | -22 | -72 |
| | Std | 78 | 43 | 42 | 27 | 26 |
| | Min - Max | -40 - 160 | -29 - 90 | -31 - 97 | -64 - 3 | -91 - -54 |
| | P-value | | 0.9015 | 0.4634 | 0.0541 | 0.0556 |
| **MD22** | N | 7 | 7 | 8 | 8 | 2 |
| | Mean | 19 | 5 | -7 | -39 | -83 |
| | Median | 28 | 10 | -4 | -44 | -83 |
| | Std | 56 | 33 | 18 | 25 | 24 |
| | Min - Max | -75 - 80 | -40 - 51 | -41 - 13 | -74 - 10 | -100 - -66 |
| | P-value | | 0.62 | 0.351 | 0.0401 | 0.1111 |
| **MD25** | N | 7 | 7 | 8 | 8 | 2 |
| | Mean | 11 | 19 | 6 | -39 | -81 |

(continued)

| Small LDL Subclass of LDL-Cholesterol, % of Baseline | | | | | | |
|---|---|---|---|---|---|---|
| **Study Day** | | **Placebo** | **50 mg** | **100 mg** | **200 mg** | **400 mg** |
| | Median | 11 | 0.9 | 0 | -38 | -81 |
| | Std | 22 | 43 | 34 | 20 | 21 |
| | Min - Max | -14 - 48 | -24 - 80 | -36 - 68 | -66 - -3 | -96 - -66 |
| | P-value | | 1 | 0.7789 | 0.0012 | 0.0556 |
| **PD39** | N | 7 | 7 | 8 | 8 | 2 |
| | Mean | 10 | 42 | -22 | -57 | -86 |
| | Median | -12 | 13 | -19 | -61 | -86 |
| | Std | 47 | 47 | 37 | 23 | 7 |
| | Min - Max | -28 - 100 | -5 - 110 | -97 - 17 | -89 - -12 | -91 - -82 |
| | P-value | | 0.1282 | 0.2319 | 0.0037 | 0.0556 |
| **PD55** | N | 7 | 7 | 8 | 8 | 2 |
| | Mean | 2 | 15 | 3 | -62 | -80 |
| | Median | 17 | 19 | -12 | -64 | -80 |
| | Std | 43 | 24 | 39 | 33 | 19 |
| | Min - Max | -70 - 45 | -20 - 56 | -26 - 93 | -99 - 1 | -94 - -67 |
| | P-value | | 0.8048 | 0.9551 | 0.0093 | 0.1111 |
| **PD69** | N | 3 | 4 | 5 | 7 | 2 |
| | Mean | 18 | 40 | -4 | -27 | -79 |
| | Median | 28 | 43 | -2 | -40 | -79 |
| | Std | 35 | 67 | 16 | 35 | 8 |
| | Min - Max | -20 - 47 | -37 - 110 | -23 - 16 | -64 - 18 | -85 - -74 |
| | P-value | | 0.8571 | 0.3929 | 0.1167 | 0.2 |
| **PD83** | N | 2 | 4 | 7 | 8 | 2 |
| | Mean | -15 | 6 | 11 | -39 | -87 |
| | Median | -15 | 30 | 4 | -48 | -87 |
| | Std | 18 | 73 | 26 | 33 | 18 |
| | Min - Max | -28 - -3 | -100 - 65 | -21 - 62 | -88 - 10 | -100 - -74 |
| | P-value | | 0.5333 | 0.2222 | 0.4 | 0.3333 |
| **PD97** | N | 0 | 2 | 5 | 8 | 1 |
| | Mean | NC | 124 | 3 | -33 | -100 |
| | Median | NC | 124 | 2 | -46 | -100 |
| | Std | NC | 7 | 28 | 39 | NC |
| | Min - Max | | 120 - 129 | -38 - 36 | -68 - 42 | -100 - -100 |
| **PD111** | N | 0 | 1 | 2 | 6 | 2 |
| | Mean | NC | -42 | 35 | -14 | -74 |
| | Median | NC | -42 | 35 | -37 | -74 |
| | Std | NC | 0 | 53 | 48 | 31 |

(continued)

| Small LDL Subclass of LDL-Cholesterol, % of Baseline | | | | | | |
|---|---|---|---|---|---|---|
| **Study Day** | | **Placebo** | **50 mg** | **100 mg** | **200 mg** | **400 mg** |
| | Min - Max | | -42 - -42 | -3 - 72 | -47 - 75 | -95 - -52 |

Pharmacodynamic Summary

The data presented herein demonstrate that treatment with ISIS 301012 resulted in a dose-dependent reduction in serum apolipoprotein B, LDL-cholesterol and total-cholesterol. The decreases in serum LDL-cholesterol, as shown in Tables 22 and 23, were observed throughout the treatment period, and at the end of the treatment period ranged from approximately 20% in the 100 mg group to greater than 30% in the 200 mg and 400 mg groups. The reductions in serum apolipoprotein B, as shown in Tables 24 and 25, were also observed throughout the treatment period and ranged from approximately 15% in the 50 mg group to greater than 40% in the 200 mg and 400 mg groups at the end of the treatment period. Total serum cholesterol levels were similarly reduced at the end of treatment, approximately 15% in the 100 mg group and greater than approximately 25% in the 200 mg and 400 mg groups. Individuals with higher baseline serum LDL-cholesterol and apolipoprotein B levels experienced greater percent reductions in these lipids, as compared to individuals with lower baseline serum LDL-cholesterol and apolipoprotein B levels.

*Duration of Effect: 2 Weeks*

The reductions in serum LDL-cholesterol, serum total cholesterol, and serum apolipoprotein B levels were sustained at PD39, two weeks following MD25. Serum LDL-cholesterol was reduced approximately 20%, 35% and 45% in the 100 mg, 200 mg and 400 mg groups, respectively. Serum apolipoprotein B was lowered by approximately 20%, 35% and 50% in the 100 mg, 200 mg and 400 mg groups, respectively. Total serum cholesterol was similarly decreased, by approximately 15%, 25% and 30% in the 100 mg, 200 mg and 400 mg groups, respectively.

*Duration of Effect: 30 Days*

The duration of effect was prolonged, with significant reductions in several lipid parameters observed 30 days following MD25. A summary of these reductions is illustrated in Table 44, where mean percent change from baseline is shown for several lipid parameters. Data are expressed as mean percent change relative to baseline. "Std" indicates standard deviation. No formal statistics were done for 400 mg cohort due to small sample size (n=2). "N" indicates the number of subject evaluated within each cohort.

**Table 44**

| Mean % Change at PD55 | | | | | | |
|---|---|---|---|---|---|---|
| **Lipid Parameter** | | **Placebo** | **50 mg** | **100 mg** | **200 mg** | **400 mg** |
| | N | 7 | 8 | 8 | 8 | 2 |
| **LDL-Cholesterol** | Mean | -1 | -6 | -20 | -34 | -41 |
| | Std | 15 | 8 | 17 | 19 | 3 |
| | P-value | | 0.8665 | 0.0939 | 0.0022 | 0.0556 |
| **Apolipoprotein B** | Mean | -11 | -14 | -28 | -42 | -47 |
| | Std | 29 | 13 | 15 | 14 | 9 |
| | P-value | | 0.1893 | 0.0541 | 0.0205 | 0.2222 |
| **Total Cholesterol** | Mean | 10 | 2 | -15 | -23 | -31 |
| | Std | 14 | 7 | 8 | 14 | 6 |
| | P-value | | 0.4634 | 0.0003 | 0.0006 | 0.0556 |
| **HDL-Cholesterol** | Mean | 10 | 11 | 1 | 7 | 25 |
| | Std | 18 | 17 | 9 | 9 | 30 |

(continued)

| Mean % Change at PD55 | | | | | | |
|---|---|---|---|---|---|---|
| Lipid Parameter | | Placebo | 50 mg | 100 mg | 200 mg | 400 mg |
| | P-value | | 0.8421 | 0.4448 | 0.9294 | 0.3056 |
| Triglycerides | Mean | 1 | -1 | 37 | -27 | -32 |
| | Std | 38 | 30 | 60 | 20 | 2 |
| | P-value | | 0.7789 | 0.2319 | 0.0541 | 0.0556 |

*Duration of Effect: Three Months*

During the post-treatment evaluation period, serum apolipoprotein B and LDL-cholesterol were 20-25% below baseline three months after MD25 in the 200 mg and 400 mg dose cohorts. These results are consistent with the long tissue half-life of ISIS 301012, which was estimated to be approximately 31 days in the 200 mg dose cohort. Serum triglycerides, VLDL-cholesterol and lipoprotein(a) also appear reduced. Serum HDL-cholesterol was not affected.

Pharmacokinetic Analysis

Non-compartmental pharmacokinetic analysis of ISIS 301012 was carried out on each individual subject data set. Plasma concentrations of ISIS 301012 were measured by hybridization-based ELISA at PPD Development (Richmond, VA). The maximum observed drug concentration ($C_{max}$) and the time taken to reach $C_{max}$ ($T_{max}$) were obtained directly from the concentration-time data. The plasma disposition half-life ($t_{1/2\lambda z}$) associated with the apparent terminal elimination phase was calculated from the equation, $t_{1/2\lambda z} = 0.693/\lambda_z$, where $\lambda_z$ is the terminal rate constant associated with the apparent terminal elimination phase. The terminal elimination rate constant is calculated using log-linear regression of the last 3 or more concentration time points. The apparent distribution half-life was calculated from a similar equation, using the apparent distribution rate constant in place of the terminal elimination rate constant. The apparent distribution rate constant is calculated using log-linear regression of distribution phase time points. Following single dosing, an area under the plasma concentration-time curve from zero time (pre-dose) to infinite time ($AUC\infty$) was calculated using the linear trapezoidal rule and extrapolation to infinity by dividing the final measurable concentration ($C_{last}$) by $\lambda_z$. Following multiple dosing, the area under the plasma concentration-time curve during the time of each dosing interval (tau,$\tau$) at steady-state ($AUC_\tau$) was calculated using the linear trapezoidal rule. Further, partial areas under the plasma concentration-time curve from zero time (pre-dose) to selected times (t) after the start of the intravenous infusion or subcutaneous administration ($AUC_\tau$) was calculated using the linear trapezoidal rule. Plasma clearance (CL) was calculated from CL = Actual Dose/$AUC_{iv}$. Steady-state volume of distribution [$V_{ss}$ = ($AUMC_{iv}$ *Actual Dose)/($AUC_{iv}$)$^2$; where $AUMC_{iv}$ is the area under the first moment curve following intravenous infusion] was also calculated. Mean absorption time following subcutaneous injection was calculated by subtracting the plasma $AUMC_{sc}$ (first moment curve for subcutaneous injection) from $AUMC_{iv}$ (first moment curve for intravenous infusion) estimated for each subject, and refers to the extent to which ISIS 301012 was distributed throughout the body at steady state concentrations. In addition, ratios of subcutaneous over intravenous plasma AUC were used to estimate subcutaneous plasma bioavailability (F) for each subject.

The amount of ISIS 301012 and total oligonucleotide excreted in the urine was determined from the following expression:

$$Ae_t = C_{urine} \text{ X } V_{urine}$$

where $Ae_t$ is the amount excreted up to some fixed time t (i.e., 24 hours), $C_{urine}$ is the urine concentration of the analyte, and $V_{urine}$ is the total urine volume. The percentage of the administered dose excreted in urine (intact or as total oligonucleotide) was then calculated from the following expression:

$$\text{\% Dose Excreted} = (Ae_t/\text{Administered dose}) \text{ X } 100\%$$

Pharmacokinetic Summary

The plasma pharmacokinetic profile of ISIS 301012 was determined from blood sampling following the first 2-hr intravenous infusion (MD1), and is summarized in Table 45. Data are presented as mean + standard deviation for each dose group. $C_{max}$ = maximal plasma concentration; $T_{max}$ = time to reach $C_{max}$; $AUC_{0\text{-}48hr}$ = area under the plasma concentration-time curve from time 0 to 48 hours after the start of dose administration; CL = plasma clearance; $V_{ss}$ = steady-state volume of distribution. Bioavailability following an intravenous administration is assumed to be 100%.

**Table 45**

| ISIS 301012 Dosed Intravenously: Plasma Pharmacokinetics | | | | |
|---|---|---|---|---|
| **Dose Group** | **50 mg** | **100 mg** | **200 mg** | **400 mg** |
| **Dose, mg/kg for 70 kg** | 0.7 ±0.1 | 1 ±0.1 | 2.7 ±0.5 | 5.9 ±1.2 |
| **N** | 8 | 8 | 8 | 3 |
| **$C_{max}$ (ug/ml)** | 5 ±1 | 9 ± 1 | 22 ± 4 | 38 ± 5 |
| **$T_{max}$ (hr)** | 2 ± 0.1 | 2 ± 0.1 | 2 ± 0.2 | 2 ± 0.2 |
| **$AUC_{0\text{-}48hr}$ (ug*hr/mL)** | 11 ± 3 | 24 ± 3 | 68 ± 14 | 148 ± 14 |
| **CL (L/hr)** | 5 ± 1 | 4 ± 0.6 | 3 ± 0.7 | 3 ± 0.3 |
| **$V_{ss}$** (L) | 6 ± 3 | 7 ± 1 | 7 ± 1 | 8 ± 0.8 |
| **Apparent Distribution $t_{1/2}$ (hr)** | 0.7 ±0.1 | 0.8 ± 0.1 | 1 ± 0.2 | 1.7 ± 0.4 |

Dose-dependent maximum plasma concentrations ($C_{max}$) following 2-hour intravenous infusions were seen at the end of infusion followed by a biphasic decline. An initial, relatively fast distribution phase (mean apparent distribution half-life ranged 0.7 to 1.7 hours) dominated the plasma clearance and was followed by a slower apparent elimination phase.

Plasma pharmacokinetics determined from blood sampling following the final subcutaneous injection (MD22) are summarized in Table 46. Data are presented as mean + standard deviation. $C_{max}$ = maximal plasma concentration; $T_{max}$ = time to reach $C_{max}$; $AUC_{0\text{-}48hr}$ = area under the plasma concentration-time curve from time 0 to 48 hours after the start of dose administration; $AUC_{0\text{-}\infty \text{ at ss}}$ = area under the plasma concentration-time curve from time 0 to infinity at steady-state; %BAV = plasma bioavailability (%) following subcutaneous administration.

**Table 46**

| ISIS 301012 Dosed Subcutaneously: Plasma Pharmacokinetics | | | | |
|---|---|---|---|---|
| **Dose Group** | **50 mg** | **100 mg** | **200 mg** | **400 mg** |
| **Dose, mg/kg for 70 kg** | 0.7 ±0.1 | 1.3 ±0.1 | 2.7 ±0.5 | 5.9 ±1.2 |
| **N** | 7 | 8 | 8 | 2 |
| **$C_{max}$ (μg/ml)** | 1 ± 0.3 | 2 ± 1 | 3 ± 1 | 7 |
| **$T_{max}$ (hr)** | 4 ± 2 | 4 ± 2 | 3 ± 2 | 7 |
| **$AUC_{0\text{-}48hr}$ (ug*hr/mL)** | 8 ± 2 | 18 ± 4 | 35 ± 7 | 109 |
| **$AUC_{0\text{-}\infty \text{ at ss}}$ (ug*hr/mL)** | 19 ± 9 | 28 ± 5 | 63 ± 13 | 160 |
| **Apparent Distribution $t_{1/2}$ (hr)** | 4 ± 1 | 5 ± 3 | 7 ± 3 | 8 |
| **Elimination $t_{1/2}$ (hr)** | 23 ± 1 | 27 ± 12 | 31 ± 11 | 47 |
| **%BAV** | 69 ± 9 | 76 ± 18 | 54 ± 11 | 78 |

The mean time to maximum plasma concentrations ($T_{max}$) following the final subcutaneous injection (MD22) of ISIS 301012 was approximately 4 hours following administration of the 50 and 100 mg doses, and approximately 3 hours following administration of the 200 mg dose. Plasma concentrations decreased more slowly from the maximum plasma concentration ($C_{max}$) following subcutaneous injection, when compared to intravenous infusion, indicating continued absorption of ISIS 301012 after achievement of $C_{max}$. Maximum plasma concentrations ($C_{max}$) ranged from approximately 1 to 3 ug/mL (50 mg to 200 mg) and were dose-dependent over the studied subcutaneous dose range, but were much lower in comparison to equivalent intravenous infusion doses. $C_{max}$, $T_{max}$, plasma AUC following the first subcutaneous dose of ISIS 301012 were similar to those shown in Table 46, following the final subcutaneous dose. Plasma drug concentration was decreased by at least 10 fold by 24 hours. The terminal elimination phase observed in plasma provided a measure of tissue elimination rate, thus the elimination half-life represents the time at which approximately 50% of ISIS 301012 was cleared from tissues. Characterization of the terminal elimination phase yielded an elimination half-life of approximately 23 (±1) to 31 (±11) days (see Table 46). This result is consistent with the slow elimination of ISIS 301012 observed from monkey tissues, and thus appears to reflect an equilibrium of oligonucleotide between plasma and tissue. Absolute plasma bioavailability (BAV) of ISIS 301012 following subcutaneous administration ranged from 54% to 78%, in comparison to intravenous infusion, and was independent of dose. Plasma BAV may underestimate the ultimate complete absorption of ISIS 301012, as nonhuman primate studies have shown that the entire dose is ultimately distributed to tissues such that there is no difference between intravenous and subcutaneous administration with regard to end organ drug concentrations.

Mean urinary excretion of total oligonucleotide was less than 8% within the first 24 hours. Excretion of chain shortened metabolites was evident. Urine excretion data indicate that ISIS 301012 is primarily distributed to tissues. Ultimate elimination is a combination of nuclease metabolism and excretion in urine.

*Exposure-Response Relationships*

The correlation between ISIS 301012 plasma concentrations, serum apolipoprotein B protein and LDL-cholesterol is shown in Table 47. Serum apolipoprotein B, LDL-cholesterol and total cholesterol are presented as percentage of baseline. The numbers in parentheses following apolipoprotein B percent baseline indicates the number of samples used to calculate the mean; all other means were calculated using the number of samples in the "N" column.

**Table 47**

| Exposure-Response Relationship: ISIS 301012 Plasma Level, apolipoprotein B protein and LDL-cholesterol in 200 mg treatment group | | | | | |
|---|---|---|---|---|---|
| **Study Day** | **N** | **ISIS 301012 ng/mL** | **Apolipoprotein B % Baseline** | **LDL-Cholesterol % Baseline** | **Total Cholesterol % Baseline** |
| **MD1** | 9 | 0.5 | 96 | 93 | 99 |
| **MD8** | 8 | 18 | 92 | 86 | 90 |
| **MD15** | 8 | 16 | 68 | 71 | 77 |
| **MD22** | 8 | 21 | 66 | 68 | 76 |
| **MD25** | 8 | 30 | 50 | 69 | 76 |
| **PD39** | 8 | 15 | 61 | 65 | 74 |
| **PD55** | 8 | 8.7 | 58 | 66 | 76 |
| **PD69** | 6 | 6.1 | 64 | 73 | 76 |
| **PD83** | 7 | 5.2 | 70 | 79 | 83 |
| **PD97** | 5 | 4.7 | 76 (7) | 82 | 87 |
| **PD111** | 4 | 5.1 | 77 (6) | 80 | 81 |

As shown in Table 47, total tissue exposure, represented by ISIS 301012 plasma concentrations (measured at least 72 hr after dosing during and following the multiple dose treatment period) was highly correlated with serum apolipoprotein B protein levels and LDL-cholesterol levels, both of which responded in similar dose-response manners. Increases in plasma AUC were slightly greater than dose-proprotional. Significant reduction in apolipoprotein B protein ($p<0.02$) from baseline for the 200 mg treatment group was achieved from Day 15 (MD 15) to Day 97 (PD 72, or 75 days after last dose), consistent with the slow elimination of ISIS 301012 in plasma (terminal elimination $t_{1/2}$ of approximately 31 days).

A comparison of serum reductions versus plasma trough AUC for 26 ISIS 301012-treated subjects at the end of the multiple dosing period (MD25) revealed a direct correlation between trough AUC and the observed reductions in serum apolipoprotein B protein levels, LDL-cholesterol and total cholesterol (r ≥0.67, p ≤0.0002) (Figure 1). Such a correlation is consistent with the fact that trough AUC is a representation of liver concentrations, as result of the equilibrium reached between drug concentration in plasma and in liver. A correlation was also observed between plasma trough concentrations and serum reductions, when plasma trough concentrations ($C_{trough}$; mean trough concentration determined from plasma levels just prior to dosing on days MD15 and MD22) were compared to reductions in serum apolipoprotein B, serum LDL-cholesterol, and serum total cholesterol. $C_{trough}$ and trough AUC correlate with reductions in lipid parametes, demonstrating that as exposure to ISIS 301012 increased, serum apolipoprotein B, LDL-cholesterol and total cholesterol decreased.

The relationship between serum apolipoprotein B protein levels and plasma trough concentrations of ISIS 301012 is described with a sigmoidal inhibitory effect $E_{max}$ model using the data collected 3 days post dosing. The estimated plasma $EC_{50}$ and predicted liver concentrations based on prior nonhuman primate studies were 18 (±2) ng/mL and 60 ug/g, respectively. Plasma trough concentrations increases following multiple doses of ISIS 301012, reflecting accumulation of ISIS 301012 in the liver; increases were 2-fold following the loading dose of ISIS 301012, and 5-fold following the final dose of ISIS 301012. Monkey and human plasma pharmacokinetics for ISIS 301012 are essentially superimposable at mg/kg equivalent doses. Utilizing these known PK similarities, measurement of drug concentrations in the terminal elimination phase in human clinical studies can be used to assess accumulation in liver. Such estimates are shown in Table 48. ISIS 301012 liver concentrations are estimated based on monkey data. Monkey average plasma concentrations represent an average from 6 animals and were measured 48 to 72 hours after the last dose in a 13 week repeat dose toxicology study (e.g. the study described in Example 2). Human plasma trough concentration data was obtained 72 hours after the last intravenous loading dose; the number of study subjects is indicated in parentheses. Monkey liver concentrations represent an average of 4-6 animals and were measured in tissues collected 48 hours after the last dose in a 13 week repeat dose toxicology study.

**Table 48**

| **ISIS 301012: Monkey and Human Plasma Pharmacokinetics** | | |
|---|---|---|
| **Dose/Route** | **Plasma $C_{trough}$ (ng/mL)** | **Actual Liver Conc. (μg/g)** |
| **Monkey** | | |
| 3.5 mg/kg/week/i.v. | 28 | 100 ± 59 |
| 7 mg/kg/week/i.v. | 107 | 293 ± 105 |
| 21 mg/kg/week/i.v. | 292 | 584 ± 129 |
| 35 mg/kg/week/s.c. | 570 | 1129 ± 242 |
| **Human** | **Plasma $C_{trough}$ (ng/mL)** | ***Estimated* Liver Conc. (μg/g)** |
| 50 mg/week/i.v./s.c. | 24 (n=5) | *10* |
| 100 mg/week/i.v./s.c. | 8 (n=3) | 28 |
| 200 mg/week/i.v./s.c. | 18 (n=6) | *60* |
| 400 mg/week/i.v./s.c. | 40 (n=2) | *150* |

Plasma trough AUC for the 50 mg, 50mg, 100 mg, 200 mg and 400 mg dose groups was found to be 3, 5, 12 and 18 μg·hr/mL, respectively, on MD25 (three days following the final dose during the multiple dosing period).

Reducing the levels of LDL-cholesterol in a human provides a means for treating, preventing and/or ameliorating coronary heart disease. As described above, the oligonucleotide ISIS 301012 produced substantial, sustained reductions serum apolipoprotein B and LDL-cholesterol, as well as in other lipid parameters, and is thus an agent useful for the treatment of hypercholesterolemia.

Example 4

Loading and Maintenance Treatments

In order to treat subjects suffering from diseases related to production of apolipoprotein B or LDL-cholesterol, a series of different loading and maintenance regimens are analyzed for their therapeutic efficacy. Subjects are treated with ISIS 301012 as described below:

Group A receives a slow loading phase, during which 4 doses of 200 mg ISIS 301012 are administered over 11 days. This slow loading phase is followed by an 11 week maintenance phase, during which a 200 mg dose of ISIS 301012 is administered every other week.

Group B receives a slow loading phase, during which 4 doses of 200 mg ISIS 301012 are administered over 11 days. This slow loading phase is followed by an 11 week maintenance phase, during which a 100 mg dose of ISIS 301012 is administered every other week.

Group C receives a slow loading phase, during which 3 doses of 200 mg ISIS 301012 are administered weekly for three weeks. This slow loading phase is followed by a 3 month maintenance phase, during which a 200 mg dose of ISIS 301012 is administered once per month.

Group D receives a fast loading phase, during which 3 doses of 200 mg ISIS 301012 are administered over 7 days. This fast loading phase is followed by an 11 week maintenance phase, during which a 200 mg dose of ISIS 301012 is administered every other week.

Group E receives a fast loading phase, during which 3 doses of 200 mg ISIS 301012 are administered over 7 days. This fast loading phase is followed by a 3 month maintenance phase, during which a 200 mg dose of ISIS 301012 is administered once per month.

Group F receives a maintenance phase only, during which a 200 mg dose of ISIS 301012 is administered once weekly for 13 weeks.

Group G receives a maintenance phase only, during which a 200 mg dose of ISIS 301012 is administered once every other week for 13 weeks.

It is discovered that treating subjects in Groups A, B, C, D, E, F, and G with ISIS 301012 provides therapeutic benefits. The therapeutic benefits include reduction in serum LDL-cholesterol, serum apolipoprotein B, triglycerides, total cholesterol, non-HDL cholesterol, VLDL-cholesterol, lipoprotein(a), the LDL:HDL ratio and apolipoprotein B:apolipoprotein A-1 ratio relative to baseline. It is found that there is no change in the levels of HDL-cholesterol. The differences in onset and degree of therapeutic benefits are used to evaluate the therapeutic efficacy of each dosing regimen.

## Example 5

### Homozygous Familial Hypercholesterolemia

Familial hypercholesterolemia (FH) is an autosomal dominant metabolic disorder characterized by markedly elevated LDL-cholesterol and premature onset of atherosclerosis. Clinical symptoms result from the development of LDL-cholesterol deposits in the skin, tendons (xanthoma) and arterial plaques (atheromata). The primary genetic defect in FH is a mutation in the LDL-receptor (LDL-R) gene. The LDL-R facilitates the uptake of LDL-cholesterol into liver cells, where it is metabolized, resulting in the release of cholesterol for metabolic use. Defects in the LDL-R result in inefficient uptake of LDL-cholesterol, which in turn leads to delayed plasma clearance of LDL-cholesterol. The longer residence of LDL-cholesterol in plasma allows for its uptake by scavenger and other cells, which deposit the extraneous LDL-cholesterol and produce xanthoma and atheromata.

The prevalence of homozygous individuals worldwide is 1:1,000,000; approximately 300 homozygous FH subjects reside in the United States. Due to founder effects, certain countries have a higher prevalence rate, for example, French Canada, South Africa, Finland and Iceland. Nearly all subjects with homozygous FH will develop xanthomata by 5 years of age and atherosclerosis before adulthood. Despite treatment with existing cholesterol-lowering therapies, few homozygous FH subjects achieve treatment goals. Thus, homozygous FH subjects are in need of alternative therapeutic options.

ISIS 301012 is provided to adult FH subjects as a 200 mg/mL solution in 1 mL total volume and is administered subcutaneously as a single injection into the abdomen or thigh. In some individuals, the dose is divided and administered as 2 or 3 non-contiguous injections into the abdomen or thigh.

A total of 12 doses of ISIS 301012 are administered subcutaneously or intravenously. The first dose is administered on Day 1 of the study, while the remaining 11 doses are administered beginning on Day 8 and weekly thereafter, at which times subjects receive a 200 mg dose of ISIS 301012.

A group of pediatric FH subjects (ages 8 through 17) are also treated wherein the dosing is based upon body weight at time of screening. Pediatric subjects who are at least 25 kg but less than 37.5 kg receive a first dose of 50 mg ISIS 301012, followed by 100 mg dose of ISIS 301012 for the remainder of the study. Subjects who are at least 37.5 kg but less than 50 kg receive a first dose of 100 mg, followed by weekly 150 mg doses for the remainder of the study. Pediatric subjects who are greater than 50 kg receive the adult dose described above.

Following the treatment regime, each subject is found to have therapeutically relevant reduced levels of LDL-cholesterol, relative to baseline (pre-treatment) LDL-cholesterol. The subjects are also found to have reduced serum apolipoprotein B protein, triglyceride, VLDL-cholesterol, lipoprotein(a) and total cholesterol levels in comparison to baseline. Accordingly, ISIS 301012 provides a therapeutic benefit to the individuals treated with this compound.

Example 6

Heterozygous Familial Hypercholesterolemia

The prevalence of individuals that are heterozygous for Familial hypercholesterolemia is approximately 1:500, with approximately 500,000 heterozygous FH subjects residing in the United States. Whereas homozygous FH subjects have two copies of a mutated LDL-receptor allele, heterozygous FH subjects have one mutated LDL-receptor allele. Heterozygous FH subjects usually develop xanthomata in the $3^{rd}$ decade of life and atherosclerosis by the $4^{th}$ decade. Approximately 30% of subjects diagnosed with FH already have a history of cardiovascular disease. Despite maximal therapy, approximately 20% of these subjects are unable to achieve treatment goals. Therefore, heterozygous FH subjects are considered a population in need of alternative cholesterol-lowering treatments. For this reason, heterozygous FH subjects are chosen for treatment with ISIS 301012.

Doses of ISIS 301012 are administered as a subcutaneous injection in the abdomen or thigh once a week for 12 weeks. The first dose of ISIS 301012 is administered as a 100 mg dose. Provided that the subjects tolerate the study drug during the first week, all subsequent doses of ISIS 301012 are 200 mg (weeks 2 through 12 doses). ISIS 301012 is supplied as a 1 mL of a 200 mg/mL solution and 100 and 200 mg doses of ISIS 301012 are administered in volumes of 0.5 and 1.0 mL, respectively.

Following the treatment regime, each subject is found to have therapeutically relevant reduced levels of LDL-cholesterol, relative to baseline (pre-treatment) LDL-cholesterol. The subjects are also found to have reduced serum apolipoprotein B protein, triglyceride, VLDL-cholesterol, lipoprotein(a) and total cholesterol levels in comparison to baseline. Accordingly, ISIS 301012 provides a therapeutic benefit to the individuals treated with this compound.

Example 7

Targeted Minimum Plasma Concentration

A human subject suffering from high cholesterol levels is treated with the oligonucleotide ISIS 301012 targeted to apolipoprotein B. The subject is first given loading doses of ISIS 301012, which are calculated to result in a plasma trough concentration of at least 5 ng/mL. In this case, the subject receives a slow loading phase, during which 4 doses of 200 mg ISIS 301012 are administered over 11 days. Following the slow loading phase, the subject is treated with maintenance doses of ISIS 301012, which are calculated to result in a plasma trough concentration of at least 5 ng/ml. In this case, the subject is treated by weekly 200 mg maintenance doses of ISIS 301012. Plasma concentrations are determined by pharmacokinetic analysis of blood samples collected during and after the dosing periods. After several months of treatment, the subject only receives monthly or quarterly treatments with appropriate concentrations of ISIS 301012 in order to maintain a plasma trough concentration of at least 5 ng/ml.

Additional subjects receive doses a fast loading phase, which are calculated to result in a plasma trough concentration of at least 5 ng/mL. In this case, the subject receives 3 doses of 200 mg ISIS 301012 that are administered over 7 days. Following the fast loading phase, the subject is treated with maintenance doses of ISIS 301012, which are calculated to result in a plasma trough concentration of at least 5 ng/mL. In this case, the subject is treated by weekly 200 mg maintenance doses of ISIS 301012. Plasma concentrations are determined by pharmacokinetic analysis of blood samples collected during and after the dosing periods. After several months of treatment, the subject only receives monthly or quarterly treatments with appropriate concentrations of ISIS 301012 in order to maintain a plasma trough concentration of at least 5 ng/ml. It is discovered that treating the subject to have a minimum plasma concentration of 5 ng/ml results in a greater than 10% reduction in serum apolipoprotein B, serum LDL-cholesterol, serum triglyceride, and serum total cholesterol levels.

Further embodiments of the invention are described below.

1. A method of reducing serum cholesterol levels in a human subject, comprising administering to said subject a plurality of doses of an oligonucleotide targeted to apolipoprotein B, wherein said administering results in a plasma trough AUC from about 2 $\mu$g·hr/mL to about 20 $\mu$g·hr/mL for the oligonucleotide in said human subject.

2. The method of embodiment 1, wherein said administering results in a plasma trough AUC from about 2 $\mu$g·hr/mL to about 10 $\mu$g·hr/mL.

3. The method of embodiment 1, wherein said administering results in a plasma trough AUC from about 4 $\mu$g·hr/mL to about 6 $\mu$g·hr/mL.

4. The method of embodiment 1, wherein said administering results in a plasma trough AUC of about 5 $\mu$g·hr/mL.

5. The method of embodiments 1, 2, 3 or 4, wherein said plasma trough AUC is achieved from about 3 to about 33 days subsequent to the administration of a dose of said plurality of doses of the oligonucleotide.

6. The method of embodiment 1, wherein said serum cholesterol levels are reduced in said human subject by at least about ten percent.

7. The method of embodiment 1, wherein said serum cholesterol levels are reduced in said human subject by at least about thirty percent.

8. The method of embodiment 1, wherein said serum cholesterol levels are serum low density lipoprotein (LDL) cholesterol levels.

9. The method of embodiment 1, wherein said serum cholesterol levels are serum very low density lipoprotein (VLDL) cholesterol levels.

10. The method of embodiment 1, wherein said oligonucleotide comprises the nucleobase sequence "GCCTCAGTCTGCTTCGCACC" (SEQ ID NO: 2).

11. The method of embodiment 1, wherein at least one dose of said plurality of doses is administered about once a week.

12. The method of embodiment 1, wherein at least one dose of said plurality of doses is administered about once a month.

13. The method of embodiment 1, wherein said human subject suffers from hypercholesterolemia.

14. The method of embodiment 1, wherein each dose of said plurality of doses comprises from about 50 mg to about 400 mg of the oligonucleotide.

15. The method of embodiment 1, wherein each dose of said plurality of doses comprises about 200 mg of the oligonucleotide.

16. A method of reducing serum cholesterol levels in a human subject, comprising administering to said subject a plurality of doses of an oligonucleotide targeted to apolipoprotein B, wherein said administering results in a plasma trough concentration from about 5 ng/mL to about 40 ng/mL of the oligonucleotide in said human subject.

17. The method of embodiment 16, wherein said administering results in a plasma trough concentration from about 5 ng/mL to about 20 ng/mL.

18. The method of embodiments 16 or 17, wherein said plasma trough concentration is achieved about 7 days subsequent to the administration of a dose of said plurality of doses of the oligonucleotide.

19. The method of embodiment 16, wherein said serum cholesterol levels are reduced in said human subject by at least about ten percent.

20. The method of embodiment 16, wherein said serum cholesterol levels are reduced in said human subject by at least about thirty percent.

21. The method of embodiment 16, wherein said serum cholesterol levels are serum low density lipoprotein (LDL) cholesterol levels.

22. The method of embodiment 16, wherein said serum cholesterol levels are serum very low density lipoprotein (VLDL) cholesterol levels.

23. The method of embodiment 16, wherein said oligonucleotide comprises the nucleobase sequence "GCCTCAGTCTGCTTCGCACC" (SEQ ID NO: 2).

24. The method of embodiment 16, wherein at least one dose of said plurality of doses is administered about once a week.

25. The method of embodiment 16, wherein at least one dose of said plurality of doses is administered about once a month.

26. The method of embodiment 16, wherein said human subject suffers from hypercholesterolemia.

27. The method of embodiment 16, wherein each dose of said plurality of doses comprises from about 50 mg to about 400 mg of the oligonucleotide.

28. The method of embodiment 16, wherein each dose of said plurality of doses comprises about 200 mg of the oligonucleotide.

29. A method of reducing serum LDL-cholesterol in a human subject, comprising administering to the human subject a dose of an oligonucleotide comprising the nucleobase sequence "GCCTCAGTCTGCTTCGCACC" (SEQ ID NO: 2), sufficient to achieve a plasma trough AUC of at least about 2 μg·hr/mL.

30. The method of embodiment 29, wherein said dose is sufficient to achieve a plasma trough AUC in the range of about 2 μg·hr/mL to about 20 μg·hr/mL.

31. The method of embodiments 29 or 30, wherein said plasma trough AUC is achieved from about 3 to about 33 days subsequent to the administration of said dose of the oligonucleotide.

32. The method of embodiment 31, wherein said dose is administered about once a week, about once a month or about once every three months.

33. A method of reducing serum LDL-cholesterol in a human subject, comprising administering to the human subject a dose of an oligonucleotide comprising the nucleobase sequence "GCCTCAGTCTGCTTCGCACC" (SEQ E) NO: 2), sufficient to achieve a plasma trough concentration of at least about 5 ng/mL.

34. The method of embodiment 33, wherein said dose is sufficient to achieve a plasma trough concentration in the range of about 5 ng/mL to about 40 ng/mL.

35. The method of embodiments 33 or 34, wherein said plasma trough concentration is achieved about 7 days subsequent to the administration of said dose of the oligonucleotide.

36. The method of embodiment 33, wherein said dose is administered about once a week, about once a month or about once every three months.

37. A method of reducing serum LDL-cholesterol in a human subject, comprising administering to the human subject a dose of an oligonucleotide comprising the nucleobase sequence "GCCTCAGTCTGCTTCGCACC" (SEQ ID NO: 2), sufficient to achieve an estimated liver concentration of at least about 10 μg/g.

38. The method of embodiment 37, wherein said dose is sufficient to achieve an estimated liver concentration in the range of about 10 μg/g to about 150 μg/g.

39. The method of embodiment 37, wherein said dose is administered about once a week, about once a month or about once every three months.

40. A method of reducing serum LDL-cholesterol in a human subject comprising administering to said human subject an oligonucleotide comprising the nucleobase sequence "GCCTCAGTCTGCTTCGCACC" (SEQ ID NO: 2), wherein said oligonucleotide is administered during a loading period and a maintenance period.

41. The method of embodiment 40, wherein said oligonucleotide is 20 to 30 nucleobases in length.

42. The method of embodiment 40, wherein the loading period comprises administering the oligonucleotide to the human subject once a day for up to 10 days.

43. The method of embodiment 42, wherein the oligonucleotide during the loading period is administered intravenously or subcutaneously.

44. The method of embodiment 40, wherein the maintenance period comprises administering the oligonucleotide at least once about every 3 months.

45. The method of embodiment 44, wherein the oligonucleotide during the maintenance period is administered once about every month.

46. The method of embodiment 44, wherein the oligonucleotide is administered once about every 2 weeks.

47. The method of embodiment 44, wherein the oligonucleotide is administered once about every 7 days.

48. The method of embodiment 44, wherein the oligonucleotide during the maintenance period is administered subcutaneously.

49. The method of embodiment 42 or 44, wherein a dose from about 0.1 mg/kg/day to about 5 mg/kg/day is administered.

50. The method of embodiment 49, wherein the dose is from about 0.1 mg/kg/day to about 1.2 mg/kg/day.

51. The method of embodiment 42 or 44, where the dose is from about 50 mg per week to about 600 mg per week.

52. The method of embodiment 51, wherein the dose is about 50 mg per week.

53. The method of embodiment 51, wherein the dose is about 100 mg per week.

54. The method of embodiment 51, wherein the dose is about 200 mg per week.

55. The method of embodiment 51, wherein the dose is about 400 mg per week

56. The method of embodiment 40, wherein said oligonucleotide comprises ISIS 301012.

57. The method of embodiment 40, wherein said method results in a reduction in serum VLDL-cholesterol, serum triglycerides, serum lipoprotein(a) or any combination of VLDL- cholesterol, serum triglycerides and serum lipoprotein(a).

58. The method of embodiment 40, wherein said human subject exhibits at least one indication selected from the group consisting of: an elevated serum total cholesterol level, an elevated serum LDL-cholesterol level, an elevated total cholesterol:HDL ratio and an elevated LDL:HDL ratio.

59. The method of embodiment 40, wherein said human subject has suffered from or suffers from homozygous familial hypercholesterolemia or heterozygous familial hypercholesterolemia.

60. The method of embodiment 40, wherein said human subject has suffered from, suffers from or is at an increased risk for nonfamilial hypercholesterolemia.

61. The method of embodiment 40, wherein the human subject has serum LDL-cholesterol levels above about 70 mg/dL prior to administering said oligonucleotide.

62. The method of embodiment 40, wherein the human subject has serum LDL-cholesterol levels above about 100 mg/dL prior to administering said oligonucleotide.

63. The method of embodiment 40, wherein the human subject has serum LDL-cholesterol levels above about 130 mg/dL prior to administering said oligonucleotide.

64. The method of embodiment 61, 62, or 63, wherein administering said oligonucleotide reduces serum LDL-cholesterol levels in the human subject to less than about 70 mg/dL.

65. The method of embodiment 62 or 63, wherein administering said oligonucleotide reduces serum LDL-cholesterol levels in the human subject to less than about 100 mg/dL.

66. The method of embodiment 63, wherein administering said oligonucleotide reduces serum LDL cholesterol levels in the human subject to less than about 130 mg/dL.

67. A method of reducing serum cholesterol levels in a human subject, comprising: selecting a human subject previously unsuccessfully treated by a statin; and administering to said human subject an oligonucleotide comprising the nucleobase sequence "GCCTCAGTCTGCTTCGCACC" (SEQ ID NO: 2).

68. The method of embodiment 67, wherein said serum cholesterol levels comprise serum LDL-cholesterol levels.

69. The method of embodiment 67, wherein the human subject is intolerant to a statin.

70. The method of embodiment 67, wherein the subject has experienced adverse effects resulting from treatment with said statin.

71. The method of embodiment 70, wherein the subject has experienced myopathy, fatigue, Central Nervous System (CNS) effects resulting from treatment with said statin or any combination of myopathy, fatigue, and CNS effects resulting from treatment with said statin.

72. The method of embodiment 67, wherein the human subject has insufficient LDL-receptor activity.

73. The method of embodiment 67, wherein said oligonucleotide comprises ISIS 301012.

74. A method of using an oligonucleotide comprising the nucleobase sequence "GCCTCAGTCTGCTTCGCACC" (SEQ ID NO: 2) in a treatment for reducing serum LDL- cholesterol in a human subject, said method comprising informing said human subject that the administration of said oligonucleotide results in a plasma trough AUC of at least about 2 μg·hr/mL.

75. The method of embodiment 74, wherein said human subject is informed that the administration of said oligonucleotide results in a plasma trough AUC in the range of about 2 μ·hr/mL to about 20 μg·hr/mL.

76. The method of embodiments 74 or 75, wherein said plasma trough AUC is achieved from about 3 to about 33 days subsequent to the administration of said dose of the oligonucleotide.

77. The method of embodiment 74, wherein informing said human subject comprises providing printed matter that advises that the administration of said oligonucleotide results in a plasma trough AUC of at least about 2 μg·hr/mL.

78. The method of embodiment 77, wherein said printed matter is a label.

79. A method of using an oligonucleotide comprising the nucleobase sequence "GCCTCAGTCTGCTTCGCACC" (SEQ ID NO: 2) in a treatment for reducing serum LDL- cholesterol in a human subject, said method comprising informing said human subject that the administration of said oligonucleotide results in a plasma trough concentration of at least about 5 ng/mL.

80. The method of embodiment 79, wherein said human subject is informed that the administration of said oligonucleotide results in a plasma trough concentration in the range of about 5 ng/mL to about 40 ng/mL.

81. The method of embodiments 79 or 80, wherein said plasma trough concentration is achieved about 7 days subsequent to the administration of said dose of the oligonucleotide.

82. The method of embodiment 79, wherein informing said human subject comprises providing printed matter that advises that the administration of said oligonucleotide results in a plasma trough concentration of at least about 5 ng/mL.

83. The method of embodiment 82, wherein said printed matter is a label.

84. A method of using an oligonucleotide comprising the nucleobase sequence "GCCTCAGTCTGCTTCGCACC" (SEQ DD NO: 2) in a treatment for reducing serum LDL- cholesterol in a human subject, said method comprising informing said human subject that the administration of said oligonucleotide results in an estimated liver concentration of said oligonucleotide of at least about 10 μg/g.

85. The method of embodiment 84, wherein said human subject is informed that the administration of said oligonucleotide results in an estimated liver concentration of said oligonucleotide in the range of about 10 μg/g to about 150 μg/g.

86. The method of embodiment 84, wherein informing said human subject comprises providing printed matter that advises that the administration of said oligonucleotide results in an estimated liver concentration of said oligonucleotide of at least about 10 μg/g.

87. The method of embodiment 86, wherein said printed matter is a label.

88. A pharmaceutical composition comprising one or more doses of an oligonucleotide that is 14 to 30 nucleobases in length and which hybridizes to a nucleic acid sequence encoding apolipoprotein B, wherein each of said one or more doses ranges from about 50 mg to about 400 mg, and wherein intravenous administration to a human subject of said oligonucleotide at about 0.7 mg/kg of body weight to about 5.9 mg/kg of body weight is sufficient to achieve a plasma $AUC_{0-48}$ from about 11 μg·hr/mL to about 148 μg·hr/mL.

89. The pharmaceutical composition of embodiment 88, wherein intravenous administration to a human subject of said oligonucleotide at about 0.7 mg/kg of body weight is sufficient to achieve a plasma $AUC_{0-48}$ of 11 μg·hr/mL ± 3 μg·hr/mL.

90. The pharmaceutical composition of embodiment 88, wherein intravenous administration to a human subject of said oligonucleotide at about 1 mg/kg of body weight is sufficient to achieve a plasma $AUC_{0-48}$ of 24 μg·hr/mL ± 3 μig·hr/mL.

91. The pharmaceutical composition of embodiment 88, wherein intravenous administration to a human subject of said oligonucleotide at about 2.7 mg/kg of body weight is sufficient to achieve a plasma $AUC_{0-48}$ of 68 μg·hr/mL ± 14 μg·hr/mL.

92. The pharmaceutical composition of embodiment 88, wherein intravenous administration to a human subject of said oligonucleotide at about 5.9 mg/kg of body weight is sufficient to achieve a plasma $AUC_{0-48}$ of 148 μg·hr/mL ± 14 μg·hr/mL.

93. The pharmaceutical composition of any of embodiments 88 to 92, wherein said oligonucleotide hybridizes to a region of said nucleic acid sequence encoding apolipoprotein B that comprises nucleotides 2920 to 3420 of SEQ ID NO: 1.

94. The pharmaceutical composition of any of embodiments 88 to 92, wherein said oligonucleotide hybridizes to a region of said nucleic acid sequence encoding apolipoprotein B that comprises nucleotides 3230 to 3288 of SEQ ID NO: 1.

95. The pharmaceutical composition of any of embodiments 88 to 92, wherein said oligonucleotide comprises the nucleobase sequence "GCCTCAGTCTGCTTCGCACC" (SEQ ID NO: 2).

96. The pharmaceutical composition of embodiment 95, wherein said oligonucleotide comprises ISIS 301012.

97. A pharmaceutical composition comprising one or more doses of an oligonucleotide that is 14 to 30 nucleobases in length and which hybridizes to a nucleic acid sequence encoding apolipoprotein B, wherein each of said one or more doses ranges from about 50 mg to about 400 mg, and wherein subcutaneous administration to a human subject of said oligonucleotide at about 0.7 mg/kg of body weight to about 5.9 mg/kg of body weight, subsequent to the administration of one or more loading doses, is sufficient to achieve a plasma AUC from about 19 μg·hr/mL to about 160 μg·hr/mL.

98. The pharmaceutical composition of embodiment 97, wherein subcutaneous administration to a human subject of said oligonucleotide at about 0.7 mg/kg of body weight is sufficient to achieve a plasma AUC of 19 μg·hr/mL ± 9 μg·hr/mL.

99. The pharmaceutical composition of embodiment 97, wherein subcutaneous administration to a human subject of said oligonucleotide at about 1 mg/kg of body weight is sufficient to achieve a plasma AUC of 28 μg·hr/mL ± 5 μg·hr/mL.

100. The pharmaceutical composition of embodiment 97, wherein subcutaneous administration to a human subject of said oligonucleotide at about 2.7 mg/kg of body weight is sufficient to achieve a plasma AUC of 63 μg·hr/mL ± 13 μg·hr/mL.

101. The pharmaceutical composition of embodiment 97, wherein subcutaneous administration to a human subject of said oligonucleotide at about 5.9 mg/kg of body weight is sufficient to achieve a plasma AUC of 160 μg·hr/mL.

102. The pharmaceutical composition of any of embodiments 97 to 101, wherein said oligonucleotide hybridizes to a region of said nucleic acid sequence encoding apolipoprotein B that comprises nucleotides 2920 to 3420 of SEQ ID NO: 1.

103. The pharmaceutical composition of any of embodiments 97 to 101, wherein said oligonucleotide hybridizes to a region of said nucleic acid sequence encoding apolipoprotein B that comprises nucleotides 3230 to 3288 of SEQ ID NO: 1.

104. The pharmaceutical composition of any of embodiments 97 to 101, wherein said oligonucleotide comprises the nucleobase sequence "GCCTCAGTCTGCTTCGCACC" (SEQ ID NO: 2).

105. The pharmaceutical composition of embodiment 104, wherein said oligonucleotide comprises ISIS 301012.

106. A pharmaceutical composition comprising one or more doses of an oligonucleotide that is 14 to 30 nucleobases in length and which hybridizes to a nucleic acid sequence encoding apolipoprotein B, wherein each of said one or more doses ranges from about 50 mg to about 400 mg, and wherein subcutaneous administration to a human subject of said oligonucleotide at about 0.7 mg/kg of body weight to about 5.9 mg/kg of body weight, subsequent to the administration of one or more loading doses, is sufficient to achieve a plasma trough concentration from about 2 ng/mL to about 40 ng/mL.

107. The pharmaceutical composition of embodiment 106, wherein said oligonucleotide hybridizes to a region of said nucleic acid sequence encoding apolipoprotein B that comprises nucleotides 2920 to 3420 of SEQ ID NO: 1.

108. The pharmaceutical composition of embodiment 106, wherein said oligonucleotide hybridizes to a region of said nucleic acid sequence encoding apolipoprotein B that comprises nucleotides 3230 to 3288 of SEQ E) NO: 1.

109. The pharmaceutical composition of embodiment 106, wherein said oligonucleotide comprises the nucleobase sequence "GCCTCAGTCTGCTTCGCACC" (SEQ ID NO: 2).

110. A pharmaceutical composition comprising one or more doses of an oligonucleotide that is 14 to 30 nucleobases in length and which hybridizes to a nucleic acid sequence encoding apolipoprotein B, wherein each of said one or more doses ranges from about 50 mg to about 400 mg, and wherein subcutaneous administration to a human subject of said oligonucleotide at about 0.7 mg/kg of body weight to about 5.9 mg/kg of body weight, subsequent to the administration of one or more loading doses, is sufficient to achieve a bioavailability of at least about 54%.

111. The pharmaceutical composition of embodiment 110, wherein said oligonucleotide hybridizes to a region of said nucleic acid sequence encoding apolipoprotein B that comprises nucleotides 2920 to 3420 of SEQ ID NO: 1.

112. The pharmaceutical composition of embodiment 110, wherein said oligonucleotide hybridizes to a region of said nucleic acid sequence encoding apolipoprotein B that comprises nucleotides 3230 to 3288 of SEQ ID NO: 1.

113. The pharmaceutical composition of embodiment 110, wherein said oligonucleotide comprises the nucleobase sequence "GCCTCAGTCTGCTTCGCACC" (SEQ ID NO: 2).

114. A pharmaceutical composition comprising one or more doses of an oligonucleotide that is 14 to 30 nucleobases in length and which hybridizes to a nucleic acid sequence encoding apolipoprotein B, wherein each of said one or more doses ranges from about 50 mg to about 400 mg, and wherein subcutaneous administration to a human subject of said oligonucleotide at about 0.7 mg/kg of body weight to about 5.9 mg/kg of body weight, subsequent to the administration of one or more loading doses, is sufficient to achieve a terminal elimination $t_{1/2}$ from about 23 to about 47 days.

115. The pharmaceutical composition of embodiment 114, wherein subcutaneous administration to a human subject of said oligonucleotide at about 0.7 mg/kg of body weight is sufficient to achieve a terminal elimination $t_{1/2}$ of about 23 days $\pm$ 1 day.

116. The pharmaceutical composition of embodiment 114, wherein subcutaneous administration to a human subject of said oligonucleotide at about 1 mg/kg of body weight is sufficient to achieve a terminal elimination $t_{1/2}$ of about 27 days $\pm$ 12 days.

117. The pharmaceutical composition of embodiment 114, wherein subcutaneous administration to a human subject of said oligonucleotide at about 2.7 mg/kg of body weight is sufficient to achieve a terminal elimination $t_{1/2}$ of about 31 days $\pm$ 11 days.

118. The pharmaceutical composition of embodiment 114, wherein subcutaneous administration to a human subject of said oligonucleotide at about 5.9 mg/kg of body weight is sufficient to achieve a terminal elimination $t_{1/2}$ of about 47 days.

119. The pharmaceutical composition of any of embodiments 114 to 118, wherein said oligonucleotide hybridizes to a region of said nucleic acid sequence encoding apolipoprotein B that comprises nucleotides 2920 to 3420 of SEQ ID NO: 1.

120. The pharmaceutical composition of any of embodiments 114 to 118, wherein said oligonucleotide hybridizes to a region of said nucleic acid sequence encoding apolipoprotein B that comprises nucleotides 3230 to 3288 of SEQ E) NO: 1.

121. The pharmaceutical composition of any of embodiments 114 to 118, wherein said oligonucleotide comprises the nucleobase sequence "GCCTCAGTCTGCTTCGCACC" (SEQ ID NO: 2).

122. The pharmaceutical composition of embodiment 121, wherein said oligonucleotide comprises ISIS 301012.

123. A plurality of doses of an oligonucleotide targeted to apolipoprotein B for reducing serum cholesterol levels wherein administration of said plurality of doses said oligonucleotide results in a plasma trough AUC for said oligonucleotide from about 2 $\mu$g·hr/mL to about 20 $\mu$g·hr/mL.

124. The plurality of doses of embodiment 123, wherein said administration results in a plasma trough AUC from about 2 $\mu$g·hr/mL to about 10 $\mu$g·hr/mL.

125. The plurality of doses of embodiment 123, wherein said administration results in a plasma trough AUC from about 4 $\mu$g·hr/mL to about 6 $\mu$g·hr/mL.

126. The plurality of doses of embodiment 123, wherein said administration results in a plasma trough AUC of about 5 $\mu$g·hr/mL.

127. The plurality of doses of embodiments 123, 124, 125 or 126, wherein said plasma trough AUC is achieved from about 3 to about 33 days subsequent to the administration of a dose of said plurality of doses of the oligonucleotide.

128. The plurality of doses of embodiment 123, wherein said serum cholesterol levels are reduced by least ten percent.

129. The plurality of doses of embodiment 123, wherein said serum cholesterol levels is are reduced by least thirty percent.

130. The plurality of doses of embodiment 123, wherein said serum cholesterol levels are serum LDL-cholesterol levels.

131. The plurality of doses of embodiment 123, wherein said serum cholesterol levels are serum VLDL- cholesterol levels.

132. The plurality of doses of embodiment 123, wherein said oligonucleotide comprises the nucleobase sequence "GCCTCAGTCTGCTTCGCACC" (SEQ ID NO: 2).

133. The plurality of doses of embodiment 127, wherein administration is about once a week.

134. The plurality of doses of embodiment 127, wherein administration is about once a month.

135. The plurality of doses of embodiment 127, wherein each of said plurality of doses comprises from about 50 mg to about 400 mg of said oligonucleotide.

136. The plurality of doses of embodiment 127, wherein each of said plurality of doses comprises about 200 mg of said oligonucleotide.

137. A plurality of doses of an oligonucleotide targeted to apolipoprotein B for reducing serum cholesterol levels wherein administration of said plurality of doses said oligonucleotide results in a plasma trough concentration from about 5 ng/mL to about 40 ng/mL.

138. The plurality of doses of embodiment 137, wherein administration results in a plasma trough concentration from about 5 ng/mL to about 20 ng/mL.

139. The plurality of doses of embodiments 137 or 138, wherein said plasma trough concentration is achieved about 7 days subsequent to the administration of a dose of said plurality of doses of the oligonucleotide.

140. The plurality of doses of embodiment 137, wherein said serum cholesterol levels are reduced by least ten percent.

141. The plurality of doses of embodiment 137, wherein said serum cholesterol levels are reduced by least thirty percent.

142. The plurality of doses of embodiment 137, wherein said serum cholesterol levels are serum LDL-cholesterol levels.

143. The plurality of doses of embodiment 137, wherein said serum cholesterol levels are serum VLDL-cholesterol levels.

144. The plurality of doses of embodiment 137, wherein said oligonucleotide comprises the nucleobase sequence "GCCTCAGTCTGCTTCGCACC" (SEQ ID NO: 2).

145. The plurality of doses of embodiment 137, wherein administration is about once a week.

146. The plurality of doses of embodiment 137, wherein administration is about once a month.

147. The plurality of doses of embodiment 137, wherein each of said plurality of doses comprises from about 50 mg to about 400 mg of said oligonucleotide.

148. The plurality of doses of embodiment 137, wherein each of said plurality of doses comprises about 200 mg of said oligonucleotide.

149. Use of a plurality of doses of an oligonucleotide targeted to apolipoprotein B for the preparation of a medicament for reducing serum cholesterol levels, wherein administration of said medicament results in a plasma trough AUC for said oligonucleotide from about 2 μg·hr/mL to about 10 μg·hr/mL.

150. The use of embodiment 149, wherein administration of said medicament results in a plasma trough AUC from

about 2 μg·hr/mL to about 10 μg·hr/mL.

151. The use of embodiment 149, wherein administration of said medicament results in a plasma trough AUC from about 4 μg·hr/mL to about 6 μg·hr/mL.

152. The use of embodiment 149, wherein administration of said medicament results in a plasma trough AUC of about 5 μg·hr/mL.

153. The use of embodiments 149, 150, 151 or 152, wherein said plasma trough AUC is achieved from about 3 to about 33 days subsequent to the administration of a dose of said medicament.

154. The use of embodiment 149, wherein said serum cholesterol levels are reduced by least about ten percent.

155. The use of embodiment 149, wherein said serum cholesterol levels are reduced by least about thirty percent.

156. The use of embodiment 149, wherein said serum cholesterol levels are serum LDL- cholesterol levels.

157. The use of embodiment 149, wherein said serum cholesterol levels are serum VLDL- cholesterol levels.

158. The use of embodiment 149, wherein said oligonucleotide comprises the nucleobase sequence "GCCTCAGTCTGCTTCGCACC" (SEQ ID NO: 2).

159. The use of embodiment 149, wherein said administration is about once a week.

160. The use of embodiment 149, wherein said administration is about once a month.

161. The use of embodiment 149, wherein each of said plurality of doses comprises from about 50 mg to about 400 mg of said oligonucleotide.

162. The use of embodiment 149, wherein each of said plurality of doses comprises about 200 mg of said oligonucleotide.

163. Use of a plurality of doses of an oligonucleotide targeted to apolipoprotein B for the preparation of a medicament for reducing serum cholesterol levels, wherein administration of said medicament results in a plasma trough concentration of said oligonucleotide from about 5 ng/mL to about 40 ng/mL.

164. The use of embodiment 163, wherein administration of said medicament results in a plasma trough concentration from about 5 ng/mL to about 20 ng/mL.

165. The use of embodiments 163 or 164, wherein said plasma trough concentration is achieved about 7 days subsequent to the administration of a dose of said medicament.

166. The use of embodiment 163, wherein said serum cholesterol levels are reduced by least about ten percent.

167. The use of embodiment 163, wherein said serum cholesterol levels are reduced by least about thirty percent.

168. The use of embodiment 163, wherein said serum cholesterol levels are serum LDL- cholesterol levels.

169. The use of embodiment 163, wherein said serum cholesterol levels are serum VLDL- cholesterol levels.

170. The use of embodiment 163, wherein said oligonucleotide comprises the nucleobase sequence "GCCTCAGTCTGCTTCGCACC" (SEQ E) NO: 2).

171. The use of embodiment 163, wherein administration is about once a week.

172. The use of embodiment 163, wherein administration is about once a month.

173. The use of embodiment 163, wherein each of said plurality of doses comprises from about 50 mg to about 400 mg of said oligonucleotide.

174. The use of embodiment 163, wherein each of said plurality of doses comprises about 200 mg of said oligonucleotide.

175. Use of an oligonucleotide comprising the nucleobase sequence "GCCTCAGTCTGCTTCGCACC" (SEQ ID NO: 2) for the preparation of a medicament for reducing serum LDL-cholesterol, wherein administration of said medicament is sufficient to achieve a plasma trough AUC for said oligonucleotide of at least about 2 μg·hr/mL.

176. The use of embodiment 175, wherein said administration of said medicament is sufficient to achieve a plasma trough AUC in the range of about 2 μg·hr/mL to about 20 μg·hr/mL.

177. The use of embodiments 175 or 176, wherein said plasma trough AUC is achieved from about 3 to about 33 days subsequent to the administration of said medicament.

178. The use of embodiment 175, wherein the administration of said medicament occurs about once a week, about once a month or about once every three months.

179. Use of an oligonucleotide comprising the nucleobase sequence "GCCTCAGTCTGCTTCGCACC" (SEQ E) NO: 2) for the preparation of a medicament for reducing serum LDL-cholesterol, wherein administration of said medicament is sufficient to achieve a plasma trough concentration for said oligonucleotide of at least about 5 ng/mL.

180. The use of embodiment 179, wherein said administration of said medicament is sufficient to achieve a plasma trough concentration in the range of about 5 ng/mL to about 40 ng/mL.

181. The use of embodiments 179 or 180, wherein said plasma trough concentration is achieved about 7 days subsequent to the administration of said medicament.

182. The use of embodiment 179, wherein the administration of said medicament occurs about once a week, about once a month or about once every three months.

183. Use of an oligonucleotide comprising the nucleobase sequence "GCCTCAGTCTGCTTCGCACC" (SEQ JD NO: 2) for the preparation of a medicament for reducing serum LDL-cholesterol, wherein administration of said medicament is sufficient to achieve an estimated liver concentration of at least about 10 μg/g.

184. The use of embodiment 183, wherein said administration of said medicament is sufficient to achieve an estimated liver concentration in the range of about 10 μg/g to about 150 μg/g.

185. The use of embodiment 183, wherein the administration of said medicament occurs about once a week, about once a month or about once every three months.

186. Use of an oligonucleotide comprising the nucleobase sequence "GCCTCAGTCTGCTTCGCACC" (SEQ ID NO: 2) for the preparation of a medicament for reducing serum LDL-cholesterol, wherein said medicament is administered during a loading period and a maintenance period.

187. The use of embodiment 186, wherein said oligonucleotide is 20 to 30 nucleobases in length.

188. The use of embodiment 186, wherein the loading period comprises administering the medicament once a day for up to 10 days.

189. The use of embodiment 188, wherein said medicament is administered intravenously or subcutaneously.

190. The use of embodiment 186, wherein the maintenance period comprises administering the medicament at least once about every 3 months.

191. The use of embodiment 190, wherein the medicament is administered once about every month.

192. The use of embodiment 190, wherein the medicament is administered once about every 2 weeks.

193. The use of embodiment 190, wherein the medicament is administered once about every 7 days.

194. The use of embodiment 190, wherein said medicament is administered intravenously or subcutaneously.

195. The use of embodiments 188 or 190, wherein the oligonucleotide present in the medicament is administered in a dose from about 0.1 mg/kg/day to about 5 mg/kg/day.

196. The use of embodiments 195, wherein the oligonucleotide present in the medicament is administered in a dose from about 0.1 mg/kg/day to about 1.2 mg/kg/day.

197. The use of embodiments 188 or 190, wherein the oligonucleotide present in the medicament is administered in a dose from about 50 mg per week to about 600 mg per week.

198. The use of embodiment 197, wherein the oligonucleotide present in the medicament is administered in a dose of about 50 mg per week.

199. The use of embodiment 197, wherein the dose is about 100 mg per week.

200. The use of embodiment 197, wherein the dose is about 200 mg per week.

201. The use of embodiment 197, wherein the dose is about 400 mg per week.

202. The use of embodiment 186, wherein the oligonucleotide comprises ISIS 301012.

203. The use of embodiment 186, wherein administration of the medicament results in a reduction in serum VLDL-cholesterol, serum triglycerides, serum lipoprotein(a) or any combination of serum VLDL-cholesterol, serum triglycerides and serum lipoprotein(a).

204. The use of embodiment 186, wherein the medicament is administered to a human subject that exhibits at least one indication selected from the group consisting of an elevated serum total cholesterol level, an elevated serum LDL-cholesterol level, an elevated total cholesterol:HDL ratio and an elevated LDL:HDL ratio.

205. The use of embodiment 186, wherein the medicament is administered to a human subject who has suffered from or suffers from homozygous familial hypercholesterolemia, or heterozygous familial hypercholesterolemia.

206. The use of embodiment 186, wherein the medicament is administered to a human subject who has suffered from, suffers from or is at an increased risk for nonfamilial hypercholesterolemia.

207. The use of embodiment 186, wherein the medicament is administered to a human subject who has serum-LDL cholesterol levels above about 70 mg/dL prior to administration.

208. The use of embodiment 186, wherein the medicament is administered to a human subject who has serum-LDL cholesterol levels above about 100 mg/dL prior to administration.

209. The use of embodiment 186, wherein the medicament is administered to a human subject who has serum-LDL cholesterol levels above about 130 mg/dL prior to administration.

210. The use of embodiments 207, 208 or 209, wherein the administering said medicament reduces the serum-LDL cholesterol levels to less than about 70 mg/dL.

211. The use of embodiments 208 or 209, wherein the administering said medicament reduces the serum-LDL cholesterol levels to less than about 100 mg/dL.

212. The use of embodiment 209, wherein the administering said medicament reduces the serum LDL-cholesterol levels to less than about 130 mg/dL.

213. The method of embodiments 29, 33, 37, 74, 79, or 84, wherein said oligonucleotide comprises ISIS 301012.

214. The pharmaceutical composition of embodiments 109 or 113, wherein said oligonucleotide comprises ISIS 301012.

215. The plurality of doses of embodiments 132 or 144, wherein said oligonucleotide comprises ISIS 301012.

216. The use of embodiments 158, 170, 175, 179, 183, 186, wherein said oligonucleotide comprises ISIS 301012.

## Claims

1. An oligonucleotide comprising the nucleobase sequence of SEQ ID NO:2, for use in treating homozygous familial hypercholesterolemia.

2. The oligonucleotide for use according to claim 1, wherein the amount of oligonucleotide administered per week is 50-600 mg, such as 50, 100, 150, 200, 250, 300, 350, 400, 450, 500, 550 or 600 mg.

3. The oligonucleotide for use according to claim 1, wherein the oligonucleotide is administered in single or multiple doses of 0.1-5 mg/kg, 0.5-3 mg/kg, 0.5-8 mg/kg, 0.25-3 mg/kg, or 0.25-2 mg/kg.

4. The oligonucleotide for use according to claim 1, wherein the oligonucleotide is administered in single or multiple doses of 25-800 mg, 25-600 mg, 25-400 mg, 25-200 mg, 50-600 mg, 50-400 mg, or 50-200 mg per day.

5. The oligonucleotide for use according to any one of the preceding claims, wherein the oligonucleotide is administered by intravenous or subcutaneous injection.

6. The oligonucleotide for use according to any one of the preceding claims, wherein the oligonucleotide consists of the nucleobase sequence of SEQ ID NO:2.

7. The oligonucleotide for use according to any one of the preceding claims, wherein the oligonucleotide is a chimeric oligonucleotide gapmer 20 nucleotides in length and is composed of a central gap region consisting of ten 2'-deoxynucleotides flanked on both sides by five-nucleotide wings composed of 2'-methoxyethyl (2'MOE) nucleotides, wherein the internucleoside backbone linkages are phosphorothioate throughout the oligonucleotide and all cytosine residues are 5-methylcytosines.

8. The oligonucleotide for use according to any one of the preceding claims, wherein the treating lowers one or more lipid parameters selected from the group consisting of: serum LDL-cholesterol, serum apolipoprotein B, serum VLDL-cholesterol, serum total cholesterol, serum lipoprotein(a), serum triglycerides, serum total cholesterol:HDL ratio, and serum LDL:HDL ratio.

9. The oligonucleotide for use according to any one of the preceding claims, wherein the treating is in a human subject previously unsuccessfully treated by a statin.

10. The oligonucleotide for use according to any one of the preceding claims, wherein the oligonucleotide is administered with a second lipid-lowering agent.

11. The oligonucleotide for use according to claim 10, wherein the second lipid-lowering agent is selected from the group consisting of: bile acid sequestrants (e.g., cholestyramine, colestipol, and colesevelam hydrochloride), fibrates (e.g., clofibrate, gemfibrozil, fenofibrate, bezafibrate, and ciprofibrate), niacin, statins (e.g., lovastatin, prevastatin, atorvastatin, simvastatin, and fluvastatin), and cholesterol absorption inhibitors (e.g., ezetimibe).

12. The oligonucleotide for use according to any one of the preceding claims, wherein the oligonucleotide is a pharmaceutically acceptable salt.

13. The oligonucleotide for use according to any one of the preceding claims, wherein the treating comprises measuring one or more biomarkers selected from the group consisting of: glucose, free fatty acids, liver transaminases, bilirubin, albumin, blood urea nitrogen, creatine, interleukins, tumor necrosis factors, intracellular adhesion molecules, C-reactive protein, testosterone, estrogen, tumor markers, vitamins, minerals and electrolytes.

14. The oligonucleotide for use according to any one of the preceding claims, wherein the treating comprises measuring one or more lipid parameters selected from the group consisting of: apolipoprotein B, LDL-cholesterol, total cholesterol, VLDL-cholesterol, lipoprotein(a), HDL-cholesterol, and triglycerides.

15. A pharmaceutical composition comprising an oligonucleotide comprising the nucleobase sequence of SEQ ID NO:2, for use in treating homozygous familial hypercholesterolemia.

## Patentansprüche

1. Oligonukleotid umfassend die Nukleobasensequenz gemäß SEQ ID NO: 2, zur Verwendung in der Behandlung von homozygoter familiärer Hypercholesterolämie.

2. Oligonukleotid zur Verwendung nach Anspruch 1, wobei die pro Woche verabreichte Oligonukleotidmenge 50-600 mg, wie zum Beispiel 50, 100, 150, 200, 250, 300, 350, 400, 450, 500, 550 oder 600 mg, beträgt.

3. Oligonukleotid zur Verwendung nach Anspruch 1, wobei das Oligonukleotid in einfachen oder mehrfachen Dosen von 0,1-5 mg/kg, 0,5-3 mg/kg, 0,5-8 mg/kg, 0,25-3 mg/kg oder 0,25-2 mg/kg verabreicht wird.

4. Oligonukleotid zur Verwendung nach Anspruch 1, wobei das Oligonukleotid in einfachen oder mehrfachen Dosen von 25-800 mg, 25-600 mg, 25-400 mg, 25-200 mg, 50-600 mg, 50-400 mg oder 50-200 mg pro Tag verabreicht wird.

5. Oligonukleotid zur Verwendung nach einem der vorhergehenden Ansprüche, wobei das Oligonukleotid mittels intravenöser oder subkutaner Injektion verabreicht wird.

6. Oligonukleotid zur Verwendung nach einem der vorhergehenden Ansprüche, wobei das Oligonukleotid aus der Nukleobasensequenz gemäß SEQ ID NO: 2 besteht.

7. Oligonukleotid zur Verwendung nach einem der vorhergehenden Ansprüche, wobei es sich bei dem Oligonukleotid um ein chimäres Oligonukleotid-Gapmer mit einer Länge von 20 Nukleotiden handelt, das aus einer zentralen Gap-Region bestehend aus zehn 2'-Desoxynukleotiden, die beidseitig von aus 2'-Methoxyethyl(2'MOE)-Nukleotiden bestehenden Fünf-Nukleotid-Wings flankiert sind, wobei es sich bei den Internukleosid-Rückgrat-Verknüpfungen in dem gesamten Oligonukleotid um Phosphorothioat handelt und bei allen Cytosinresten um 5-Methylcytosine handelt.

8. Oligonukleotid zur Verwendung nach einem der vorhergehenden Ansprüche, wobei das Behandeln einen oder mehrere Lipidparameter, ausgewählt aus der Gruppe bestehend aus den Folgenden, umfasst: Serum-LDL-Cholesterin, Serum-Apolipoprotein B, Serum-VLDL-Cholesterin, Gesamtserumcholesterin, Serumlipoprotein(a), Serumtriglyceride, Gesamtserumcholesterin:HDL-Verhältnis sowie Serum-LDL:HDL-Verhältnis.

9. Oligonukleotid zur Verwendung nach einem der vorhergehenden Ansprüche, wobei das Behandeln an einem menschlichen Individuum, das zuvor erfolglos mit einem Statin behandelt wurde, durchgeführt wird.

10. Oligonukleotid zur Verwendung nach einem der vorhergehenden Ansprüche, wobei das Oligonukleotid mit einem zweiten lipidsenkenden Mittel verabreicht wird.

11. Oligonukleotid zur Verwendung nach Anspruch 10, wobei das zweite lipidsenkende Mittel aus der Gruppe bestehend aus den Folgenden ausgewählt ist: Gallensäure-Ionenaustauschern (z.B. Cholestyramin, Colestipol und Colesevelam-hydrochlorid), Fibraten (z.B. Clofibrat, Gemfibrozil, Fenofibrat, Bezafibrat und Ciprofibrat), Niacin, Statinen (z.B. Lovastatin, Prevastatin, Atorvastatin, Simvastatin und Fluvastatin) und Cholesterinresorptionshemmern (z.B. Ezetimib) ausgewählt ist.

12. Oligonukleotid zur Verwendung nach einem der vorhergehenden Ansprüche, wobei es sich bei dem Oligonukleotid um ein pharmazeutisch annehmbares Salz handelt.

13. Oligonukleotid zur Verwendung nach einem der vorhergehenden Ansprüche, wobei das Behandeln das Messen von einem oder mehreren Biomarkern, ausgewählt aus der Gruppe bestehend aus den Folgenden, umfasst: Glucose, freien Fettsäuren, Lebertransaminasen, Bilirubin, Albumin, Blutharnstoffstickstoff, Kreatin, Interleukinen, Tumornekrosefaktoren, intrazellulären Adhäsionsmolekülen, C-reaktivem Protein, Testosteron, Östrogen, Tumormarkern, Vitaminen, Mineralstoffen und Elektrolyten.

14. Oligonukleotid zur Verwendung nach einem der vorhergehenden Ansprüche, wobei das Behandeln das Messen von einem oder mehreren Lipidparametern, ausgewählt aus der Gruppe bestehend aus den Folgenden, umfasst: Apolipoprotein B, LDL-Cholesterin, Gesamtcholesterin, VLDL-Cholesterin, Lipoprotein(a), HDL-Cholesterin und Triglyceriden.

15. Pharmazeutische Zusammensetzung, umfassend ein Oligonukleotid umfassend die Nukleobasensequenz gemäß SEQ ID NO: 2, zur Verwendung in der Behandlung von homozygoter familiärer Hypercholesterolämie.

## Revendications

1. Oligonucléotide, comprenant la séquence de nucléobases de SEQ ID n° : 2, destiné à être utilisé dans le traitement d'une hypercholestérolémie familiale homozygote.

2. Oligonucléotide destiné à être utilisé selon la revendication 1, dans lequel la quantité d'oligonucléotide administrée par semaine est de 50 à 600 mg, telle que 50, 100, 150, 200, 250, 300, 350, 400, 450, 500, 550 ou 600 mg.

3. Oligonucléotide destiné à être utilisé selon la revendication 1, l'oligonucléotide étant administré en une seule dose ou en doses multiples de 0,1 à 5 mg/kg, de 0,5 à 3 mg/kg, de 0,5 à 8 mg/kg, de 0,25 à 3 mg/kg ou de 0,25 à 2 mg/kg.

4. Oligonucléotide destiné à être utilisé selon la revendication 1, l'oligonucléotide étant administré en une seule dose ou en doses multiples de 25 à 800 mg, de 25 à 600 mg, de 25 à 400 mg, de 25 à 200 mg, de 50 à 600 mg, de 50 à 400 mg ou de 50 à 200 mg par jour.

5. Oligonucléotide destiné à être utilisé selon l'une quelconque des revendications précédentes, l'oligonucléotide étant administré par injection intraveineuse ou sous-cutanée.

6. Oligonucléotide destiné à être utilisé selon l'une quelconque des revendications précédentes, l'oligonucléotide étant constitué par la séquence de nucléobases de SEQ ID n° : 2.

7. Oligonucléotide destiné à être utilisé selon l'une quelconque des revendications précédentes, l'oligonucléotide étant un gapmer d'oligonucléotide chimère d'une longueur de 20 nucléotides et étant composé d'une région de brèche centrale constituée par dix 2'-désoxynucléotides flanqués sur les deux côtés par cinq ailes nucléotidiques, composées de nucléotides à 2'-méthoxyéthyle (2'MOE), dans lequel les liaisons de squelette inter-nucléosidiques sont de phosphorothioate dans tout l'oligonucléotide et tous les résidus de cytosine sont des 5-méthylcytosines.

8. Oligonucléotide destiné à être utilisé selon l'une quelconque des revendications précédentes, dans lequel le traitement abaisse un ou plusieurs paramètre(s) lipidique(s) choisi(s) dans le groupe constitué par : le LDL-cholestérol sérique, l'apolipoprotéine B sérique, le VLDL-cholestérol sérique, le cholestérol total sérique, la lipoprotéine(a) sérique, les triglycérides sériques, le rapport cholestérol total-HDL sérique et le rapport LDL-HDL sérique.

9. Oligonucléotide destiné à être utilisé selon l'une quelconque des revendications précédentes, dans lequel le traitement se fait chez un sujet humain ayant été traité préalablement sans succès par une statine.

10. Oligonucléotide destiné à être utilisé selon l'une quelconque des revendications précédentes, l'oligonucléotide étant administré avec un deuxième agent hypolipidémiant.

11. Oligonucléotide destiné à être utilisé selon la revendication 10, dans lequel le deuxième agent hypolipidémiant est choisi dans le groupe constitué par : des séquestrants d'un acide biliaire (à titre d'exemple, la cholestyramine, le colestipol et le chlorhydrate de colésévélam), des fibrates (à titre d'exemple, le clofibrate, le gemfibrozil, le fénofibrate, le bézafibrate et le ciprofibrate), la niacine, des statines (à titre d'exemple, la lovastatine, la prévastatine, l'atorvastatine, la simvastatine et la fluvastatine) et des inhibiteurs de l'absorption du cholestérol (à titre d'exemple, l'ézétimibe) .

12. Oligonucléotide destiné à être utilisé selon l'une quelconque des revendications précédentes, l'oligonucléotide étant un sel acceptable d'un point de vue pharmaceutique.

13. Oligonucléotide destiné à être utilisé selon l'une quelconque des revendications précédentes, dans lequel le traitement comprend une mesure d'un ou de plusieurs biomarqueur(s) choisi(s) dans le groupe constitué par : le glucose, des acides gras libres, des transaminases hépatiques, la bilirubine, l'albumine, l'azote uréique sanguin, la créatine, des interleukines, des facteurs de nécrose tumorale, des molécules d'adhérence intracellulaire, la protéine réactive C, la testostérone, un estrogène, des marqueurs tumoraux, vitamines, minéraux et électrolytes.

14. Oligonucléotide destiné à être utilisé selon l'une quelconque des revendications précédentes, dans lequel le traitement comprend une mesure d'un ou de plusieurs paramètre(s) lipidique(s) choisi(s) dans le groupe constitué par : l'apolipoprotéine B, le LDL-cholestérol, le cholestérol total, le VLDL-cholestérol, la lipoprotéine(a), le HDL-cholestérol et les triglycérides.

15. Composition pharmaceutique comprenant un oligonucléotide, qui comprend la séquence de nucléobases de SEQ ID n° : 2, destinée à être utilisée dans le traitement d'une hypercholestérolémie familiale homozygote.

# Figure 1

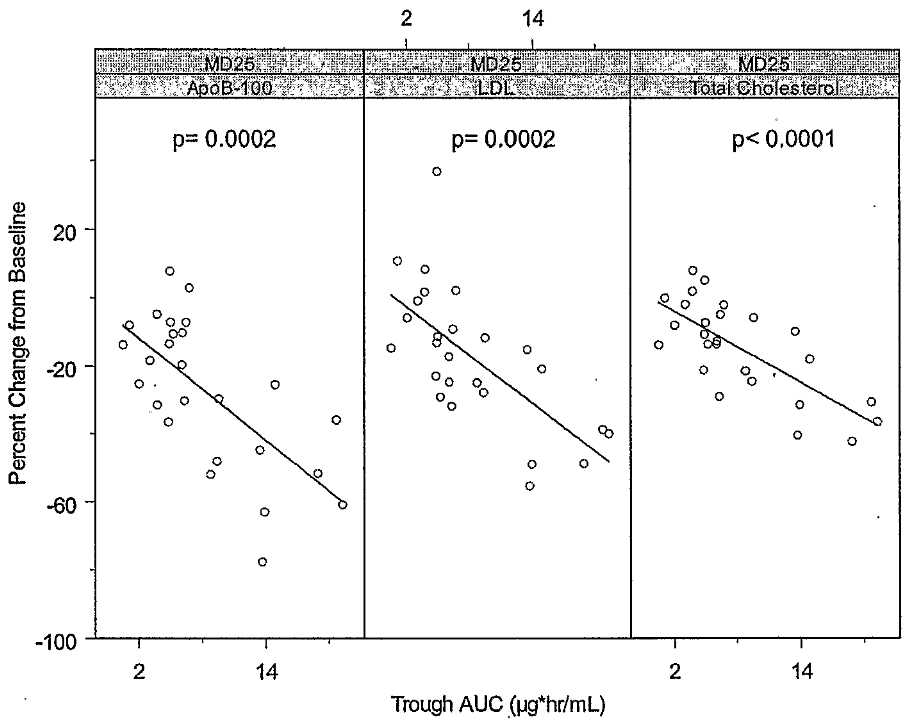
MD25
ApoB-100
MD25
LDL
MD25
Total Cholesterol
p= 0.0002
p= 0.0002
p< 0.0001
Percent Change from Baseline
20
-20
-60
-100
2
14
Trough AUC (µg*hr/mL)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 712795 A
- US 5013830 A
- US 5149797 A
- US 5220007 A
- US 5256775 A
- US 5366878 A
- US 5403711 A
- US 5491133 A
- US 5565350 A
- US 5623065 A
- US 5652355 A
- US 5652356 A
- US 5700922 A
- WO 9324510 A, Gosselin
- WO 9426764 A
- US 5770713 A, Imbach
- WO 2004044181 A
- US 60612831 B
- US 60600785 B
- US 2005028342 W
- EP 05784428 A
- EP 11171206 A


### Non-patent literature cited in the description


- **KNOPP.** *New Engl. J. Medicine,* 1999, vol. 341, 498-511
- **DAVIS ; HUI.** *Arterioscler. Thromb. Vasc. Biol.,* 2001, vol. 21, 887-898
- **BONOW.** *Circulation,* 2002, vol. 106, 3140-3141
- **GRUNDY et al.** *Circulation,* 2004, vol. 110, 227-239
- **DAVIDSON ; SHELNESS.** *Annu. Rev. Nutr.,* 2000, vol. 20, 169-193
- **SEED et al.** *N. Engl. J. Med.,* 1990, vol. 322, 1494-1499
- **SANDKAMP et al.** *Clin. Chem,* 1990, vol. 36, 20-23
- **NOWAK-GOTTL et al.** *Pediatrics,* 1997, vol. 99, E11
- **KANE ; HAVEL.** The Metabolic and Molecular Bases of Inherited Diseases. 2001, 2717-2751
- **GRUNDY.** *Am. J. Cardiol.,* 1998, vol. 81, 18B-25B
- **CHAN et al.** *Diabetes,* 2002, vol. 51, 2377-2386
- **CHAN et al.** *Metabolism,* 2002, vol. 51, 1041-1046
- **KIM ; YOUNG.** *J. Lipid Res.,* 1998, vol. 39, 703-723
- **NISHINA et al.** *J. Lipid Res.,* 1990, vol. 31, 859-869
- **SCHONFELD et al.** *J. Lipid Res.,* 2003, vol. 44, 878-883
- **FARESE et al.** *Proc. Natl. Acad. Sci. U. S. A.,* 1995, vol. 92, 1774-1778
- **MARTIN et al.** *Helv. Chim. Acta,* 1995, vol. 78, 486-504
- Antisense Research and Applications. CRC Press, 1993, 276-278
- **G. S. BANKER.** Modern Pharmaceutics, Drugs and the Pharmaceutical Sciences. May 2002
- **VAN BERGE-HENEGOUWEN et al.** *Gastroenterol.,* 1977, vol. 73, 300